(19) Europäisches Patentamt
European Patent Office
Office européen des brevets

(11) Veröffentlichungsnummer : **0 432 503 B1**

(12) # EUROPÄISCHE PATENTSCHRIFT

(45) Veröffentlichungstag der Patentschrift : **19.01.94 Patentblatt 94/03**

(51) Int. Cl.[5] : **C07C 327/22**, C07C 327/36, A01N 37/44

(21) Anmeldenummer : **90121826.3**

(22) Anmeldetag : **14.11.90**

(54) **Thiolcarbonsäureester und diese enthaltende Fungizide.**

Die Akte enthält technische Angaben, die nach dem Eingang der Anmeldung eingereicht wurden und die nicht in dieser Patentschrift enthalten sind.

(30) Priorität : **16.11.89 DE 3938054**
**13.09.90 DE 4029092**

(43) Veröffentlichungstag der Anmeldung : **19.06.91 Patentblatt 91/25**

(45) Bekanntmachung des Hinweises auf die Patenterteilung : **19.01.94 Patentblatt 94/03**

(84) Benannte Vertragsstaaten : **AT BE CH DE DK ES FR GB GR IT LI NL SE**

(56) Entgegenhaltungen :
**EP-A- 0 253 213**
**EP-A- 0 299 694**

(73) Patentinhaber : **BASF Aktiengesellschaft**
**Carl-Bosch-Strasse 38**
**D-67063 Ludwigshafen (DE)**

(72) Erfinder : **Wingert, Horst, Dr.**
**D 3,4**
**W-6800 Mannheim 1 (DE)**
Erfinder : **Sauter, Hubert, Dr.**
**Neckarpromenade 20**
**W-6800 Mannheim 1 (DE)**
Erfinder : **Brand, Siegbert, Dr.**
**Eyersheimer Strasse 42**
**W-6701 Birkenheide (DE)**
Erfinder : **Wenderoth, Bernd, Dr.**
**Schwalbenstrasse 26**
**W-6840 Lampertheim (DE)**
Erfinder : **Lorenz, Gisela, Dr.**
**Erlenweg 13**
**W-6730 Neustadt (DE)**
Erfinder : **Ammermann, Eberhard, Dr.**
**Sachsenstrasse 3**
**W-6700 Ludwigshafen (DE)**

EP 0 432 503 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Thiolcarbonsäureester der allgemeinen Formel I,

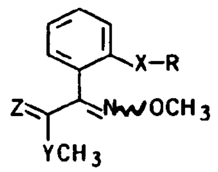

I

in der die Substituenten folgende Bedeutung haben:

X Sauerstoff, Schwefel, Oxymethylen, Methylenoxy, Thiomethylen, Methylenthio, Ethylen, Ethenylen oder Ethinylen,

Y, Z Schwefel oder Sauerstoff, wobei nicht Y mit Z zusammen gleichzeitig Sauerstoff bedeuten,

R $C_1$-$C_6$-Alkyl, ein-, zwei- oder dreikerniges Aryl oder Heteroaryl, wobei Aryl und Heteroaryl folgende Reste $R^1$ tragen können:

$R^1$ Halogen, Cyano, Nitro, $C_1$-$C_6$-Alkyl, $C_3$-$C_6$-Cycloalkyl, $C_1$-$C_6$-Alkoxy, Trifluormethyl, ein- oder zweikerniges Aryloxy oder ein-, zwei- oder dreikerniges Aryl, wobei Aryloxy und Aryl ihrerseits durch die genannten Reste $R^1$ substituiert sein können.

Des weiteren betrifft die Erfindung ein Verfahren zur Herstellung dieser Verbindungen, die Verbindungen enthaltende fungizide Mittel, sowie ein Verfahren zur Bekämpfung von Pilzen mit Hilfe dieser Verbindungen bzw. dieser fungiziden Mittel.

Fungizide mit einer Phenylglyoxylsäureoxim-Struktur sind aus EP-A1-253 213, EP-A2-254 426 und EP-A2-299 694 bekannt. Ein Großteil der in den aufgeführten Schriften beschriebenen Verbindungen läßt hinsichtlich ihrer fungiziden Wirkung zu wünschen übrig.

Der Erfindung lag nun die Aufgabe zugrunde, Fungizide mit besserer Wirkung bereitzustellen.

Demgemäß wurden die eingangs definierten Thiolcarbonsäureester I gefunden.

Von den Thiolcarbonsäureestern I sind diejenigen bevorzugt, bei denen die Substituenten die folgende Bedeutung haben:

X Methylenoxy, Methylenthio, Oxymethylen, Thiomethylen, Ethylen, Ethenylen oder Ethinylen.

R Methyl, Ethyl, n-, iso-Propyl, n-, iso-, sec- oder tert.-Butyl, $C_6$-$C_{14}$-Aryl (wie Phenyl, 1-Naphtyl, 2-Naphtyl oder Phenanthryl), heteroaromatische fünf oder sechsgliedrige Ringe, die eines, zwei, drei oder vier der Heteroatome N, O, oder S enthalten (wie Pyridyl, Pyrimidyl, Pyrazinyl, Pyridazinyl, Imidazolyl, 1,2,3-, 1,2,4-Triazolyl, 1,2,4,5-Tetrazinyl, Furyl, Pyrazolyl, Thienyl, Thiazolyl, Thiadiazolyl) und einen oder zwei ankondensierte aromatische Ringe tragen können (wie Benzofuryl, Benzothienyl, Benzothiazolyl, Chinolinyl, Chinoxazolyl), wobei Aryl und Heteroaryl substiuiert sein und beispielsweise folgende Reste $R^1$ tragen können:

$R^1$ Fluor, Chlor, Brom, Cyano, Nitro, Trifluormethyl, $C_1$-$C_4$-Alkyl, wie Methyl, Ethyl, n-, iso-Propyl, n-, iso-, sec-, tert.-Butyl, Cyclopropyl, Cyclopentyl, Cyclohexyl, $C_1$-$C_4$-Alkoxy, wie Methoxy, Ethoxy, n-, iso-Propoxy, n-, iso-, sec.-, tert.-Butoxy, Phenoxy, 1-Naphthyloxy, 2-Naphthyloxy, Phenyl, 1-Naphthyl, 2-Napthyl, wobei Aryloxy und Aryl ihrerseits durch Reste substituiert sein können, die dieselbe bevorzugte Bedeutung wie $R^1$ haben.

Man erhält die Thiolcarbonsäureester I beispielsweise dadurch, daß man einen Carbonsäureester der allgemeinen Formel II in an sich bekannter Weise (Organikum, 16. Aufl., S. 415, 622) durch Verseifung in die Carbonsäure III überführt, diese dann in ebenfalls an sich bekannter Weise (Houben-Weyl, Bd. E5, S. 855ff, 1985) mit Methanthiol oder einem Alkalimethanthiolat zu Thiolcarbonsäureestern I umsetzt:

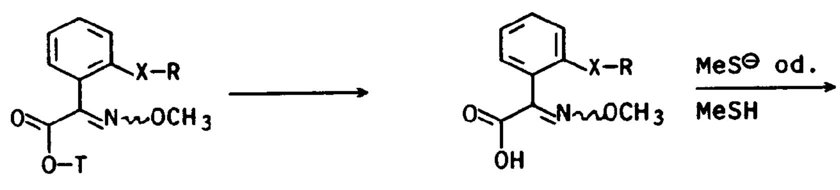


II III I

X und R haben die gleiche Bedeutung wie in der Formel I, T bezeichnet den Rest eines Alkohols, vorzugs-

weise einen $C_1$- bis $C_4$-Alkylrest.

Die Ausgangsverbindungen II können aufgrund der C=N-Doppelbindung als E/Z-Isomerengemische oder aber als reine E- oder Z-Isomere vorliegen, so daß die entsprechenden Folgeprodukte, einschließlich der Thiolcarbonsäureester I, entweder als E/Z-Isomerengemische oder als E oder Z-Isomere anfallen. Die Wellenlinie in den Formeln gibt diesen Sachverhalt wieder. Sowohl die einzelnen isomeren Verbindungen als auch ihre Gemische werden von der Erfindung umfaßt. Die Carbonsäureester II erhält man nach bekannten Methoden, die in den Patentschriften EP-A1-253 213, EP-A2-254 426 und EP-A2-299 694 ausführlich beschrieben sind.

Die Carbonsäureester II werden in der Regel bei Temperaturen von 60 bis 120°C, vorzugsweise 90 bis 110°C verseift, indem man eine wäßrige Alkalihydroxidlösung, bevorzugt ist eine KOH-Lösung, zweckmäßig ohne ein zusätzliches Lösungsmittel mit dem Carbonsäureester zur Reaktion bringt. Das Reaktionsgemisch wird anschließend in der Regel mit konzentrierter Salzsäure bei Raumtemperatur auf pH 0.5 bis 1.5, bevorzugt pH 0.8 bis pH 1.2, angesäuert, wobei die Carbonsäure III ausgefällt wird.

Die Umsetzung der Carbonsäuren III zu den Thiolcarbonsäureestern I kann beispielsweise über Carbonsäurechloride als Zwischenstufe erfolgen (vgl. Houben-Weyl, Bd. 8, S. 464ff, 1952), bevorzugt wird aber der einfachere Weg über ein Imidazolid oder Triazolid. Man arbeitet bevorzugt mit Carbonyldiimidazol, kann aber auch beispielsweise Carbonyl-di-1,2,4-triazol verwenden (vgl. Houben-Weyl, Bd. E5, S. 855f, 1985). Die Reaktion führt man in inerten wasserfreien Lösungsmitteln wie Acetonitril oder Dimethylsulfoxid, insbesondere in Dimethylformamid, bei Temperaturen von (-20) bis 10°C, vorzugsweise (-15) bis 0°C, in einem pH-Bereich von 6 bis 8, vorzugsweise 6.5 bis 7.5, durch.

Nach dieser Reaktion wird in der Regel ohne Isolierung der aktivierten Säure bei Temperaturen von (-20) bis 40°C, insbesondere (-15) bis 30°C, Methanthiol oder ein Alkalimethanthiolat, insbesondere Natriummethanthiolat oder Kaliummethanthiolat, zur Reaktionsmischung gegeben, wobei das Imidazolid oder Triazolid mit dem Thiol oder Thiolat zum Thiolcarbonsäureester I reagiert.

Die erfindungsgemäßen Thiolcarbonsäureester erhält man außerdem, indem man die entsprechenden Carbonsäureester IV, mit Y=O, bzw. die Thiolcarbonsäureester IV, mit Y=S bei Temperaturen von 100-200°C mit einem Schwefelungsmittel, wie z.B. $P_4S_{10}$ oder Lawesson-Reagenz, bevorzugt mit Lawesson-Reagenz (2,4-Bis(4-methoxyphenyl)-1,3-dithia-2,4-diphosphetan-2,4-disulfid)* beispielsweise in einem Verdünnungsmittel, wie z.B. Xylol oder Toluol, umsetzt (vgl. S. O. Lawesson, Bull. Soc. Chim. Belg., 87, 293 (1978)).

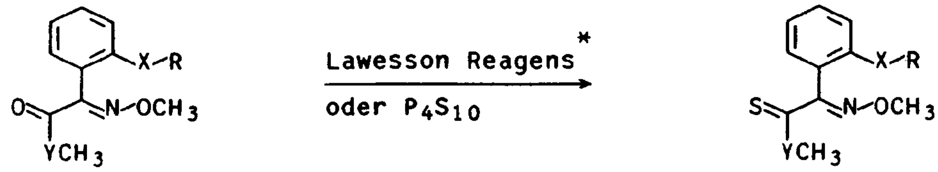


II I

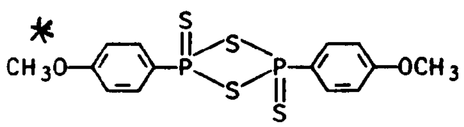

Die folgenden Beispiele erläutern die Herstellung der Thiolcarbonsäureester I.

**Beispiel 1**

Herstellung von 2-(2-Chlorphenoxymethyl)-phenylthiolglyoxylsäure-methylester-O-methyloxim.

5 g 2-(2-Chlorphenoxymethyl)-phenylglyoxylsäure-methylester-O-methyloxim wurden in 50 ml 1N wäßriger KOH-Lösung 2 Stunden unter Rückfluß erhitzt. Nach dem Abkühlen wurde mit konz. Salzsäure ein pH von 1 eingestellt. Der dabei anfallende Niederschlag wurde abgesaugt und getrocknet. Man erhielt 4 g 2-(2-Chlorphenoxymethyl)phenylglyoxylsäureomethyloxim als farblosen Feststoff, der anschließend in 50 ml wasserfreiem DMF gelöst wurde. Bei (-10)°C gab man 2.1 g Carbonyldiimidazol zu und rührte 2 h bei dieser Temperatur. Man versetzte dann mit 0.89 g Natriummethanthiolat, rührte weitere 90 min. bei (-10)°C und anschließend weitere 90 min. bei Raumtemperatur. Nach Zugabe von 100 ml Methylenchlorid und 200 ml gesättigter, wäßriger Natriumchlorid-Lösung, trennte man die organische Phase ab, trocknete über Magnesiumsulfat und entfernte das Lösungsmittel im Vakuum. Man erhielt 3.8 g Rohprodukt. Dieses wurde mittels Flash-Chromatographie an Kieselgel mit Hexan/Essigester gereinigt, wobei 2.3 g Produkt als gelber Feststoff mit einem Schmelzpunkt von 58 bis 60°C erhalten wurden.

**Beispiel 2**

Herstellung von 2-[2-Methylphenoxymethyl]-phenylthio-glyoxylsäure-methylester-O-methyloxim

10.2 g (33 mmol) 2-[2-Methyl-phenoxymethyl]-phenylglyoxylsäure-methylester-O-methyloxim werden in 50 ml Xylol mit 16.6 g (41 mmol) Lawesson-Reagenz versetzt. Man erhitzt 24 Stunden unter Rückfluß und entfernt das Lösungsmittel im Vakuum. Chromatographie an Kieselgel mit Essigester/Hexan liefert 6.3 g (59 %) der Verbindung als schwarzes Öl.

$^1$H-NMR(CDCl3): $\delta$ = 2,27 (s,3H), 4.03 (s,3H), 4.19 (s,3H), 4.95 (s,2H), 6.78-7.61 ppm (m,8H).

**Beispiel 3**

Herstellung von 2-(2-Methylphenoxymethyl)-phenyldithio-glyoxylsäure-methylester-O-methyloxim

Eine Lösung von 5g (15 mmol) 2-(2-Methylphenoxymethyl)-phenylthio-glyoxylsäure-methylester-O-methyloxim in 20 ml Toluol wird mit 3.6 g (9 mmol) Lawesson-Reagenz versetzt. Man erhitzt 24 Stunden unter Rückfluß und engt ein. Der Rückstand wird an Kieselgel mit Essigester/Hexan chromatographiert. Man erhält 2.8 g (54 %) der Verbindung als schwarzes Öl.

$^1$H-NMR(CDCl3): $\delta$ = 2.25 (s,3H), 2.60 (s,3H), 4.02(s,3H), 4.91 (s,2H), 6.78-7.61 ppm (m,8H).

In entsprechender Weise können die in den folgenden Tabellen aufgeführten Verbindungen hergestellt werden.

Tabelle 1

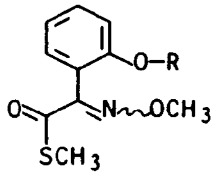

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |

| Nr. | R | Fp($^{o}C$)/IR($cm^{-1}$) |
|---|---|---|
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |
| 35 | 2,4-$Br_2$-phenyl | |
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |

| Nr. | R | Fp(°C)/IR($cm^{-1}$) |
|---|---|---|
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |
| 77 | $Br_5$-phenyl | |
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |

| Nr. | R | Fp($^{o}$C)/IR($cm^{-1}$) |
|---|---|---|
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-$(i-C_3H_7)_2$-phenyl | |
| 116 | 2,6-$(i-C_3H_7)_2$-phenyl | |
| 117 | 3,5-$(i-C_3H_7)_2$-phenyl | |
| 118 | 2,4,6-$(i-C_3H_7)_3$-phenyl | |
| 119 | 2,3-$(t-C_4H_9)_2$-phenyl | |
| 120 | 2,4-$(t-C_4H_9)_2$-phenyl | |
| 121 | 2,5-$(t-C_4H_9)_2$-phenyl | |
| 122 | 2,6-$(t-C_4H_9)_2$-phenyl | |
| 123 | 3,5-$(t-C_4H_9)_2$-phenyl | |
| 124 | 2,4,6-$(t-C_4H_9)_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-$(t-C_4H_9)_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-$(t-C_4H_9)_2$-6-i-$C_3H_7$-phenyl | |
| 135 | 2-cyclo-$C_6H_{11}$-phenyl | |
| 136 | 3-cyclo-$C_6H_{11}$-phenyl | |

| Nr. | R | Fp(°C)/IR($cm^{-1}$) |
|---|---|---|
| 137 | 4-cyclo-$C_6H_{11}$-phenyl | |
| 138 | 2,4-(cyclo-$C_6H_{11}$)$_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-($CH_3$)$_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |
| 161 | 2-Br, 4-cyclo-$C_6H_{11}$-phenyl | |
| 162 | 2-$OCH_3$-phenyl | |
| 163 | 3-$OCH_3$-phenyl | |
| 164 | 4-$OCH_3$-phenyl | |
| 165 | 2,4-($OCH_3$)$_2$-phenyl | |
| 166 | 2-$CF_3$-phenyl | |
| 167 | 3-$CF_3$-phenyl | |
| 168 | 4-$CF_3$-phenyl | |
| 169 | 2-$NO_2$-phenyl | |
| 170 | 3-$NO_2$-phenyl | |
| 171 | 4-$NO_2$-phenyl | |
| 172 | 2-CN-phenyl | |
| 173 | 3-CN-phenyl | |

| Nr. | R | Fp($^{o}$C)/IR($cm^{-1}$) |
|---|---|---|
| 174 | 4-CN-phenyl | |
| 175 | 2-$CH_3$, 3-Cl-phenyl | |
| 176 | 2-$CH_3$, 4-Cl-phenyl | |
| 177 | 2-$CH_3$, 5-Cl-phenyl | |
| 178 | 2-$CH_3$, 6-Cl-phenyl | |
| 179 | 2-$CH_3$, 3-F-phenyl | |
| 180 | 2-$CH_3$, 4-F-phenyl | |
| 181 | 2-$CH_3$, 5-F-phenyl | |
| 182 | 2-$CH_3$, 6-F-phenyl | |
| 183 | 2-$CH_3$, 3-Br-phenyl | |
| 184 | 2-$CH_3$, 4-Br-phenyl | |
| 185 | 2-$CH_3$, 5-Br-phenyl | |
| 186 | 2-$CH_3$, 6-Br-phenyl | |
| 187 | 2-Cl, 3-$CH_3$-phenyl | |
| 188 | 2-Cl, 4-$CH_3$-phenyl | |
| 189 | 2-Cl, 5-$CH_3$-phenyl | |
| 190 | 2-Cl, 3-i-$C_3H_7$ | |
| 191 | 2-F, 3-$CH_3$-phenyl | |
| 192 | 2-F, 4-$CH_3$-phenyl | |
| 193 | 2-F, 5-$CH_3$-phenyl | |
| 194 | 2-Br, 3-$CH_3$-phenyl | |
| 195 | 2-Br, 4-$CH_3$-phenyl | |
| 196 | 3-$CH_3$, 4-Cl-phenyl | |
| 197 | 3-$CH_3$, 5-Cl-phenyl | |
| 198 | 2-Br, 5-$CH_3$-phenyl | |
| 199 | 3-$CH_3$, 4-F-phenyl | |
| 200 | 3-$CH_3$, 5-F-phenyl | |
| 201 | 3-$CH_3$, 4-Br-phenyl | |
| 202 | 3-$CH_3$, 5-Br-phenyl | |
| 203 | 3-F, 4-$CH_3$-phenyl | |
| 204 | 3-Cl, 4-$CH_3$-phenyl | |
| 205 | 3-Br, 4-$CH_3$-phenyl | |
| 206 | 2-Cl, 4,5-$(CH_3)_2$-phenyl | |
| 207 | 2-Br, 4,5-$(CH_3)_2$-phenyl | |
| 208 | 2-Cl, 3,5-$(CH_3)_2$-phenyl | |
| 209 | 2-Br, 3,5-$(CH_3)_2$-phenyl | |
| 210 | 2,6-$Cl_2$, 4-$CH_3$-phenyl | |
| 211 | 2,6-$F_2$, 4-$CH_3$-phenyl | |

| Nr. | R | Fp(°C)/IR($cm^{-1}$) |
|---|---|---|
| 212 | 2,6-$Br_2$, 4-$CH_3$-phenyl | |
| 213 | 2,4-$Cl_2$, 6-$CH_3$-phenyl | |
| 214 | 2,4-$F_2$, 6-$CH_3$-phenyl | |
| 215 | 2,4-$Br_2$, 6-$CH_3$-phenyl | |
| 216 | 2,6-$(CH_3)_2$, 4-F-phenyl | |
| 217 | 2,6-$(CH_3)_2$, 4-Cl-phenyl | |
| 218 | 2,6-$(CH_3)_2$, 4-Br-phenyl | |
| 219 | 3,5-$(CH_3)_2$, 4-F-phenyl | |
| 220 | 3,5-$(CH_3)_2$, 4-Cl-phenyl | |
| 221 | 3,5-$(CH_3)_2$, 4-Br-phenyl | |
| 222 | 2,3,6-$(CH_3)_3$, 4-F-phenyl | |
| 223 | 2,3,6-$(CH_3)_3$, 4-Cl-phenyl | |
| 224 | 2,3,6-$(CH_3)_3$, 4-Br-phenyl | |
| 225 | 2,4-$(CH_3)_2$, 6-F-phenyl | |
| 226 | 2,4-$(CH_3)_2$, 6-Cl-phenyl | |
| 227 | 2,4-$(CH_3)_2$, 6-Br-phenyl | |
| 228 | 2-i-$C_3H_7$, 4-Cl, 5-$CH_3$-phenyl | |
| 229 | 2-Cl, 4-$NO_2$-phenyl | |
| 230 | 2-$NO_2$, 4-Cl-phenyl | |
| 231 | 2-$OCH_3$, 5-$NO_2$-phenyl | |
| 232 | 2,4-$Cl_2$, 5-$NO_2$-phenyl | |
| 233 | 2,4-$Cl_2$, 6-$NO_2$-phenyl | |
| 234 | 2,6-$Cl_2$, 4-$NO_2$-phenyl | |
| 235 | 2,6-$Br_2$, 4-$NO_2$-phenyl | |
| 236 | 2,6-$J_2$, 4-$NO_2$-phenyl | |
| 237 | 2-$C_6H_5O$-phenyl | |
| 238 | 3-$C_6H_5O$-phenyl | |
| 239 | 4-$C_6H_5O$-phenyl | |
| 240 | 3-t-$C_4H_9O$-phenyl | |
| 241 | 4-t-$C_4H_9O$-phenyl | |
| 242 | 1-Naphthyl | |
| 243 | 2-Naphthyl | |
| 244 | 2-Pyridyl | |
| 245 | 6-Methyl-2-pyridyl | |
| 246 | 6-Ethyl-2-pyridyl | |
| 247 | 6-n-Propyl-2-pyridyl | |

| Nr. | R | Fp(°C)/IR($cm^{-1}$) |
|---|---|---|
| 248 | 6-iso-Propyl-2-pyridyl | |
| 249 | 6-n-Butyl-2-pyridyl | |
| 250 | 6-tert.-Butyl-2-pyridyl | |
| 251 | 6-n-Pentyl-2-pyridyl | |
| 252 | 6-n-Hexyl-2-pyridyl | |
| 253 | 6-Phenyl-2-pyridyl | |
| 254 | 6-Benzyl-2-pyridyl | |
| 255 | 6-Trifluormethyl-2-pyridyl | |
| 256 | 6-Methoxy-2-pyridyl | |
| 257 | 6-Chloro-2-pyridyl | |
| 258 | 3,6-Dimethyl-2-pyridyl | |
| 259 | 3,6-Diethyl-2-pyridyl | |
| 260 | 4,6-Dimethyl-2-pyridyl | |
| 261 | 5,6-Dimethyl-2-pyridyl | |
| 262 | 4-Phenyl-6-methyl-2-pyridyl | |
| 263 | 4,6-Diphenyl-2-pyridyl | |
| 264 | 3,4-Dichloro-6-methyl-2-pyridyl | |
| 265 | 3,4,5-Trichloro-6-phenyl-2-pyridyl | |
| 266 | 4-Trifluoromethyl-6-methyl-2-pyridyl | |
| 267 | 3-Cyano-6-methyl-2-pyridyl | |
| 268 | 3-Cyano-6-ethyl-2-pyridyl | |
| 269 | 3-Cyano-6-n-propyl-2-pyridyl | |
| 270 | 3-Cyano-6-iso-propyl-2-pyridyl | |
| 271 | 3-Cyano-6-cyclo-propyl-2-pyridyl | |
| 272 | 3-Cyano-6-n-butyl-2-pyridyl | |
| 273 | 3-Cyano-6-tert.-butyl-2-pyridyl | |
| 274 | 3-Cyano-6-cyclo-hexyl-2-pyridyl | |
| 275 | 3-Cyano-6-phenyl-2-pyridyl | |
| 276 | 3-Cyano-4,6-dimethyl-2-pyridyl | |
| 277 | 3,5,6-Trichloro-2-pyridyl | |
| 278 | 5-Trifluoromethyl-2-pyridyl | |
| 279 | 3-Chloro-5-trifluoromethyl-2-pyridyl | |
| 280 | 2-Chinolyl | |
| 281 | 3-Methyl-2-chinolyl | |
| 282 | 4-Methyl-2-chinolyl | |

| Nr. | R | Fp(°C)/IR($cm^{-1}$) |
|---|---|---|
| 283 | 4-Ethyl-2-chinolyl | |
| 284 | 4-Phenyl-2-chinolyl | |
| 285 | 6-Methyl-2-chinolyl | |
| 286 | 6-Chloro-2-chinolyl | |
| 287 | 8-Methyl-2-chinolyl | |
| 288 | 8-Chloro-2-chinolyl | |
| 289 | 3,4-Dimethyl-2-chinolyl | |
| 290 | 4-Methyl-8-methoxy-2-chinolyl | |
| 291 | 4-Phenyl-8-ethoxy-2-chinolyl | |
| 292 | 4-Methyl-8-chloro-2-chinolyl | |
| 293 | 4-Methyl-8-fluoro-2-chinolyl | |
| 294 | 4-Chinolyl | |
| 295 | 2-Methyl-4-chinolyl | |
| 296 | 2-Trifluoromethyl-4-chinolyl | |
| 297 | 2-iso-Propyl-4-chinolyl | |
| 298 | 2-n-Pentyl-4-chinolyl | |
| 299 | 2-Phenyl-4-chinolyl | |
| 300 | 2,6-Dimethyl-4-chinolyl | |
| 301 | 2-Methyl-6-chloro-4-chinolyl | |
| 302 | 2-Methyl-6-fluoro-4-chinolyl | |
| 303 | 8-Chinolyl | |
| 304 | 2-Methyl-8-chinolyl | |
| 305 | 5,7-Dichloro-8-chinolyl | |
| 306 | 4,6-Dimethyl-2-pyrimidinyl | |
| 307 | 4-Trifluormethyl-2-pyrimidinyl | |
| 308 | 4,5,6-Trimethyl-2-pyrimidinyl | |
| 309 | 4-Benzyl-6-methyl-2-pyrimidinyl | |
| 310 | 4-Methyl-6-phenyl-2-pyrimidinyl | |
| 311 | 4,6-Dimethyl-5-chloro-2-pyrimidinyl | |
| 312 | 2,6-Dimethyl-4-pyrimidinyl | |
| 313 | 2,6-Bis-(trifluormethyl)-4-pyrimidinyl | |
| 314 | 2-iso-Propyl-6-methyl-4-pyrimidinyl | |
| 315 | 2-cyclo-Propyl-6-methyl-4-pyrimidinyl | |
| 316 | 2-Methyl-6-methoxymethyl-4-pyrimidinyl | |
| 317 | 2-iso-Propyl-6-methoxymethyl-4-pyrimidinyl | |
| 318 | 2-Phenyl-4-pyrimidinyl | |

| Nr. | R | Fp(°C)/IR($cm^{-1}$) |
|---|---|---|
| 319 | 3,5-Dimethyl-4-pyrimidinyl | |
| 320 | 2-Methyl-6-trifluoromethyl-4-pyrimidinyl | |
| 321 | 2-n-Propyl-6-trifluoromethyl-4-pyrimidinyl | |
| 322 | 2-iso-Propyl-6-trifluoromethyl-4-pyrimidinyl | |
| 323 | 2-Methyl-5-chloro-6-trifluoromethyl-4-pyrimidinyl | |
| 324 | 2-n-Propyl-5-chloro-6-trifluoromethyl-4-pyrimidinyl | |
| 325 | 2-iso-Propyl-5-chloro-6-trifluoromethyl-4-pyrimidinyl | |
| 326 | 2-tert.-Butyl-5-chloro-6-trifluoromethyl-4-pyrimidinyl | |

Tabelle 2

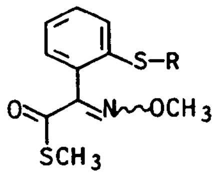

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |

| Nr. | R | Fp(°C)/IR($cm^{-1}$) |
|---|---|---|
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |
| 35 | 2,4-$Br_2$-phenyl | |
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |

| Nr. | R | Fp(°C)/IR($cm^{-1}$) |
|---|---|---|
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |
| 77 | $Br_5$-phenyl | |
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |

| Nr. | R | Fp($^oC$)/IR($cm^{-1}$) |
|---|---|---|
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-$(i\text{-}C_3H_7)_2$-phenyl | |
| 116 | 2,6-$(i\text{-}C_3H_7)_2$-phenyl | |
| 117 | 3,5-$(i\text{-}C_3H_7)_2$-phenyl | |
| 118 | 2,4,6-$(i\text{-}C_3H_7)_3$-phenyl | |
| 119 | 2,3-$(t\text{-}C_4H_9)_2$-phenyl | |
| 120 | 2,4-$(t\text{-}C_4H_9)_2$-phenyl | |
| 121 | 2,5-$(t\text{-}C_4H_9)_2$-phenyl | |
| 122 | 2,6-$(t\text{-}C_4H_9)_2$-phenyl | |
| 123 | 3,5-$(t\text{-}C_4H_9)_2$-phenyl | |
| 124 | 2,4,6-$(t\text{-}C_4H_9)_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-$(t\text{-}C_4H_9)_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-$(t\text{-}C_4H_9)_2$-6-i-$C_3H_7$-phenyl | |
| 135 | 2-cyclo-$C_6H_{11}$-phenyl | |
| 136 | 3-cyclo-$C_6H_{11}$-phenyl | |

| Nr. | R | Fp(°C)/IR($cm^{-1}$) |
|---|---|---|
| 137 | 4-cyclo-$C_6H_{11}$-phenyl | |
| 138 | 2,4-(cyclo-$C_6H_{11}$)$_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-($CH_3$)$_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |
| 161 | 2-Br, 4-cyclo-$C_6H_{11}$-phenyl | |
| 162 | 3-$OCH_3$-phenyl | |
| 163 | 2,4-($OCH_3$)$_2$-phenyl | |
| 164 | 2-$CF_3$-phenyl | |
| 165 | 3-$CF_3$-phenyl | |
| 166 | 4-$CF_3$-phenyl | |
| 167 | 2-$NO_2$-phenyl | |
| 168 | 3-$NO_2$-phenyl | |
| 169 | 4-$NO_2$-phenyl | |
| 170 | 2-CN-phenyl | |
| 171 | 3-CN-phenyl | |

| Nr. | R | Fp(°C)/IR($cm^{-1}$) |
|---|---|---|
| 172 | 4-CN-phenyl | |
| 173 | 2-$CH_3$, 3-Cl-phenyl | |
| 174 | 2-$CH_3$, 4-Cl-phenyl | |
| 175 | 2-$CH_3$, 5-Cl-phenyl | |
| 176 | 2-$CH_3$, 6-Cl-phenyl | |
| 177 | 2-$CH_3$, 3-F-phenyl | |
| 178 | 2-$CH_3$, 4-F-phenyl | |
| 179 | 2-$CH_3$, 5-F-phenyl | |
| 180 | 2-$CH_3$, 6-F-phenyl | |
| 181 | 2-$CH_3$, 3-Br-phenyl | |
| 182 | 2-$CH_3$, 4-Br-phenyl | |
| 183 | 2-$CH_3$, 5-Br-phenyl | |
| 184 | 2-$CH_3$, 6-Br-phenyl | |
| 185 | 2-Cl, 3-$CH_3$-phenyl | |
| 186 | 2-Cl, 4-$CH_3$-phenyl | |
| 187 | 2-Cl, 5-$CH_3$-phenyl | |
| 188 | 2-Cl, 3-i-$C_3H_7$ | |
| 189 | 2-F, 3-$CH_3$-phenyl | |
| 190 | 2-F, 4-$CH_3$-phenyl | |
| 191 | 2-F, 5-$CH_3$-phenyl | |
| 192 | 2-Br, 3-$CH_3$-phenyl | |
| 193 | 2-Br, 4-$CH_3$-phenyl | |
| 194 | 3-$CH_3$, 4-Cl-phenyl | |
| 195 | 3-$CH_3$, 5-Cl-phenyl | |
| 196 | 2-Br, 5-$CH_3$-phenyl | |
| 197 | 3-$CH_3$, 4-F-phenyl | |
| 198 | 3-$CH_3$, 5-F-phenyl | |
| 199 | 3-$CH_3$, 4-Br-phenyl | |
| 200 | 3-$CH_3$, 5-Br-phenyl | |
| 201 | 3-F, 4-$CH_3$-phenyl | |
| 202 | 3-Cl, 4-$CH_3$-phenyl | |
| 203 | 3-Br, 4-$CH_3$-phenyl | |
| 204 | 2-Cl, 4,5-$(CH_3)_2$-phenyl | |
| 205 | 2-Br, 4,5-$(CH_3)_2$-phenyl | |
| 206 | 2-Cl, 3,5-$(CH_3)_2$-phenyl | |
| 207 | 2-Br, 3,5-$(CH_3)_2$-phenyl | |
| 208 | 2,6-$Cl_2$, 4-$CH_3$-phenyl | |
| 209 | 2,6-$F_2$, 4-$CH_3$-phenyl | |

| Nr. | R | Fp($^{o}C$)/IR($cm^{-1}$) |
|---|---|---|
| 210 | 2,6-$Br_2$, 4-$CH_3$-phenyl | |
| 211 | 2,4-$Cl_2$, 6-$CH_3$-phenyl | |
| 212 | 2,4-$F_2$, 6-$CH_3$-phenyl | |
| 213 | 2,4-$Br_2$, 6-$CH_3$-phenyl | |
| 214 | 2,6-$(CH_3)_2$, 4-F-phenyl | |
| 215 | 2,6-$(CH_3)_2$, 4-Cl-phenyl | |
| 216 | 2,6-$(CH_3)_2$, 4-Br-phenyl | |
| 217 | 3,5-$(CH_3)_2$, 4-F-phenyl | |
| 218 | 3,5-$(CH_3)_2$, 4-Cl-phenyl | |
| 219 | 3,5-$(CH_3)_2$, 4-Br-phenyl | |
| 220 | 2,3,6-$(CH_3)_3$, 4-F-phenyl | |
| 221 | 2,3,6-$(CH_3)_3$, 4-Cl-phenyl | |
| 222 | 2,3,6-$(CH_3)_3$, 4-Br-phenyl | |
| 223 | 2,4-$(CH_3)_2$, 6-F-phenyl | |
| 224 | 2,4-$(CH_3)_2$, 6-Cl-phenyl | |
| 225 | 2,4-$(CH_3)_2$, 6-Br-phenyl | |
| 226 | 2-i-$C_3H_7$, 4-Cl, 5-$CH_3$-phenyl | |
| 227 | 2-Cl, 4-$NO_2$-phenyl | |
| 228 | 2-$NO_2$, 4-Cl-phenyl | |
| 229 | 2-$OCH_3$, 5-$NO_2$-phenyl | |
| 230 | 2,4-$Cl_2$, 5-$NO_2$-phenyl | |
| 231 | 2,4-$Cl_2$, 6-$NO_2$-phenyl | |
| 232 | 2,6-$Cl_2$, 4-$NO_2$-phenyl | |
| 233 | 2,6-$Br_2$, 4-$NO_2$-phenyl | |
| 234 | 2,6-$J_2$, 4-$NO_2$-phenyl | |
| 235 | 2-$C_6H_5O$-phenyl | |
| 236 | 3-$C_6H_5O$-phenyl | |
| 237 | 4-$C_6H_5O$-phenyl | |
| 238 | 3-t-$C_4H_9O$-phenyl | |
| 239 | 4-t-$C_4H_9O$-phenyl | |
| 240 | 1-Naphthyl | |
| 241 | 2-Naphthyl | |
| 242 | 2-Pyridyl | |
| 243 | 6-Methyl-2-pyridyl | |
| 244 | 6-Ethyl-2-pyridyl | |
| 245 | 6-n-Propyl-2-pyridyl | |

| Nr. | R | Fp($^{o}$C)/IR(cm$^{-1}$) |
|---|---|---|
| 246 | 6-iso-Propyl-2-pyridyl | |
| 247 | 6-n-Butyl-2-pyridyl | |
| 248 | 6-tert.-Butyl-2-pyridyl | |
| 249 | 6-n-Pentyl-2-pyridyl | |
| 250 | 6-n-Hexyl-2-pyridyl | |
| 251 | 6-Phenyl-2-pyridyl | |
| 252 | 6-Benzyl-2-pyridyl | |
| 253 | 6-Trifluormethyl-2-pyridyl | |
| 254 | 6-Methoxy-2-pyridyl | |
| 255 | 6-Chloro-2-pyridyl | |
| 256 | 3,6-Dimethyl-2-pyridyl | |
| 257 | 3,6-Diethyl-2-pyridyl | |
| 258 | 4,6-Dimethyl-2-pyridyl | |
| 259 | 5,6-Dimethyl-2-pyridyl | |
| 260 | 4-Phenyl-6-methyl-2-pyridyl | |
| 261 | 4,6-Diphenyl-2-pyridyl | |
| 262 | 3,4-Dichloro-6-methyl-2-pyridyl | |
| 263 | 3,4,5-Trichloro-6-phenyl-2-pyridyl | |
| 264 | 4-Trifluoromethyl-6-methyl-2-pyridyl | |
| 265 | 3-Cyano-6-methyl-2-pyridyl | |
| 266 | 3-Cyano-6-ethyl-2-pyridyl | |
| 267 | 3-Cyano-6-n-propyl-2-pyridyl | |
| 268 | 3-Cyano-6-iso-propyl-2-pyridyl | |
| 269 | 3-Cyano-6-cyclo-propyl-2-pyridyl | |
| 270 | 3-Cyano-6-n-butyl-2-pyridyl | |
| 271 | 3-Cyano-6-tert.-butyl-2-pyridyl | |
| 272 | 3-Cyano-6-cyclo-hexyl-2-pyridyl | |
| 273 | 3-Cyano-6-phenyl-2-pyridyl | |
| 274 | 3-Cyano-4,6-dimethyl-2-pyridyl | |
| 275 | 3,5,6-Trichloro-2-pyridyl | |
| 276 | 5-Trifluoromethyl-2-pyridyl | |
| 277 | 3-Chloro-5-trifluoromethyl-2-pyridyl | |
| 278 | 2-Chinolyl | |
| 279 | 3-Methyl-2-chinolyl | |
| 280 | 4-Methyl-2-chinolyl | |

| Nr. | R | Fp(°C)/IR($cm^{-1}$) |
|---|---|---|
| 281 | 4-Ethyl-2-chinolyl | |
| 282 | 4-Phenyl-2-chinolyl | |
| 283 | 6-Methyl-2-chinolyl | |
| 284 | 6-Chloro-2-chinolyl | |
| 285 | 8-Methyl-2-chinolyl | |
| 286 | 8-Chloro-2-chinolyl | |
| 287 | 3,4-Dimethyl-2-chinolyl | |
| 288 | 4-Methyl-8-methoxy-2-chinolyl | |
| 289 | 4-Phenyl-8-ethoxy-2-chinolyl | |
| 290 | 4-Methyl-8-chloro-2-chinolyl | |
| 291 | 4-Methyl-8-fluoro-2-chinolyl | |
| 292 | 4-Chinolyl | |
| 293 | 2-Methyl-4-chinolyl | |
| 294 | 2-Trifluoromethyl-4-chinolyl | |
| 295 | 2-iso-Propyl-4-chinolyl | |
| 296 | 2-n-Pentyl-4-chinolyl | |
| 297 | 2-Phenyl-4-chinolyl | |
| 298 | 2,6-Dimethyl-4-chinolyl | |
| 299 | 2-Methyl-6-chloro-4-chinolyl | |
| 300 | 2-Methyl-6-fluoro-4-chinolyl | |
| 301 | 8-Chinolyl | |
| 302 | 2-Methyl-8-chinolyl | |
| 303 | 5,7-Dichloro-8-chinolyl | |
| 304 | 4,6-Dimethyl-2-pyrimidinyl | |
| 305 | 4-Trifluormethyl-2-pyrimidinyl | |
| 306 | 4,5,6-Trimethyl-2-pyrimidinyl | |
| 307 | 4-Benzyl-6-methyl-2-pyrimidinyl | |
| 308 | 4-Methyl-6-phenyl-2-pyrimidinyl | |
| 309 | 4,6-Dimethyl-5-chloro-2-pyrimidinyl | |
| 310 | 2,6-Dimethyl-4-pyrimidinyl | |
| 311 | 2,6-Bis-(trifluormethyl)-4-pyrimidinyl | |
| 312 | 2-iso-Propyl-6-methyl-4-pyrimidinyl | |
| 313 | 2-cyclo-Propyl-6-methyl-4-pyrimidinyl | |
| 314 | 2-Methyl-6-methoxymethyl-4-pyrimidinyl | |
| 315 | 2-iso-Propyl-6-methoxymethyl-4-pyrimidinyl | |
| 316 | 2-Phenyl-4-pyrimidinyl | |

| Nr. | R | Fp(°C)/IR($cm^{-1}$) |
|---|---|---|
| 317 | 3,5-Dimethyl-4-pyrimidinyl | |
| 318 | 2-Methyl-6-trifluoromethyl-4-pyrimidinyl | |
| 319 | 2-n-Propyl-6-trifluoromethyl-4-pyrimidinyl | |
| 320 | 2-iso-Propyl-6-trifluoromethyl-4-pyrimidinyl | |
| 321 | 2-Methyl-5-chloro-6-trifluoromethyl-4-pyrimidinyl | |
| 322 | 2-n-Propyl-5-chloro-6-trifluoromethyl-4-pyrimidinyl | |
| 323 | 2-iso-Propyl-5-chloro-6-trifluoromethyl-4-pyrimidinyl | |
| 324 | 2-tert.-Butyl-5-chloro-6-trifluoromethyl-4-pyrimidinyl | |
| 325 | 6-EtO-benzothiazol-2-yl | |
| 326 | benzoxazol-2-yl | |
| 327 | 1-Me-imidazol-2-yl | |
| 328 | 4-But-1H-imidazol-2-yl | |
| 329 | 2-thiazolin-2-yl | |
| 330 | 5-$CF_3$-benzimidazol-2-yl | |
| 331 | 1-Ph-tetrazol-5-yl | |
| 332 | 5-$CF_3$-benzothiazol-2-yl | |
| 333 | 4,4-$Me_2$-5-methylene-2-thiazolin-2-yl | |
| 334 | 6-Cl-4-Me-benzothiazol-2-yl | |
| 335 | 5-Me-benzothiazol-2-yl | |
| 336 | 4-Cl-benzothiazol-2-yl | |
| 337 | 1-(3-$NO_2$-Ph)tetrazol-5-yl | |
| 338 | 2-thienyl | |
| 339 | 5-Me-benzoxazol-2-yl | |
| 340 | 7-Cl-benzothiazol-2-yl | |
| 341 | 5,6-$Cl_3$-1H-benzimidazol-2-yl | |
| 342 | 5-Cl-benzoxazol-2-yl | |
| 343 | 6-Cl-benzothiazol-2-yl | |
| 344 | 2-Ph-thiazol-2-yl | |
| 345 | 3-CN-4,6-$Me_2$-2-pyridyl | |
| 346 | 1-Ph-1,2,4-triazol-3-yl | |
| 347 | 1-Pri-benzimidazol-2-yl | |
| 348 | 5-Br-benzothiazol-2-yl | |
| 349 | 5-Br-1H-benzimidazol-2-yl | |
| 350 | 7-Cl-4-MeO-benzothiazol-2-yl | |
| 351 | 1H-benzimidazol-2-yl | |

| Nr. | R | Fp($^{o}$C)/IR($cm^{-1}$) |
|---|---|---|
| 352 | 5-Cl-benzothiazol-2-yl | |
| 353 | 5-$NO_2$-benzoxazol-2-yl | |
| 354 | 5-t-Bu-benzoxazol-2-yl | |
| 355 | 4,6,7-$Cl_3$-benzothiozol-2-yl | |
| 356 | 5-Ph-thiazol-2-yl | |
| 357 | 5,7-$Me_2$-benzoxazol-2-yl | |
| 358 | 6-Me-benzoxazol-2-yl | |
| 359 | 1,2,4-triazin-3-yl | |
| 360 | 6-Pr-benzothiazol-2-yl | |
| 361 | 6-PhO-benzothiazol-2-yl | |
| 362 | 4-(4-Cl-Ph)-thiazol-2-yl | |
| 363 | 4-(4-Me-Ph)-thiazol-2-yl | |
| 364 | 5-Me-4-Ph-thiazol-2-yl | |
| 365 | 5-Cl-1-H-benzimidazol-2-yl | |
| 366 | 5-Ph-1,2,4-triazol-3-yl | |
| 367 | 3-Ph-1,2,4-thiadiazol-5-yl | |

Tabelle 3

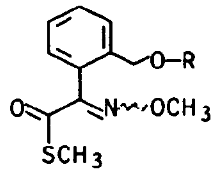

| Nr. | R | Fp($^{o}C$)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | 1663, 1598, 1587, 1496, 1242, 1221, 1026, 845, 756, 691 |
| 12 | 2-F-Phenyl | |
| 13 | 3-F-Phenyl | |
| 14 | 4-F-Phenyl | |
| 15 | 2-Cl-Phenyl | 58- 60°C |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-$CH_3$-Phenyl | 1662, 1494, 1461, 1240, 1190, 1122, 1051, 1025, 845, 753 ($cm^{-1}$) |
| 25 | 2-CN-Phenyl | |
| 26 | 3-CN-Phenyl | |
| 27 | 4-CN-Phenyl | |
| 28 | 2,3-$F_2$-phenyl | |
| 29 | 2,4-$F_2$-phenyl | |

| Nr. | R | Fp(°C)/IR($cm^{-1}$) |
|---|---|---|
| 30 | 2,3-$Cl_2$-phenyl | |
| 31 | 2,4-$Cl_2$-phenyl | |
| 32 | 2,5-$Cl_2$-phenyl | |
| 33 | 2,6-$Cl_2$-phenyl | |
| 34 | 3,4-$Cl_2$-phenyl | |
| 35 | 3,5-$Cl_2$-phenyl | |
| 36 | 2,4-$Br_2$-phenyl | |
| 37 | 2,5-$Br_2$-phenyl | |
| 38 | 2,6-$Br_2$-phenyl | |
| 39 | 2,4-$J_2$-phenyl | |
| 40 | 2-Cl, 3-F-phenyl | |
| 41 | 2-Cl, 4-F-phenyl | |
| 42 | 2-Cl, 5-F-phenyl | |
| 43 | 2-Cl, 6-F-phenyl | |
| 44 | 2-Cl, 3-Br-phenyl | |
| 45 | 2-Cl, 4-Br-phenyl | |
| 46 | 2-Cl, 5-Br-phenyl | |
| 47 | 2-Cl, 6-Br-phenyl | |
| 48 | 2-Br, 3-Cl-phenyl | |
| 49 | 2-Br, 4-Cl-phenyl | |
| 50 | 2-Br, 3-F-phenyl | |
| 51 | 2-Br, 4-F-phenyl | |
| 52 | 2-Br, 5-F-phenyl | |
| 53 | 2-Br, 6-F-phenyl | |
| 54 | 2-F, 3-Cl-phenyl | |
| 55 | 2-F, 4-Cl-phenyl | |
| 56 | 2-F, 5-Cl-phenyl | |
| 57 | 3-Cl, 4-F-phenyl | |
| 58 | 3-Cl, 5-F-phenyl | |
| 59 | 3-Cl, 4-Br-phenyl | |
| 60 | 3-Cl, 5-Br-phenyl | |
| 61 | 3-F, 4-Cl-phenyl | |
| 62 | 3-F, 4-Br-phenyl | |
| 63 | 3-Br, 4-Cl-phenyl | |
| 64 | 3-Br, 4-F-phenyl | |
| 65 | 2,4,6-$F_3$-phenyl | |
| 66 | 2,3,4,-$Cl_3$-phenyl | |

| Nr. | R | Fp(°C)/IR($cm^{-1}$) |
|---|---|---|
| 67 | 2,3,5-$Cl_3$-phenyl | |
| 68 | 2,3,6-$Cl_3$-phenyl | |
| 69 | 2,4,5-$Cl_3$-phenyl | |
| 70 | 2,4,6-$Cl_3$-phenyl | |
| 71 | 3,4,5-$Cl_3$-phenyl | |
| 72 | 2,4,6-$Br_3$-phenyl | |
| 73 | 2,6-$Cl_2$-4-Br-phenyl | |
| 74 | 2,3,4,6-$Cl_4$-phenyl | |
| 75 | 2,3,5,6-$Cl_4$-phenyl | |
| 76 | $F_5$-phenyl | |
| 77 | $Cl_5$-phenyl | |
| 78 | $Br_5$-phenyl | |
| 79 | 2-$CH_3$-phenyl | |
| 80 | 3-$CH_3$-phenyl | |
| 81 | 4-$CH_3$-phenyl | |
| 82 | 2-$C_2H_5$-phenyl | |
| 83 | 3-$C_2H_5$-phenyl | |
| 84 | 4-$C_2H_5$-phenyl | |
| 85 | 2-n-$C_3H_7$-phenyl | |
| 86 | 3-n-$C_3H_7$-phenyl | |
| 87 | 4-n-$C_3H_7$-phenyl | |
| 88 | 2-i-$C_3H_7$-phenyl | |
| 89 | 3-i-$C_3H_7$-phenyl | |
| 90 | 4-i-$C_3H_7$-phenyl | |
| 91 | 2-s-$C_4H_9$-phenyl | |
| 92 | 3-s-$C_4H_9$-phenyl | |
| 93 | 4-s-$C_4H_9$-phenyl | |
| 94 | 2-t-$C_4H_9$-phenyl | |
| 95 | 3-t-$C_4H_9$-phenyl | |
| 96 | 4-t-$C_4H_9$-phenyl | |
| 97 | 2,3-$(CH_3)_2$-phenyl | |
| 98 | 2,4-$(CH_3)_2$-phenyl | |
| 99 | 2,5-$(CH_3)_2$-phenyl | |
| 100 | 2,6-$(CH_3)_2$-phenyl | |
| 101 | 3,4-$(CH_3)_2$-phenyl | |
| 102 | 3,5-$(CH_3)_2$-phenyl | |
| 103 | 2,3,4-$(CH_3)_3$-phenyl | |
| 104 | 2,3,5-$(CH_3)_3$-phenyl | |

| Nr. | R | Fp(°C)/IR(cm$^{-1}$) |
|---|---|---|
| 105 | 2,3,6-$(CH_3)_3$-phenyl | |
| 106 | 2,4,5-$(CH_3)_3$-phenyl | |
| 107 | 2,4,6-$(CH_3)_3$-phenyl | |
| 108 | 3,4,5-$(CH_3)_3$-phenyl | |
| 109 | 2,3,4,6-$(CH_3)_4$-phenyl | |
| 110 | 2,3,5,6-$(CH_3)_4$-phenyl | |
| 111 | $(CH_3)_5$-phenyl | |
| 112 | 2,4-$(C_2H_5)_2$-phenyl | |
| 113 | 2,6-$(C_2H_5)_2$-phenyl | |
| 114 | 3,5-$(C_2H_5)_2$-phenyl | |
| 115 | 2,4,6-$(C_2H_5)_3$-phenyl | |
| 116 | 2,4-$(i\text{-}C_3H_7)_2$-phenyl | |
| 117 | 2,6-$(i\text{-}C_3H_7)_2$-phenyl | |
| 118 | 3,5-$(i\text{-}C_3H_7)_2$-phenyl | |
| 119 | 2,4,6-$(i\text{-}C_3H_7)_3$-phenyl | |
| 120 | 2,3-$(t\text{-}C_4H_9)_2$-phenyl | |
| 121 | 2,4-$(t\text{-}C_4H_9)_2$-phenyl | |
| 122 | 2,5-$(t\text{-}C_4H_9)_2$-phenyl | |
| 123 | 2,6-$(t\text{-}C_4H_9)_2$-phenyl | |
| 124 | 3,5-$(t\text{-}C_4H_9)_2$-phenyl | |
| 125 | 2,4,6-$(t\text{-}C_4H_9)_3$-phenyl | |
| 126 | 2-t-$C_4H_9$, 4-$CH_3$-phenyl | |
| 127 | 2-t-$C_4H_9$, 5-$CH_3$-phenyl | |
| 128 | 2,6-$(t\text{-}C_4H_9)_2$, 4-$CH_3$-phenyl | |
| 129 | 2-$CH_3$, 4-t-$C_4H_9$-phenyl | |
| 130 | 2-$CH_3$, 6-t-$C_4H_9$-phenyl | |
| 131 | 1-$CH_3$, 4-i-$C_3H_7$-phenyl | |
| 132 | 2-$CH_3$, 5-i-$C_3H_7$-phenyl | |
| 133 | 3-$CH_3$, 4-i-$C_3H_7$-phenyl | |
| 134 | 2-i-$C_3H_7$, 5-$CH_3$-phenyl | |
| 135 | 2,4-$(t\text{-}C_4H_9)_2$-6-i-$C_3H_7$-phenyl | |
| 136 | 2-cyclo-$C_6H_{11}$-phenyl | |
| 137 | 3-cyclo-$C_6H_{11}$-phenyl | |

| Nr. | R | Fp(°C)/IR($cm^{-1}$) |
|---|---|---|
| 138 | 4-cyclo-$C_6H_{11}$-phenyl | |
| 139 | 2,4-(cyclo-$C_6H_{11}$)$_2$-6-$CH_3$-phenyl | |
| 140 | 2-$CH_3$, 4-cyclo-$C_6H_{11}$-phenyl | |
| 141 | 2-$CH_2C_6H_5$-phenyl | |
| 142 | 3-$CH_2C_6H_5$-phenyl | |
| 143 | 4-$CH_2C_6H_5$-phenyl | |
| 144 | 2-$CH_2C_6H_5$, 4-$CH_3$-phenyl | |
| 145 | 2-$CH_3$, 4-$CH_2C_6H_5$-phenyl | |
| 146 | 2-$C_6H_5$-phenyl | |
| 147 | 3-$C_6H_5$-phenyl | |
| 148 | 4-$C_6H_5$-phenyl | |
| 149 | 4-(2-i-$C_3H_7$-$C_6H_4$)-phenyl | |
| 150 | 4-$C_6H_5$, 2,6-($CH_3$)$_2$-phenyl | |
| 151 | 2-Cl, 4-$C_6H_5$-phenyl | |
| 152 | 2-Br, 4-$C_6H_5$-phenyl | |
| 153 | 2-$C_6H_5$, 4-Cl-phenyl | |
| 154 | 2-$C_6H_5$, 4-Br-phenyl | |
| 155 | 2-$CH_2C_6H_5$, 4-Cl-phenyl | |
| 156 | 2-$CH_2C_6H_5$, 4-Br-phenyl | |
| 157 | 2-Cl, 4-$CH_2C_6H_5$-phenyl | |
| 158 | 2-Br, 4-$CH_2C_6H_5$-phenyl | |
| 159 | 2-cyclo-$C_6H_{11}$, 4-Cl-phenyl | |
| 160 | 2-cyclo-$C_6H_{11}$, 4-Br-phenyl | |
| 161 | 2-Cl, 4-cyclo-$C_6H_{11}$-phenyl | |
| 162 | 2-Br, 4-cyclo-$C_6H_{11}$-phenyl | |
| 163 | 3-$OCH_3$-phenyl | |
| 164 | 2,4-($OCH_3$)$_2$-phenyl | |
| 165 | 2-$CF_3$-phenyl | |
| 166 | 3-$CF_3$-phenyl | |
| 167 | 4-$CF_3$-phenyl | |
| 168 | 2-$NO_2$-phenyl | |
| 169 | 3-$NO_2$-phenyl | |
| 170 | 4-$NO_2$-phenyl | |
| 171 | 2-CN-phenyl | |
| 172 | 3-CN-phenyl | |

| Nr. | R | Fp(°C)/IR($cm^{-1}$) |
|---|---|---|
| 173 | 4-CN-phenyl | |
| 174 | 2-$CH_3$, 3-Cl-phenyl | |
| 175 | 2-$CH_3$, 4-Cl-phenyl | |
| 176 | 2-$CH_3$, 5-Cl-phenyl | |
| 177 | 2-$CH_3$, 6-Cl-phenyl | |
| 178 | 2-$CH_3$, 3-F-phenyl | |
| 179 | 2-$CH_3$, 4-F-phenyl | |
| 180 | 2-$CH_3$, 5-F-phenyl | |
| 181 | 2-$CH_3$, 6-F-phenyl | |
| 182 | 2-$CH_3$, 3-Br-phenyl | |
| 183 | 2-$CH_3$, 4-Br-phenyl | |
| 184 | 2-$CH_3$, 5-Br-phenyl | |
| 185 | 2-$CH_3$, 6-Br-phenyl | |
| 186 | 2-Cl, 3-$CH_3$-phenyl | |
| 187 | 2-Cl, 4-$CH_3$-phenyl | |
| 188 | 2-Cl, 5-$CH_3$-phenyl | |
| 189 | 2-Cl, 3-i-$C_3H_7$ | |
| 190 | 2-F, 3-$CH_3$-phenyl | |
| 191 | 2-F, 4-$CH_3$-phenyl | |
| 192 | 2-F, 5-$CH_3$-phenyl | |
| 193 | 2-Br, 3-$CH_3$-phenyl | |
| 194 | 2-Br, 4-$CH_3$-phenyl | |
| 195 | 3-$CH_3$, 4-Cl-phenyl | |
| 196 | 3-$CH_3$, 5-Cl-phenyl | |
| 197 | 2-Br, 5-$CH_3$-phenyl | |
| 198 | 3-$CH_3$, 4-F-phenyl | |
| 199 | 3-$CH_3$, 5-F-phenyl | |
| 200 | 3-$CH_3$, 4-Br-phenyl | |
| 201 | 3-$CH_3$, 5-Br-phenyl | |
| 202 | 3-F, 4-$CH_3$-phenyl | |
| 203 | 3-Cl, 4-$CH_3$-phenyl | |
| 204 | 3-Br, 4-$CH_3$-phenyl | |
| 205 | 2-Cl, 4,5-$(CH_3)_2$-phenyl | |
| 206 | 2-Br, 4,5-$(CH_3)_2$-phenyl | |
| 207 | 2-Cl, 3,5-$(CH_3)_2$-phenyl | |
| 208 | 2-Br, 3,5-$(CH_3)_2$-phenyl | |
| 209 | 2,6-$Cl_2$, 4-$CH_3$-phenyl | |
| 210 | 2,6-$F_2$, 4-$CH_3$-phenyl | |

| Nr. | R | Fp($^{o}C$)/IR($cm^{-1}$) |
|---|---|---|
| 211 | 2,6-$Br_2$, 4-$CH_3$-phenyl | |
| 212 | 2,4-$Cl_2$, 6-$CH_3$-phenyl | |
| 213 | 2,4-$F_2$, 6-$CH_3$-phenyl | |
| 214 | 2,4-$Br_2$, 6-$CH_3$-phenyl | |
| 215 | 2,6-$(CH_3)_2$, 4-F-phenyl | |
| 216 | 2,6-$(CH_3)_2$, 4-Cl-phenyl | |
| 217 | 2,6-$(CH_3)_2$, 4-Br-phenyl | |
| 218 | 3,5-$(CH_3)_2$, 4-F-phenyl | |
| 219 | 3,5-$(CH_3)_2$, 4-Cl-phenyl | |
| 220 | 3,5-$(CH_3)_2$, 4-Br-phenyl | |
| 221 | 2,3,6-$(CH_3)_3$, 4-F-phenyl | |
| 222 | 2,3,6-$(CH_3)_3$, 4-Cl-phenyl | |
| 223 | 2,3,6-$(CH_3)_3$, 4-Br-phenyl | |
| 224 | 2,4-$(CH_3)_2$, 6-F-phenyl | |
| 225 | 2,4-$(CH_3)_2$, 6-Cl-phenyl | |
| 226 | 2,4-$(CH_3)_2$, 6-Br-phenyl | |
| 227 | 2-i-$C_3H_7$, 4-Cl, 5-$CH_3$-phenyl | |
| 228 | 2-Cl, 4-$NO_2$-phenyl | |
| 229 | 2-$NO_2$, 4-Cl-phenyl | |
| 230 | 2-$OCH_3$, 5-$NO_2$-phenyl | |
| 231 | 2,4-$Cl_2$, 5-$NO_2$-phenyl | |
| 232 | 2,4-$Cl_2$, 6-$NO_2$-phenyl | |
| 233 | 2,6-$Cl_2$, 4-$NO_2$-phenyl | |
| 234 | 2,6-$Br_2$, 4-$NO_2$-phenyl | |
| 235 | 2,6-$J_2$, 4-$NO_2$-phenyl | |
| 236 | 2-$C_6H_5O$-phenyl | |
| 237 | 3-$C_6H_5O$-phenyl | |
| 238 | 4-$C_6H_5O$-phenyl | |
| 239 | 3-t-$C_4H_9O$-phenyl | |
| 240 | 4-t-$C_4H_9O$-phenyl | |
| 241 | 1-Naphthyl | |
| 242 | 2-Naphthyl | |
| 243 | 2-Pyridyl | |
| 244 | 6-Methyl-2-pyridyl | |
| 245 | 6-Ethyl-2-pyridyl | |
| 246 | 6-n-Propyl-2-pyridyl | |

| Nr. | R | Fp(°C)/IR($cm^{-1}$) |
|---|---|---|
| 247 | 6-iso-Propyl-2-pyridyl | |
| 248 | 6-n-Butyl-2-pyridyl | |
| 249 | 6-tert.-Butyl-2-pyridyl | |
| 250 | 6-n-Pentyl-2-pyridyl | |
| 251 | 6-n-Hexyl-2-pyridyl | |
| 252 | 6-Phenyl-2-pyridyl | |
| 253 | 6-Benzyl-2-pyridyl | |
| 254 | 6-Trifluormethyl-2-pyridyl | |
| 255 | 6-Methoxy-2-pyridyl | |
| 256 | 6-Chloro-2-pyridyl | |
| 257 | 3,6-Dimethyl-2-pyridyl | |
| 258 | 3,6-Diethyl-2-pyridyl | |
| 259 | 4,6-Dimethyl-2-pyridyl | |
| 260 | 5,6-Dimethyl-2-pyridyl | |
| 261 | 4-Phenyl-6-methyl-2-pyridyl | |
| 262 | 4,6-Diphenyl-2-pyridyl | |
| 263 | 3,4-Dichloro-6-methyl-2-pyridyl | |
| 264 | 3,4,5-Trichloro-6-phenyl-2-pyridyl | |
| 265 | 4-Trifluoromethyl-6-methyl-2-pyridyl | |
| 266 | 3-Cyano-6-methyl-2-pyridyl | |
| 267 | 3-Cyano-6-ethyl-2-pyridyl | |
| 268 | 3-Cyano-6-n-propyl-2-pyridyl | |
| 269 | 3-Cyano-6-iso-propyl-2-pyridyl | |
| 270 | 3-Cyano-6-cyclo-propyl-2-pyridyl | |
| 271 | 3-Cyano-6-n-butyl-2-pyridyl | |
| 272 | 3-Cyano-6-tert.-butyl-2-pyridyl | |
| 273 | 3-Cyano-6-cyclo-hexyl-2-pyridyl | |
| 274 | 3-Cyano-6-phenyl-2-pyridyl | |
| 275 | 3-Cyano-4,6-dimethyl-2-pyridyl | |
| 276 | 3,5,6-Trichloro-2-pyridyl | |
| 277 | 5-Trifluoromethyl-2-pyridyl | |
| 278 | 3-Chloro-5-trifluoromethyl-2-pyridyl | |
| 279 | 2-Chinolyl | |
| 280 | 3-Methyl-2-chinolyl | |
| 281 | 4-Methyl-2-chinolyl | |

| Nr. | R | Fp(°C)/IR($cm^{-1}$) |
|---|---|---|
| 282 | 4-Ethyl-2-chinolyl | |
| 283 | 4-Phenyl-2-chinolyl | |
| 284 | 6-Methyl-2-chinolyl | |
| 285 | 6-Chloro-2-chinolyl | |
| 286 | 8-Methyl-2-chinolyl | |
| 287 | 8-Chloro-2-chinolyl | |
| 288 | 3,4-Dimethyl-2-chinolyl | |
| 289 | 4-Methyl-8-methoxy-2-chinolyl | |
| 290 | 4-Phenyl-8-ethoxy-2-chinolyl | |
| 291 | 4-Methyl-8-chloro-2-chinolyl | |
| 292 | 4-Methyl-8-fluoro-2-chinolyl | |
| 293 | 4-Chinolyl | |
| 294 | 2-Methyl-4-chinolyl | |
| 295 | 2-Trifluoromethyl-4-chinolyl | |
| 296 | 2-iso-Propyl-4-chinolyl | |
| 297 | 2-n-Pentyl-4-chinolyl | |
| 298 | 2-Phenyl-4-chinolyl | |
| 299 | 2,6-Dimethyl-4-chinolyl | |
| 300 | 2-Methyl-6-chloro-4-chinolyl | |
| 301 | 2-Methyl-6-fluoro-4-chinolyl | |
| 302 | 8-Chinolyl | |
| 303 | 2-Methyl-8-chinolyl | |
| 304 | 5,7-Dichloro-8-chinolyl | |
| 305 | 4,6-Dimethyl-2-pyrimidinyl | |
| 306 | 4-Trifluormethyl-2-pyrimidinyl | |
| 307 | 4,5,6-Trimethyl-2-pyrimidinyl | |
| 308 | 4-Benzyl-6-methyl-2-pyrimidinyl | |
| 309 | 4-Methyl-6-phenyl-2-pyrimidinyl | |
| 310 | 4,6-Dimethyl-5-chloro-2-pyrimidinyl | |
| 311 | 2,6-Dimethyl-4-pyrimidinyl | |
| 312 | 2,6-Bis-(trifluormethyl)-4-pyrimidinyl | |
| 313 | 2-iso-Propyl-6-methyl-4-pyrimidinyl | |
| 314 | 2-cyclo-Propyl-6-methyl-4-pyrimidinyl | |
| 315 | 2-Methyl-6-methoxymethyl-4-pyrimidinyl | |
| 316 | 2-iso-Propyl-6-methoxymethyl-4-pyrimidinyl | |
| 317 | 2-Phenyl-4-pyrimidinyl | |

| Nr. | R | Fp(°C)/IR($cm^{-1}$) |
|---|---|---|
| 318 | 3,5-Dimethyl-4-pyrimidinyl | |
| 319 | 2-Methyl-6-trifluoromethyl-4-pyrimidinyl | |
| 320 | 2-n-Propyl-6-trifluoromethyl-4-pyrimidinyl | |
| 321 | 2-iso-Propyl-6-trifluoromethyl-4-pyrimidinyl | |
| 322 | 2-Methyl-5-chloro-6-trifluoromethyl-4-pyrimidinyl | |
| 323 | 2-n-Propyl-5-chloro-6-trifluoromethyl-4-pyrimidinyl | |
| 324 | 2-iso-Propyl-5-chloro-6-trifluoromethyl-4-pyrimidinyl | |
| 325 | 2-tert.-Butyl-5-chloro-6-trifluoromethyl-4-pyrimidinyl | |

Tabelle 4

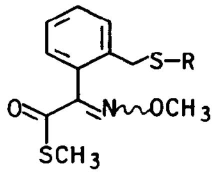

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | 1661,1451,1443,1139,1115,1043,1035,1024,846,749 |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |

| Nr. | R | Fp($^{o}$C)/IR($cm^{-1}$) |
|---|---|---|
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |
| 35 | 2,4-$Br_2$-phenyl | |
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |

| Nr. | R | Fp(°C)/IR($cm^{-1}$) |
|---|---|---|
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |
| 77 | $Br_5$-phenyl | |
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |

| Nr. | R | Fp($^{o}C$)/IR($cm^{-1}$) |
|---|---|---|
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-(i-$C_3H_7)_2$-phenyl | |
| 116 | 2,6-(i-$C_3H_7)_2$-phenyl | |
| 117 | 3,5-(i-$C_3H_7)_2$-phenyl | |
| 118 | 2,4,6-(i-$C_3H_7)_3$-phenyl | |
| 119 | 2,3-(t-$C_4H_9)_2$-phenyl | |
| 120 | 2,4-(t-$C_4H_9)_2$-phenyl | |
| 121 | 2,5-(t-$C_4H_9)_2$-phenyl | |
| 122 | 2,6-(t-$C_4H_9)_2$-phenyl | |
| 123 | 3,5-(t-$C_4H_9)_2$-phenyl | |
| 124 | 2,4,6-(t-$C_4H_9)_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-(t-$C_4H_9)_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-(t-$C_4H_9)_2$-6-i-$C_3H_7$-phenyl | |
| 135 | 2-cyclo-$C_6H_{11}$-phenyl | |
| 136 | 3-cyclo-$C_6H_{11}$-phenyl | |

| Nr. | R | Fp(°C)/IR($cm^{-1}$) |
|---|---|---|
| 137 | 4-cyclo-$C_6H_{11}$-phenyl | |
| 138 | 2,4-(cyclo-$C_6H_{11}$)$_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-($CH_3$)$_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |
| 161 | 2-Br, 4-cyclo-$C_6H_{11}$-phenyl | |
| 162 | 3-$OCH_3$-phenyl | |
| 163 | 2,4-($OCH_3$)$_2$-phenyl | |
| 164 | 2-$CF_3$-phenyl | |
| 165 | 3-$CF_3$-phenyl | |
| 166 | 4-$CF_3$-phenyl | |
| 167 | 2-$NO_2$-phenyl | |
| 168 | 3-$NO_2$-phenyl | |
| 169 | 4-$NO_2$-phenyl | |
| 170 | 2-CN-phenyl | |
| 171 | 3-CN-phenyl | |

| Nr. | R | Fp(°C)/IR($cm^{-1}$) |
|---|---|---|
| 172 | 4-CN-phenyl | |
| 173 | 2-$CH_3$, 3-Cl-phenyl | |
| 174 | 2-$CH_3$, 4-Cl-phenyl | |
| 175 | 2-$CH_3$, 5-Cl-phenyl | |
| 176 | 2-$CH_3$, 6-Cl-phenyl | |
| 177 | 2-$CH_3$, 3-F-phenyl | |
| 178 | 2-$CH_3$, 4-F-phenyl | |
| 179 | 2-$CH_3$, 5-F-phenyl | |
| 180 | 2-$CH_3$, 6-F-phenyl | |
| 181 | 2-$CH_3$, 3-Br-phenyl | |
| 182 | 2-$CH_3$, 4-Br-phenyl | |
| 183 | 2-$CH_3$, 5-Br-phenyl | |
| 184 | 2-$CH_3$, 6-Br-phenyl | |
| 185 | 2-Cl, 3-$CH_3$-phenyl | |
| 186 | 2-Cl, 4-$CH_3$-phenyl | |
| 187 | 2-Cl, 5-$CH_3$-phenyl | |
| 188 | 2-Cl, 3-i-$C_3H_7$ | |
| 189 | 2-F, 3-$CH_3$-phenyl | |
| 190 | 2-F, 4-$CH_3$-phenyl | |
| 191 | 2-F, 5-$CH_3$-phenyl | |
| 192 | 2-Br, 3-$CH_3$-phenyl | |
| 193 | 2-Br, 4-$CH_3$-phenyl | |
| 194 | 3-$CH_3$, 4-Cl-phenyl | |
| 195 | 3-$CH_3$, 5-Cl-phenyl | |
| 196 | 2-Br, 5-$CH_3$-phenyl | |
| 197 | 3-$CH_3$, 4-F-phenyl | |
| 198 | 3-$CH_3$, 5-F-phenyl | |
| 199 | 3-$CH_3$, 4-Br-phenyl | |
| 200 | 3-$CH_3$, 5-Br-phenyl | |
| 201 | 3-F, 4-$CH_3$-phenyl | |
| 202 | 3-Cl, 4-$CH_3$-phenyl | |
| 203 | 3-Br, 4-$CH_3$-phenyl | |
| 204 | 2-Cl, 4,5-$(CH_3)_2$-phenyl | |
| 205 | 2-Br, 4,5-$(CH_3)_2$-phenyl | |
| 206 | 2-Cl, 3,5-$(CH_3)_2$-phenyl | |
| 207 | 2-Br, 3,5-$(CH_3)_2$-phenyl | |
| 208 | 2,6-$Cl_2$, 4-$CH_3$-phenyl | |
| 209 | 2,6-$F_2$, 4-$CH_3$-phenyl | |

| Nr. | R | Fp($^{o}$C)/IR($cm^{-1}$) |
|---|---|---|
| 210 | 2,6-$Br_2$, 4-$CH_3$-phenyl | |
| 211 | 2,4-$Cl_2$, 6-$CH_3$-phenyl | |
| 212 | 2,4-$F_2$, 6-$CH_3$-phenyl | |
| 213 | 2,4-$Br_2$, 6-$CH_3$-phenyl | |
| 214 | 2,6-$(CH_3)_2$, 4-F-phenyl | |
| 215 | 2,6-$(CH_3)_2$, 4-Cl-phenyl | |
| 216 | 2,6-$(CH_3)_2$, 4-Br-phenyl | |
| 217 | 3,5-$(CH_3)_2$, 4-F-phenyl | |
| 218 | 3,5-$(CH_3)_2$, 4-Cl-phenyl | |
| 219 | 3,5-$(CH_3)_2$, 4-Br-phenyl | |
| 220 | 2,3,6-$(CH_3)_3$, 4-F-phenyl | |
| 221 | 2,3,6-$(CH_3)_3$, 4-Cl-phenyl | |
| 222 | 2,3,6-$(CH_3)_3$, 4-Br-phenyl | |
| 223 | 2,4-$(CH_3)_2$, 6-F-phenyl | |
| 224 | 2,4-$(CH_3)_2$, 6-Cl-phenyl | |
| 225 | 2,4-$(CH_3)_2$, 6-Br-phenyl | |
| 226 | 2-i-$C_3H_7$, 4-Cl, 5-$CH_3$-phenyl | |
| 227 | 2-Cl, 4-$NO_2$-phenyl | |
| 228 | 2-$NO_2$, 4-Cl-phenyl | |
| 229 | 2-$OCH_3$, 5-$NO_2$-phenyl | |
| 230 | 2,4-$Cl_2$, 5-$NO_2$-phenyl | |
| 231 | 2,4-$Cl_2$, 6-$NO_2$-phenyl | |
| 232 | 2,6-$Cl_2$, 4-$NO_2$-phenyl | |
| 233 | 2,6-$Br_2$, 4-$NO_2$-phenyl | |
| 234 | 2,6-$J_2$, 4-$NO_2$-phenyl | |
| 235 | 2-$C_6H_5O$-phenyl | |
| 236 | 3-$C_6H_5O$-phenyl | |
| 237 | 4-$C_6H_5O$-phenyl | |
| 238 | 3-t-$C_4H_9O$-phenyl | |
| 239 | 4-t-$C_4H_9O$-phenyl | |
| 240 | 1-Naphthyl | |
| 241 | 2-Naphthyl | |
| 242 | 2-Pyridyl | |
| 243 | 6-Methyl-2-pyridyl | 89-94°C |
| 244 | 6-Ethyl-2-pyridyl | |
| 245 | 6-n-Propyl-2-pyridyl | |

| Nr. | R | Fp(°C)/IR($cm^{-1}$) |
|---|---|---|
| 246 | 6-iso-Propyl-2-pyridyl | |
| 247 | 6-n-Butyl-2-pyridyl | |
| 248 | 6-tert.-Butyl-2-pyridyl | |
| 249 | 6-n-Pentyl-2-pyridyl | |
| 250 | 6-n-Hexyl-2-pyridyl | |
| 251 | 6-Phenyl-2-pyridyl | |
| 252 | 6-Benzyl-2-pyridyl | |
| 253 | 6-Trifluormethyl-2-pyridyl | |
| 254 | 6-Methoxy-2-pyridyl | |
| 255 | 6-Chloro-2-pyridyl | |
| 256 | 3,6-Dimethyl-2-pyridyl | |
| 257 | 3,6-Diethyl-2-pyridyl | |
| 258 | 4,6-Dimethyl-2-pyridyl | |
| 259 | 5,6-Dimethyl-2-pyridyl | |
| 260 | 4-Phenyl-6-methyl-2-pyridyl | |
| 261 | 4,6-Diphenyl-2-pyridyl | |
| 262 | 3,4-Dichloro-6-methyl-2-pyridyl | |
| 263 | 3,4,5-Trichloro-6-phenyl-2-pyridyl | |
| 264 | 4-Trifluoromethyl-6-methyl-2-pyridyl | |
| 265 | 3-Cyano-6-methyl-2-pyridyl | |
| 266 | 3-Cyano-6-ethyl-2-pyridyl | |
| 267 | 3-Cyano-6-n-propyl-2-pyridyl | |
| 268 | 3-Cyano-6-iso-propyl-2-pyridyl | |
| 269 | 3-Cyano-6-cyclo-propyl-2-pyridyl | |
| 270 | 3-Cyano-6-n-butyl-2-pyridyl | |
| 271 | 3-Cyano-6-tert.-butyl-2-pyridyl | |
| 272 | 3-Cyano-6-cyclo-hexyl-2-pyridyl | |
| 273 | 3-Cyano-6-phenyl-2-pyridyl | |
| 274 | 3-Cyano-4,6-dimethyl-2-pyridyl | |
| 275 | 3,5,6-Trichloro-2-pyridyl | |
| 276 | 5-Trifluoromethyl-2-pyridyl | |
| 277 | 3-Chloro-5-trifluoromethyl-2-pyridyl | |
| 278 | 2-Chinolyl | |
| 279 | 3-Methyl-2-chinolyl | |
| 280 | 4-Methyl-2-chinolyl | |

| Nr. | R | Fp(°C)/IR($cm^{-1}$) |
|---|---|---|
| 281 | 4-Ethyl-2-chinolyl | |
| 282 | 4-Phenyl-2-chinolyl | |
| 283 | 6-Methyl-2-chinolyl | |
| 284 | 6-Chloro-2-chinolyl | |
| 285 | 8-Methyl-2-chinolyl | |
| 286 | 8-Chloro-2-chinolyl | |
| 287 | 3,4-Dimethyl-2-chinolyl | |
| 288 | 4-Methyl-8-methoxy-2-chinolyl | |
| 289 | 4-Phenyl-8-ethoxy-2-chinolyl | |
| 290 | 4-Methyl-8-chloro-2-chinolyl | |
| 291 | 4-Methyl-8-fluoro-2-chinolyl | |
| 292 | 4-Chinolyl | |
| 293 | 2-Methyl-4-chinolyl | |
| 294 | 2-Trifluoromethyl-4-chinolyl | |
| 295 | 2-iso-Propyl-4-chinolyl | |
| 296 | 2-n-Pentyl-4-chinolyl | |
| 297 | 2-Phenyl-4-chinolyl | |
| 298 | 2,6-Dimethyl-4-chinolyl | |
| 299 | 2-Methyl-6-chloro-4-chinolyl | |
| 300 | 2-Methyl-6-fluoro-4-chinolyl | |
| 301 | 8-Chinolyl | |
| 302 | 2-Methyl-8-chinolyl | |
| 303 | 5,7-Dichloro-8-chinolyl | |
| 304 | 4,6-Dimethyl-2-pyrimidinyl | |
| 305 | 4-Trifluormethyl-2-pyrimidinyl | |
| 306 | 4,5,6-Trimethyl-2-pyrimidinyl | |
| 307 | 4-Benzyl-6-methyl-2-pyrimidinyl | |
| 308 | 4-Methyl-6-phenyl-2-pyrimidinyl | |
| 309 | 4,6-Dimethyl-5-chloro-2-pyrimidinyl | |
| 310 | 2,6-Dimethyl-4-pyrimidinyl | |
| 311 | 2,6-Bis-(trifluormethyl)-4-pyrimidinyl | |
| 312 | 2-iso-Propyl-6-methyl-4-pyrimidinyl | |
| 313 | 2-cyclo-Propyl-6-methyl-4-pyrimidinyl | |
| 314 | 2-Methyl-6-methoxymethyl-4-pyrimidinyl | |
| 315 | 2-iso-Propyl-6-methoxymethyl-4-pyrimidinyl | |
| 316 | 2-Phenyl-4-pyrimidinyl | |

| Nr. | R | Fp($^{o}C$)/IR($cm^{-1}$) |
|---|---|---|
| 317 | 3,5-Dimethyl-4-pyrimidinyl | |
| 318 | 2-Methyl-6-trifluoromethyl-4-pyrimidinyl | |
| 319 | 2-n-Propyl-6-trifluoromethyl-4-pyrimidinyl | |
| 320 | 2-iso-Propyl-6-trifluoromethyl-4-pyrimidinyl | |
| 321 | 2-Methyl-5-chloro-6-trifluoromethyl-4-pyrimidinyl | |
| 322 | 2-n-Propyl-5-chloro-6-trifluoromethyl-4-pyrimidinyl | |
| 323 | 2-iso-Propyl-5-chloro-6-trifluoromethyl-4-pyrimidinyl | |
| 324 | 2-tert.-Butyl-5-chloro-6-trifluoromethyl-4-pyrimidinyl | |
| 325 | 6-EtO-benzothiazol-2-yl | |
| 326 | benzoxazol-2-yl | |
| 327 | 1-Me-imidazol-2-yl | |
| 328 | 4-But-1H-imidazol-2-yl | |
| 329 | 2-thiazolin-2-yl | |
| 330 | 5-$CF_3$-benzimidazol-2-yl | |
| 331 | 1-Ph-tetrazol-5-yl | |
| 332 | 5-$CF_3$-benzothiazol-2-yl | |
| 333 | 4,4-$Me_2$-5-methylene-2-thiazolin-2-yl | |
| 334 | 6-Cl-4-Me-benzothiazol-2-yl | |
| 335 | 5-Me-benzothiazol-2-yl | |
| 336 | 4-Cl-benzothiazol-2-yl | |
| 337 | 1-(3-$NO_2$-Ph)tetrazol-5-yl | |
| 338 | 2-thienyl | |
| 339 | 5-Me-benzoxazol-2-yl | |
| 340 | 7-Cl-benzothiazol-2-yl | |
| 341 | 5,6-$Cl_3$-1H-benzimidazol-2-yl | |
| 342 | 5-Cl-benzoxazol-2-yl | |
| 343 | 6-Cl-benzothiazol-2-yl | |
| 344 | 2-Ph-thiazol-2-yl | |
| 345 | 3-CN-4,6-$Me_2$-2-pyridyl | |
| 346 | 1-Ph-1,2,4-triazol-3-yl | |
| 347 | 1-Pri-benzimidazol-2-yl | |
| 348 | 5-Br-benzothiazol-2-yl | |
| 349 | 5-Br-1H-benzimidazol-2-yl | |
| 350 | 7-Cl-4-MeO-benzothiazol-2-yl | |
| 351 | 1H-benzimidazol-2-yl | |

| Nr. | R | Fp($^{o}C$)/IR($cm^{-1}$) |
|---|---|---|
| 352 | 5-Cl-benzothiazol-2-yl | |
| 353 | 5-$NO_2$-benzoxazol-2-yl | |
| 354 | 5-t-Bu-benzoxazol-2-yl | |
| 355 | 4,6,7-$Cl_3$-benzothiozol-2-yl | |
| 356 | 5-Ph-thiazol-2-yl | |
| 357 | 5,7-$Me_2$-benzoxazol-2-yl | |
| 358 | 6-Me-benzoxazol-2-yl | |
| 359 | 1,2,4-triazin-3-yl | |
| 360 | 6-Pr-benzothiazol-2-yl | |
| 361 | 6-PhO-benzothiazol-2-yl | |
| 362 | 4-(4-Cl-Ph)-thiazol-2-yl | |
| 363 | 4-(4-Me-Ph)-thiazol-2-yl | |
| 364 | 5-Me-4-Ph-thiazol-2-yl | |
| 365 | 5-Cl-1-H-benzimidazol-2-yl | |
| 366 | 5-Ph-1,2,4-triazol-3-yl | |
| 367 | 3-Ph-1,2,4-thiadiazol-5-yl | |

Tabelle 5

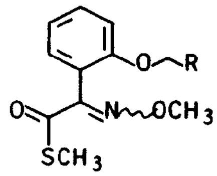

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp(°C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 6

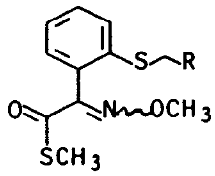

| Nr. | R | Fp($^{o}C$)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp($^oC$)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 7

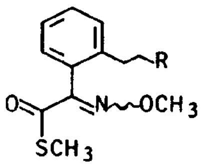

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp($^{o}$C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 8

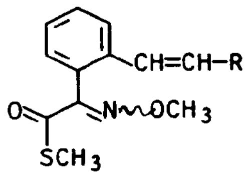

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp($^{o}C$)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 9

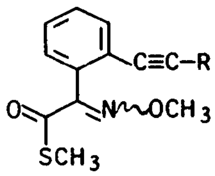

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_2$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp(°C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 10

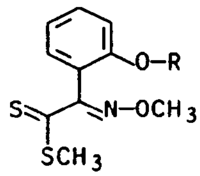

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |
| 35 | 2,4-$Br_2$-phenyl | |

| Nr. | R | Fp(°C)/IR($cm^{-1}$) |
|---|---|---|
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |
| 77 | $Br_5$-phenyl | |

| Nr. | R | Fp(°C)/IR($cm^{-1}$) |
|---|---|---|
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-$(i-C_3H_7)_2$-phenyl | |
| 116 | 2,6-$(i-C_3H_7)_2$-phenyl | |
| 117 | 3,5-$(i-C_3H_7)_2$-phenyl | |
| 118 | 2,4,6-$(i-C_3H_7)_3$-phenyl | |
| 119 | 2,3-$(t-C_4H_9)_2$-phenyl | |

| Nr. | R | Fp(°C)/IR($cm^{-1}$) |
|---|---|---|
| 120 | 2,4-$(t-C_4H_9)_2$-phenyl | |
| 121 | 2,5-$(t-C_4H_9)_2$-phenyl | |
| 122 | 2,6-$(t-C_4H_9)_2$-phenyl | |
| 123 | 3,5-$(t-C_4H_9)_2$-phenyl | |
| 124 | 2,4,6-$(t-C_4H_9)_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-$(t-C_4H_9)_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-$(t-C_4H_9)_2$-6-i-$C_3H_7$-phenyl | |
| 135 | 2-cyclo-$C_6H_{11}$-phenyl | |
| 136 | 3-cyclo-$C_6H_{11}$-phenyl | |
| 137 | 4-cyclo-$C_6H_{11}$-phenyl | |
| 138 | 2,4-(cyclo-$C_6H_{11})_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-$(CH_3)_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |
| 161 | 2-Br, 4-cyclo-$C_6H_{11}$-phenyl | |

| Nr. | R | Fp(°C)/IR($cm^{-1}$) |
|---|---|---|
| 162 | 2-$OCH_3$-phenyl | |
| 163 | 3-$OCH_3$-phenyl | |
| 164 | 4-$OCH_3$-phenyl | |
| 165 | 2,4-$(OCH_3)_2$-phenyl | |
| 166 | 2-$CF_3$-phenyl | |
| 167 | 3-$CF_3$-phenyl | |
| 168 | 4-$CF_3$-phenyl | |
| 169 | 2-$NO_2$-phenyl | |
| 170 | 3-$NO_2$-phenyl | |
| 171 | 4-$NO_2$-phenyl | |
| 172 | 2-CN-phenyl | |
| 173 | 3-CN-phenyl | |
| 174 | 4-CN-phenyl | |
| 175 | 2-$CH_3$, 3-Cl-phenyl | |
| 176 | 2-$CH_3$, 4-Cl-phenyl | |
| 177 | 2-$CH_3$, 5-Cl-phenyl | |
| 178 | 2-$CH_3$, 6-Cl-phenyl | |
| 179 | 2-$CH_3$, 3-F-phenyl | |
| 180 | 2-$CH_3$, 4-F-phenyl | |
| 181 | 2-$CH_3$, 5-F-phenyl | |
| 182 | 2-$CH_3$, 6-F-phenyl | |
| 183 | 2-$CH_3$, 3-Br-phenyl | |
| 184 | 2-$CH_3$, 4-Br-phenyl | |
| 185 | 2-$CH_3$, 5-Br-phenyl | |
| 186 | 2-$CH_3$, 6-Br-phenyl | |
| 187 | 2-Cl, 3-$CH_3$-phenyl | |
| 188 | 2-Cl, 4-$CH_3$-phenyl | |
| 189 | 2-Cl, 5-$CH_3$-phenyl | |
| 190 | 2-Cl, 3-i-$C_3H_7$ | |
| 191 | 2-F, 3-$CH_3$-phenyl | |
| 192 | 2-F, 4-$CH_3$-phenyl | |
| 193 | 2-F, 5-$CH_3$-phenyl | |
| 194 | 2-Br, 3-$CH_3$-phenyl | |
| 195 | 2-Br, 4-$CH_3$-phenyl | |
| 196 | 3-$CH_3$, 4-Cl-phenyl | |
| 197 | 3-$CH_3$, 5-Cl-phenyl | |
| 198 | 2-Br, 5-$CH_3$-phenyl | |
| 199 | 3-$CH_3$, 4-F-phenyl | |
| 200 | 3-$CH_3$, 5-F-phenyl | |
| 201 | 3-$CH_3$, 4-Br-phenyl | |
| 202 | 3-$CH_3$, 5-Br-phenyl | |
| 203 | 3-F, 4-$CH_3$-phenyl | |

| Nr. | R | Fp(°C)/IR($cm^{-1}$) |
|---|---|---|
| 204 | 3-Cl, 4-$CH_3$-phenyl | |
| 205 | 3-Br, 4-$CH_3$-phenyl | |
| 206 | 2-Cl, 4,5-$(CH_3)_2$-phenyl | |
| 207 | 2-Br, 4,5-$(CH_3)_2$-phenyl | |
| 208 | 2-Cl, 3,5-$(CH_3)_2$-phenyl | |
| 209 | 2-Br, 3,5-$(CH_3)_2$-phenyl | |
| 210 | 2,6-$Cl_2$, 4-$CH_3$-phenyl | |
| 211 | 2,6-$F_2$, 4-$CH_3$-phenyl | |
| 212 | 2,6-$Br_2$, 4-$CH_3$-phenyl | |
| 213 | 2,4-$Cl_2$, 6-$CH_3$-phenyl | |
| 214 | 2,4-$F_2$, 6-$CH_3$-phenyl | |
| 215 | 2,4-$Br_2$, 6-$CH_3$-phenyl | |
| 216 | 2,6-$(CH_3)_2$, 4-F-phenyl | |
| 217 | 2,6-$(CH_3)_2$, 4-Cl-phenyl | |
| 218 | 2,6-$(CH_3)_2$, 4-Br-phenyl | |
| 219 | 3,5-$(CH_3)_2$, 4-F-phenyl | |
| 220 | 3,5-$(CH_3)_2$, 4-Cl-phenyl | |
| 221 | 3,5-$(CH_3)_2$, 4-Br-phenyl | |
| 222 | 2,3,6-$(CH_3)_3$, 4-F-phenyl | |
| 223 | 2,3,6-$(CH_3)_3$, 4-Cl-phenyl | |
| 224 | 2,3,6-$(CH_3)_3$, 4-Br-phenyl | |
| 225 | 2,4-$(CH_3)_2$, 6-F-phenyl | |
| 226 | 2,4-$(CH_3)_2$, 6-Cl-phenyl | |
| 227 | 2,4-$(CH_3)_2$, 6-Br-phenyl | |
| 228 | 2-i-$C_3H_7$, 4-Cl, 5-$CH_3$-phenyl | |
| 229 | 2-Cl, 4-$NO_2$-phenyl | |
| 230 | 2-$NO_2$, 4-Cl-phenyl | |
| 231 | 2-$OCH_3$, 5-$NO_2$-phenyl | |
| 232 | 2,4-$Cl_2$, 5-$NO_2$-phenyl | |
| 233 | 2,4-$Cl_2$, 6-$NO_2$-phenyl | |
| 234 | 2,6-$Cl_2$, 4-$NO_2$-phenyl | |
| 235 | 2,6-$Br_2$, 4-$NO_2$-phenyl | |
| 236 | 2,6-$J_2$, 4-$NO_2$-phenyl | |
| 237 | 2-$C_6H_5O$-phenyl | |
| 238 | 3-$C_6H_5O$-phenyl | |
| 239 | 4-$C_6H_5O$-phenyl | |
| 240 | 3-t-$C_4H_9O$-phenyl | |
| 241 | 4-t-$C_4H_9O$-phenyl | |
| 242 | 1-Naphthyl | . |
| 243 | 2-Naphthyl | |
| 244 | 2-Pyridyl | |
| 245 | 6-Methyl-2-pyridyl | |

| Nr. | R | Fp(°C)/IR($cm^{-1}$) |
|---|---|---|
| 246 | 6-Ethyl-2-pyridyl | |
| 247 | 6-n-Propyl-2-pyridyl | |
| 248 | 6-iso-Propyl-2-pyridyl | |
| 249 | 6-n-Butyl-2-pyridyl | |
| 250 | 6-tert.-Butyl-2-pyridyl | |
| 251 | 6-n-Pentyl-2-pyridyl | |
| 252 | 6-n-Hexyl-2-pyridyl | |
| 253 | 6-Phenyl-2-pyridyl | |
| 254 | 6-Benzyl-2-pyridyl | |
| 255 | 6-Trifluormethyl-2-pyridyl | |
| 256 | 6-Methoxy-2-pyridyl | |
| 257 | 6-Chloro-2-pyridyl | |
| 258 | 3,6-Dimethyl-2-pyridyl | |
| 259 | 3,6-Diethyl-2-pyridyl | |
| 260 | 4,6-Dimethyl-2-pyridyl | |
| 261 | 5,6-Dimethyl-2-pyridyl | |
| 262 | 4-Phenyl-6-methyl-2-pyridyl | |
| 263 | 4,6-Diphenyl-2-pyridyl | |
| 264 | 3,4-Dichloro-6-methyl-2-pyridyl | |
| 265 | 3,4,5-Trichloro-6-phenyl-2-pyridyl | |
| 266 | 4-Trifluoromethyl-6-methyl-2-pyridyl | |
| 267 | 3-Cyano-6-methyl-2-pyridyl | |
| 268 | 3-Cyano-6-ethyl-2-pyridyl | |
| 269 | 3-Cyano-6-n-propyl-2-pyridyl | |
| 270 | 3-Cyano-6-iso-propyl-2-pyridyl | |
| 271 | 3-Cyano-6-cyclo-propyl-2-pyridyl | |
| 272 | 3-Cyano-6-n-butyl-2-pyridyl | |
| 273 | 3-Cyano-6-tert.-butyl-2-pyridyl | |
| 274 | 3-Cyano-6-cyclo-hexyl-2-pyridyl | |
| 275 | 3-Cyano-6-phenyl-2-pyridyl | |
| 276 | 3-Cyano-4,6-dimethyl-2-pyridyl | |
| 277 | 3,5,6-Trichloro-2-pyridyl | |
| 278 | 5-Trifluoromethyl-2-pyridyl | |
| 279 | 3-Chloro-5-trifluoromethyl-2-pyridyl | |
| 280 | 2-Chinolyl | |
| 281 | 3-Methyl-2-chinolyl | |
| 282 | 4-Methyl-2-chinolyl | |
| 283 | 4-Ethyl-2-chinolyl | |
| 284 | 4-Phenyl-2-chinolyl | |
| 285 | 6-Methyl-2-chinolyl | |
| 286 | 6-Chloro-2-chinolyl | |
| 287 | 8-Methyl-2-chinolyl | |

| Nr. | R | Fp($^{o}$C)/IR(cm$^{-1}$) |
|---|---|---|
| 288 | 8-Chloro-2-chinolyl | |
| 289 | 3,4-Dimethyl-2-chinolyl | |
| 290 | 4-Methyl-8-methoxy-2-chinolyl | |
| 291 | 4-Phenyl-8-ethoxy-2-chinolyl | |
| 292 | 4-Methyl-8-chloro-2-chinolyl | |
| 293 | 4-Methyl-8-fluoro-2-chinolyl | |
| 294 | 4-Chinolyl | |
| 295 | 2-Methyl-4-chinolyl | |
| 296 | 2-Trifluoromethyl-4-chinolyl | |
| 297 | 2-iso-Propyl-4-chinolyl | |
| 298 | 2-n-Pentyl-4-chinolyl | |
| 299 | 2-Phenyl-4-chinolyl | |
| 300 | 2,6-Dimethyl-4-chinolyl | |
| 301 | 2-Methyl-6-chloro-4-chinolyl | |
| 302 | 2-Methyl-6-fluoro-4-chinolyl | |
| 303 | 8-Chinolyl | |
| 304 | 2-Methyl-8-chinolyl | |
| 305 | 5,7-Dichloro-8-chinolyl | |
| 306 | 4,6-Dimethyl-2-pyrimidinyl | |
| 307 | 4-Trifluormethyl-2-pyrimidinyl | |
| 308 | 4,5,6-Trimethyl-2-pyrimidinyl | |
| 309 | 4-Benzyl-6-methyl-2-pyrimidinyl | |
| 310 | 4-Methyl-6-phenyl-2-pyrimidinyl | |
| 311 | 4,6-Dimethyl-5-chloro-2-pyrimidinyl | |
| 312 | 2,6-Dimethyl-4-pyrimidinyl | |
| 313 | 2,6-Bis-(trifluormethyl)-4-pyrimidinyl | |
| 314 | 2-iso-Propyl-6-methyl-4-pyrimidinyl | |
| 315 | 2-cyclo-Propyl-6-methyl-4-pyrimidinyl | |
| 316 | 2-Methyl-6-methoxymethyl-4-pyrimidinyl | |
| 317 | 2-iso-Propyl-6-methoxymethyl-4-pyrimidinyl | |
| 318 | 2-Phenyl-4-pyrimidinyl | |
| 319 | 3,5-Dimethyl-4-pyrimidinyl | |
| 320 | 2-Methyl-6-trifluoromethyl-4-pyrimidinyl | |
| 321 | 2-n-Propyl-6-trifluoromethyl-4-pyrimidinyl | |
| 322 | 2-iso-Propyl-6-trifluoromethyl-4-pyrimidinyl | |
| 323 | 2-Methyl-5-chloro-6-trifluoromethyl-4-pyrimidinyl | |
| 324 | 2-n-Propyl-5-chloro-6-trifluoromethyl-4-pyrimidinyl | |
| 325 | 2-iso-Propyl-5-chloro-6-trifluoromethyl-4-pyrimidinyl | |
| 326 | 2-tert.-Butyl-5-chloro-6-trifluoromethyl-4-pyrimidinyl | |

Tabelle 11

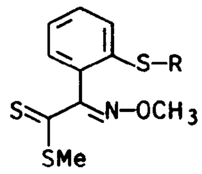

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |
| 35 | 2,4-$Br_2$-phenyl | |

| Nr. | R | Fp(°C)/IR($cm^{-1}$) |
|---|---|---|
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |
| 77 | $Br_5$-phenyl | |

| Nr. | R | Fp($^oC$)/IR($cm^{-1}$) |
|---|---|---|
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-$(i\text{-}C_3H_7)_2$-phenyl | |
| 116 | 2,6-$(i\text{-}C_3H_7)_2$-phenyl | |
| 117 | 3,5-$(i\text{-}C_3H_7)_2$-phenyl | |
| 118 | 2,4,6-$(i\text{-}C_3H_7)_3$-phenyl | |
| 119 | 2,3-$(t\text{-}C_4H_9)_2$-phenyl | |

| Nr. | R | Fp(°C)/IR($cm^{-1}$) |
|---|---|---|
| 120 | 2,4-(t-$C_4H_9$)$_2$-phenyl | |
| 121 | 2,5-(t-$C_4H_9$)$_2$-phenyl | |
| 122 | 2,6-(t-$C_4H_9$)$_2$-phenyl | |
| 123 | 3,5-(t-$C_4H_9$)$_2$-phenyl | |
| 124 | 2,4,6-(t-$C_4H_9$)$_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-(t-$C_4H_9$)$_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-(t-$C_4H_9$)$_2$-6-i-$C_3H_7$-phenyl | |
| 135 | 2-cyclo-$C_6H_{11}$-phenyl | |
| 136 | 3-cyclo-$C_6H_{11}$-phenyl | |
| 137 | 4-cyclo-$C_6H_{11}$-phenyl | |
| 138 | 2,4-(cyclo-$C_6H_{11}$)$_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-($CH_3$)$_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |
| 161 | 2-Br, 4-cyclo-$C_6H_{11}$-phenyl | |

| Nr. | R | Fp(°C)/IR($cm^{-1}$) |
|---|---|---|
| 162 | 3-$OCH_3$-phenyl | |
| 163 | 2,4-$(OCH_3)_2$-phenyl | |
| 164 | 2-$CF_3$-phenyl | |
| 165 | 3-$CF_3$-phenyl | |
| 166 | 4-$CF_3$-phenyl | |
| 167 | 2-$NO_2$-phenyl | |
| 168 | 3-$NO_2$-phenyl | |
| 169 | 4-$NO_2$-phenyl | |
| 170 | 2-CN-phenyl | |
| 171 | 3-CN-phenyl | |
| 172 | 4-CN-phenyl | |
| 173 | 2-$CH_3$, 3-Cl-phenyl | |
| 174 | 2-$CH_3$, 4-Cl-phenyl | |
| 175 | 2-$CH_3$, 5-Cl-phenyl | |
| 176 | 2-$CH_3$, 6-Cl-phenyl | |
| 177 | 2-$CH_3$, 3-F-phenyl | |
| 178 | 2-$CH_3$, 4-F-phenyl | |
| 179 | 2-$CH_3$, 5-F-phenyl | |
| 180 | 2-$CH_3$, 6-F-phenyl | |
| 181 | 2-$CH_3$, 3-Br-phenyl | |
| 182 | 2-$CH_3$, 4-Br-phenyl | |
| 183 | 2-$CH_3$, 5-Br-phenyl | |
| 184 | 2-$CH_3$, 6-Br-phenyl | |
| 185 | 2-Cl, 3-$CH_3$-phenyl | |
| 186 | 2-Cl, 4-$CH_3$-phenyl | |
| 187 | 2-Cl, 5-$CH_3$-phenyl | |
| 188 | 2-Cl, 3-i-$C_3H_7$ | |
| 189 | 2-F, 3-$CH_3$-phenyl | |
| 190 | 2-F, 4-$CH_3$-phenyl | |
| 191 | 2-F, 5-$CH_3$-phenyl | |
| 192 | 2-Br, 3-$CH_3$-phenyl | |
| 193 | 2-Br, 4-$CH_3$-phenyl | |
| 194 | 3-$CH_3$, 4-Cl-phenyl | |
| 195 | 3-$CH_3$, 5-Cl-phenyl | |
| 196 | 2-Br, 5-$CH_3$-phenyl | |
| 197 | 3-$CH_3$, 4-F-phenyl | |
| 198 | 3-$CH_3$, 5-F-phenyl | |
| 199 | 3-$CH_3$, 4-Br-phenyl | |
| 200 | 3-$CH_3$, 5-Br-phenyl | |
| 201 | 3-F, 4-$CH_3$-phenyl | |
| 202 | 3-Cl, 4-$CH_3$-phenyl | |
| 203 | 3-Br, 4-$CH_3$-phenyl | |

| Nr. | R | Fp(°C)/IR($cm^{-1}$) |
|---|---|---|
| 204 | 2-Cl, 4,5-$(CH_3)_2$-phenyl | |
| 205 | 2-Br, 4,5-$(CH_3)_2$-phenyl | |
| 206 | 2-Cl, 3,5-$(CH_3)_2$-phenyl | |
| 207 | 2-Br, 3,5-$(CH_3)_2$-phenyl | |
| 208 | 2,6-$Cl_2$, 4-$CH_3$-phenyl | |
| 209 | 2,6-$F_2$, 4-$CH_3$-phenyl | |
| 210 | 2,6-$Br_2$, 4-$CH_3$-phenyl | |
| 211 | 2,4-$Cl_2$, 6-$CH_3$-phenyl | |
| 212 | 2,4-$F_2$, 6-$CH_3$-phenyl | |
| 213 | 2,4-$Br_2$, 6-$CH_3$-phenyl | |
| 214 | 2,6-$(CH_3)_2$, 4-F-phenyl | |
| 215 | 2,6-$(CH_3)_2$, 4-Cl-phenyl | |
| 216 | 2,6-$(CH_3)_2$, 4-Br-phenyl | |
| 217 | 3,5-$(CH_3)_2$, 4-F-phenyl | |
| 218 | 3,5-$(CH_3)_2$, 4-Cl-phenyl | |
| 219 | 3,5-$(CH_3)_2$, 4-Br-phenyl | |
| 220 | 2,3,6-$(CH_3)_3$, 4-F-phenyl | |
| 221 | 2,3,6-$(CH_3)_3$, 4-Cl-phenyl | |
| 222 | 2,3,6-$(CH_3)_3$, 4-Br-phenyl | |
| 223 | 2,4-$(CH_3)_2$, 6-F-phenyl | |
| 224 | 2,4-$(CH_3)_2$, 6-Cl-phenyl | |
| 225 | 2,4-$(CH_3)_2$, 6-Br-phenyl | |
| 226 | 2-i-$C_3H_7$, 4-Cl, 5-$CH_3$-phenyl | |
| 227 | 2-Cl, 4-$NO_2$-phenyl | |
| 228 | 2-$NO_2$, 4-Cl-phenyl | |
| 229 | 2-$OCH_3$, 5-$NO_2$-phenyl | |
| 230 | 2,4-$Cl_2$, 5-$NO_2$-phenyl | |
| 231 | 2,4-$Cl_2$, 6-$NO_2$-phenyl | |
| 232 | 2,6-$Cl_2$, 4-$NO_2$-phenyl | |
| 233 | 2,6-$Br_2$, 4-$NO_2$-phenyl | |
| 234 | 2,6-$J_2$, 4-$NO_2$-phenyl | |
| 235 | 2-$C_6H_5O$-phenyl | |
| 236 | 3-$C_6H_5O$-phenyl | |
| 237 | 4-$C_6H_5O$-phenyl | |
| 238 | 3-t-$C_4H_9O$-phenyl | |
| 239 | 4-t-$C_4H_9O$-phenyl | |
| 240 | 1-Naphthyl | |
| 241 | 2-Naphthyl | |
| 242 | 2-Pyridyl | |
| 243 | 6-Methyl-2-pyridyl | |
| 244 | 6-Ethyl-2-pyridyl | |
| 245 | 6-n-Propyl-2-pyridyl | |

| Nr. | R | Fp(°C)/IR(cm$^{-1}$) |
|---|---|---|
| 246 | 6-iso-Propyl-2-pyridyl | |
| 247 | 6-n-Butyl-2-pyridyl | |
| 248 | 6-tert.-Butyl-2-pyridyl | |
| 249 | 6-n-Pentyl-2-pyridyl | |
| 250 | 6-n-Hexyl-2-pyridyl | |
| 251 | 6-Phenyl-2-pyridyl | |
| 252 | 6-Benzyl-2-pyridyl | |
| 253 | 6-Trifluormethyl-2-pyridyl | |
| 254 | 6-Methoxy-2-pyridyl | |
| 255 | 6-Chloro-2-pyridyl | |
| 256 | 3,6-Dimethyl-2-pyridyl | |
| 257 | 3,6-Diethyl-2-pyridyl | |
| 258 | 4,6-Dimethyl-2-pyridyl | |
| 259 | 5,6-Dimethyl-2-pyridyl | |
| 260 | 4-Phenyl-6-methyl-2-pyridyl | |
| 261 | 4,6-Diphenyl-2-pyridyl | |
| 262 | 3,4-Dichloro-6-methyl-2-pyridyl | |
| 263 | 3,4,5-Trichloro-6-phenyl-2-pyridyl | |
| 264 | 4-Trifluoromethyl-6-methyl-2-pyridyl | |
| 265 | 3-Cyano-6-methyl-2-pyridyl | |
| 266 | 3-Cyano-6-ethyl-2-pyridyl | |
| 267 | 3-Cyano-6-n-propyl-2-pyridyl | |
| 268 | 3-Cyano-6-iso-propyl-2-pyridyl | |
| 269 | 3-Cyano-6-cyclo-propyl-2-pyridyl | |
| 270 | 3-Cyano-6-n-butyl-2-pyridyl | |
| 271 | 3-Cyano-6-tert.-butyl-2-pyridyl | |
| 272 | 3-Cyano-6-cyclo-hexyl-2-pyridyl | |
| 273 | 3-Cyano-6-phenyl-2-pyridyl | |
| 274 | 3-Cyano-4,6-dimethyl-2-pyridyl | |
| 275 | 3,5,6-Trichloro-2-pyridyl | |
| 276 | 5-Trifluoromethyl-2-pyridyl | |
| 277 | 3-Chloro-5-trifluoromethyl-2-pyridyl | |
| 278 | 2-Chinolyl | |
| 279 | 3-Methyl-2-chinolyl | |
| 280 | 4-Methyl-2-chinolyl | |
| 281 | 4-Ethyl-2-chinolyl | |
| 282 | 4-Phenyl-2-chinolyl | |
| 283 | 6-Methyl-2-chinolyl | |
| 284 | 6-Chloro-2-chinolyl | |
| 285 | 8-Methyl-2-chinolyl | |
| 286 | 8-Chloro-2-chinolyl | |
| 287 | 3,4-Dimethyl-2-chinolyl | |

| Nr. | R | Fp($^{o}$C)/IR($cm^{-1}$) |
|---|---|---|
| 288 | 4-Methyl-8-methoxy-2-chinolyl | |
| 289 | 4-Phenyl-8-ethoxy-2-chinolyl | |
| 290 | 4-Methyl-8-chloro-2-chinolyl | |
| 291 | 4-Methyl-8-fluoro-2-chinolyl | |
| 292 | 4-Chinolyl | |
| 293 | 2-Methyl-4-chinolyl | |
| 294 | 2-Trifluoromethyl-4-chinolyl | |
| 295 | 2-iso-Propyl-4-chinolyl | |
| 296 | 2-n-Pentyl-4-chinolyl | |
| 297 | 2-Phenyl-4-chinolyl | |
| 298 | 2,6-Dimethyl-4-chinolyl | |
| 299 | 2-Methyl-6-chloro-4-chinolyl | |
| 300 | 2-Methyl-6-fluoro-4-chinolyl | |
| 301 | 8-Chinolyl | |
| 302 | 2-Methyl-8-chinolyl | |
| 303 | 5,7-Dichloro-8-chinolyl | |
| 304 | 4,6-Dimethyl-2-pyrimidinyl | |
| 305 | 4-Trifluormethyl-2-pyrimidinyl | |
| 306 | 4,5,6-Trimethyl-2-pyrimidinyl | |
| 307 | 4-Benzyl-6-methyl-2-pyrimidinyl | |
| 308 | 4-Methyl-6-phenyl-2-pyrimidinyl | |
| 309 | 4,6-Dimethyl-5-chloro-2-pyrimidinyl | |
| 310 | 2,6-Dimethyl-4-pyrimidinyl | |
| 311 | 2,6-Bis-(trifluormethyl)-4-pyrimidinyl | |
| 312 | 2-iso-Propyl-6-methyl-4-pyrimidinyl | |
| 313 | 2-cyclo-Propyl-6-methyl-4-pyrimidinyl | |
| 314 | 2-Methyl-6-methoxymethyl-4-pyrimidinyl | |
| 315 | 2-iso-Propyl-6-methoxymethyl-4-pyrimidinyl | |
| 316 | 2-Phenyl-4-pyrimidinyl | |
| 317 | 3,5-Dimethyl-4-pyrimidinyl | |
| 318 | 2-Methyl-6-trifluoromethyl-4-pyrimidinyl | |
| 319 | 2-n-Propyl-6-trifluoromethyl-4-pyrimidinyl | |
| 320 | 2-iso-Propyl-6-trifluoromethyl-4-pyrimidinyl | |
| 321 | 2-Methyl-5-chloro-6-trifluoromethyl-4-pyrimidinyl | |
| 322 | 2-n-Propyl-5-chloro-6-trifluoromethyl-4-pyrimidinyl | |
| 323 | 2-iso-Propyl-5-chloro-6-trifluoromethyl-4-pyrimidinyl | |
| 324 | 2-tert.-Butyl-5-chloro-6-trifluoromethyl-4-pyrimidinyl | |
| 325 | 6-EtO-benzothiazol-2-yl | |
| 326 | benzoxazol-2-yl | |
| 327 | 1-Me-imidazol-2-yl | |
| 328 | 4-But-1H-imidazol-2-yl | |
| 329 | 2-Thiazolin-2-yl | |

| Nr. | R | Fp(°C)/IR($cm^{-1}$) |
|---|---|---|
| 330 | 5-$CF_3$-benzimidazol-2-yl | |
| 331 | 1-Ph-tetrazol-5-yl | |
| 332 | 5-$CF_3$-benzothiazol-2-yl | |
| 333 | 4,4-$Me_2$-5-methylene-2-thiazolin-2-yl | |
| 334 | 6-Cl-4-Me-benzothiazol-2-yl | |
| 335 | 5-Me-benzothiazol-2-yl | |
| 336 | 4-Cl-benzothiazol-2-yl | |
| 337 | 1-(3-$NO_2$-Ph)tetrazol-5-yl | |
| 338 | 2-thienyl | |
| 339 | 5-Me-benzoxazol-2-yl | |
| 340 | 7-Cl-benzothiazol-2-yl | |
| 341 | 5,6-$Cl_3$-1H-benzimidazol-2-yl | |
| 342 | 5-Cl-benzoxazol-2-yl | |
| 343 | 6-Cl-benzothiazol-2-yl | |
| 344 | 2-Ph-thiazol-2-yl | |
| 345 | 3-CN-4,6-$Me_2$-2-pyridyl | |
| 346 | 1-Ph-1,2,4-triazol-3-yl | |
| 347 | 1-Pri-benzimidazol-2-yl | |
| 348 | 5-Br-benzothiazol-2-yl | |
| 349 | 5-Br-1H-benzimidazol-2-yl | |
| 350 | 7-Cl-4-MeO-benzothiazol-2-yl | |
| 351 | 1H-benzimidazol-2-yl | |
| 352 | 5-Cl-benzothiazol-2-yl | |
| 353 | 5-$NO_2$-benzoxazol-2-yl | |
| 354 | 5-t-Bu-benzoxazol-2-yl | |
| 355 | 4,6,7-$Cl_3$-benzothiozol-2-yl | |
| 356 | 5-Ph-thiazol-2-yl | |
| 357 | 5,7-$Me_2$-benzoxazol-2-yl | |
| 358 | 6-Me-benzoxazol-2-yl | |
| 359 | 1,2,4-triazin-3-yl | |
| 360 | 6-Pr-benzothiazol-2-yl | |
| 361 | 6-PhO-benzothiazol-2-yl | |
| 362 | 4-(4-Cl-Ph)-thiazol-2-yl | |
| 363 | 4-(4-Me-Ph)-thiazol-2-yl | |
| 364 | 5-Me-4-Ph-thiazol-2-yl | |
| 365 | 5-Cl-1-H-benzimidazol-2-yl | |
| 366 | 5-Ph-1,2,4-triazol-3-yl | |
| 367 | 3-Ph-1,2,4-thiadiazol-5-yl | |

Tabelle 12

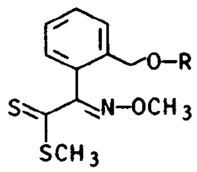

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | 58- 60°C |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-$CH_3$-Phenyl | 1662, 1494, 1461, 1240, 1190, 1122, 1051, 1025, 845, 753 ($cm^{-1}$) |
| 25 | 2-CN-Phenyl | |
| 26 | 3-CN-Phenyl | |
| 27 | 4-CN-Phenyl | |
| 28 | 2,3-$F_2$-phenyl | |
| 29 | 2,4-$F_2$-phenyl | |
| 30 | 2,3-$Cl_2$-phenyl | |
| 31 | 2,4-$Cl_2$-phenyl | |
| 32 | 2,5-$Cl_2$-phenyl | |
| 33 | 2,6-$Cl_2$-phenyl | |
| 34 | 3,4-$Cl_2$-phenyl | |

| Nr. | R | Fp(°C)/IR(cm$^{-1}$) |
|---|---|---|
| 35 | 3,5-$Cl_2$-phenyl | |
| 36 | 2,4-$Br_2$-phenyl | |
| 37 | 2,5-$Br_2$-phenyl | |
| 38 | 2,6-$Br_2$-phenyl | |
| 39 | 2,4-$J_2$-phenyl | |
| 40 | 2-Cl, 3-F-phenyl | |
| 41 | 2-Cl, 4-F-phenyl | |
| 42 | 2-Cl, 5-F-phenyl | |
| 43 | 2-Cl, 6-F-phenyl | |
| 44 | 2-Cl, 3-Br-phenyl | |
| 45 | 2-Cl, 4-Br-phenyl | |
| 46 | 2-Cl, 5-Br-phenyl | |
| 47 | 2-Cl, 6-Br-phenyl | |
| 48 | 2-Br, 3-Cl-phenyl | |
| 49 | 2-Br, 4-Cl-phenyl | |
| 50 | 2-Br, 3-F-phenyl | |
| 51 | 2-Br, 4-F-phenyl | |
| 52 | 2-Br, 5-F-phenyl | |
| 53 | 2-Br, 6-F-phenyl | |
| 54 | 2-F, 3-Cl-phenyl | |
| 55 | 2-F, 4-Cl-phenyl | |
| 56 | 2-F, 5-Cl-phenyl | |
| 57 | 3-Cl, 4-F-phenyl | |
| 58 | 3-Cl, 5-F-phenyl | |
| 59 | 3-Cl, 4-Br-phenyl | |
| 60 | 3-Cl, 5-Br-phenyl | |
| 61 | 3-F, 4-Cl-phenyl | |
| 62 | 3-F, 4-Br-phenyl | |
| 63 | 3-Br, 4-Cl-phenyl | |
| 64 | 3-Br, 4-F-phenyl | |
| 65 | 2,4,6-$F_3$-phenyl | |
| 66 | 2,3,4,-$Cl_3$-phenyl | |
| 67 | 2,3,5-$Cl_3$-phenyl | |
| 68 | 2,3,6-$Cl_3$-phenyl | |
| 69 | 2,4,5-$Cl_3$-phenyl | |
| 70 | 2,4,6-$Cl_3$-phenyl | |
| 71 | 3,4,5-$Cl_3$-phenyl | |
| 72 | 2,4,6-$Br_3$-phenyl | |
| 73 | 2,6-$Cl_2$-4-Br-phenyl | |
| 74 | 2,3,4,6-$Cl_4$-phenyl | |
| 75 | 2,3,5,6-$Cl_4$-phenyl | |
| 76 | $F_5$-phenyl | |

| Nr. | R | Fp($^{o}$C)/IR($cm^{-1}$) |
|---|---|---|
| 77 | $Cl_5$-phenyl | |
| 78 | $Br_5$-phenyl | |
| 79 | 2-$CH_3$-phenyl | |
| 80 | 3-$CH_3$-phenyl | |
| 81 | 4-$CH_3$-phenyl | |
| 82 | 2-$C_2H_5$-phenyl | |
| 83 | 3-$C_2H_5$-phenyl | |
| 84 | 4-$C_2H_5$-phenyl | |
| 85 | 2-n-$C_3H_7$-phenyl | |
| 86 | 3-n-$C_3H_7$-phenyl | |
| 87 | 4-n-$C_3H_7$-phenyl | |
| 88 | 2-i-$C_3H_7$-phenyl | |
| 89 | 3-i-$C_3H_7$-phenyl | |
| 90 | 4-i-$C_3H_7$-phenyl | |
| 91 | 2-s-$C_4H_9$-phenyl | |
| 92 | 3-s-$C_4H_9$-phenyl | |
| 93 | 4-s-$C_4H_9$-phenyl | |
| 94 | 2-t-$C_4H_9$-phenyl | |
| 95 | 3-t-$C_4H_9$-phenyl | |
| 96 | 4-t-$C_4H_9$-phenyl | |
| 97 | 2,3-$(CH_3)_2$-phenyl | |
| 98 | 2,4-$(CH_3)_2$-phenyl | |
| 99 | 2,5-$(CH_3)_2$-phenyl | |
| 100 | 2,6-$(CH_3)_2$-phenyl | |
| 101 | 3,4-$(CH_3)_2$-phenyl | |
| 102 | 3,5-$(CH_3)_2$-phenyl | |
| 103 | 2,3,4-$(CH_3)_3$-phenyl | |
| 104 | 2,3,5-$(CH_3)_3$-phenyl | |
| 105 | 2,3,6-$(CH_3)_3$-phenyl | |
| 106 | 2,4,5-$(CH_3)_3$-phenyl | |
| 107 | 2,4,6-$(CH_3)_3$-phenyl | |
| 108 | 3,4,5-$(CH_3)_3$-phenyl | |
| 109 | 2,3,4,6-$(CH_3)_4$-phenyl | |
| 110 | 2,3,5,6-$(CH_3)_4$-phenyl | |
| 111 | $(CH_3)_5$-phenyl | |
| 112 | 2,4-$(C_2H_5)_2$-phenyl | |
| 113 | 2,6-$(C_2H_5)_2$-phenyl | |
| 114 | 3,5-$(C_2H_5)_2$-phenyl | |
| 115 | 2,4,6-$(C_2H_5)_3$-phenyl | |
| 116 | 2,4-(i-$C_3H_7)_2$-phenyl | |
| 117 | 2,6-(i-$C_3H_7)_2$-phenyl | |
| 118 | 3,5-(i-$C_3H_7)_2$-phenyl | |

| Nr. | R | Fp($^oC$)/IR($cm^{-1}$) |
|---|---|---|
| 119 | 2,4,6-(i-$C_3H_7$)$_3$-phenyl | |
| 120 | 2,3-(t-$C_4H_9$)$_2$-phenyl | |
| 121 | 2,4-(t-$C_4H_9$)$_2$-phenyl | |
| 122 | 2,5-(t-$C_4H_9$)$_2$-phenyl | |
| 123 | 2,6-(t-$C_4H_9$)$_2$-phenyl | |
| 124 | 3,5-(t-$C_4H_9$)$_2$-phenyl | |
| 125 | 2,4,6-(t-$C_4H_9$)$_3$-phenyl | |
| 126 | 2-t-$C_4H_9$, 4-$CH_3$-phenyl | |
| 127 | 2-t-$C_4H_9$, 5-$CH_3$-phenyl | |
| 128 | 2,6-(t-$C_4H_9$)$_2$, 4-$CH_3$-phenyl | |
| 129 | 2-$CH_3$, 4-t-$C_4H_9$-phenyl | |
| 130 | 2-$CH_3$, 6-t-$C_4H_9$-phenyl | |
| 131 | 1-$CH_3$, 4-i-$C_3H_7$-phenyl | |
| 132 | 2-$CH_3$, 5-i-$C_3H_7$-phenyl | |
| 133 | 3-$CH_3$, 4-i-$C_3H_7$-phenyl | |
| 134 | 2-i-$C_3H_7$, 5-$CH_3$-phenyl | |
| 135 | 2,4-(t-$C_4H_9$)$_2$-6-i-$C_3H_7$-phenyl | |
| 136 | 2-cyclo-$C_6H_{11}$-phenyl | |
| 137 | 3-cyclo-$C_6H_{11}$-phenyl | |
| 138 | 4-cyclo-$C_6H_{11}$-phenyl | |
| 139 | 2,4-(cyclo-$C_6H_{11}$)$_2$-6-$CH_3$-phenyl | |
| 140 | 2-$CH_3$, 4-cyclo-$C_6H_{11}$-phenyl | |
| 141 | 2-$CH_2C_6H_5$-phenyl | |
| 142 | 3-$CH_2C_6H_5$-phenyl | |
| 143 | 4-$CH_2C_6H_5$-phenyl | |
| 144 | 2-$CH_2C_6H_5$, 4-$CH_3$-phenyl | |
| 145 | 2-$CH_3$, 4-$CH_2C_6H_5$-phenyl | |
| 146 | 2-$C_6H_5$-phenyl | |
| 147 | 3-$C_6H_5$-phenyl | |
| 148 | 4-$C_6H_5$-phenyl | |
| 149 | 4-(2-i-$C_3H_7$-$C_6H_4$)-phenyl | |
| 150 | 4-$C_6H_5$, 2,6-($CH_3$)$_2$-phenyl | |
| 151 | 2-Cl, 4-$C_6H_5$-phenyl | |
| 152 | 2-Br, 4-$C_6H_5$-phenyl | |
| 153 | 2-$C_6H_5$, 4-Cl-phenyl | |
| 154 | 2-$C_6H_5$, 4-Br-phenyl | |
| 155 | 2-$CH_2C_6H_5$, 4-Cl-phenyl | |
| 156 | 2-$CH_2C_6H_5$, 4-Br-phenyl | |
| 157 | 2-Cl, 4-$CH_2C_6H_5$-phenyl | |
| 158 | 2-Br, 4-$CH_2C_6H_5$-phenyl | |
| 159 | 2-cyclo-$C_6H_{11}$, 4-Cl-phenyl | |
| 160 | 2-cyclo-$C_6H_{11}$, 4-Br-phenyl | |

| Nr. | R | Fp(°C)/IR($cm^{-1}$) |
|---|---|---|
| 161 | 2-Cl, 4-cyclo-$C_6H_{11}$-phenyl | |
| 162 | 2-Br, 4-cyclo-$C_6H_{11}$-phenyl | |
| 163 | 3-$OCH_3$-phenyl | |
| 164 | 2,4-$(OCH_3)_2$-phenyl | |
| 165 | 2-$CF_3$-phenyl | |
| 166 | 3-$CF_3$-phenyl | |
| 167 | 4-$CF_3$-phenyl | |
| 168 | 2-$NO_2$-phenyl | |
| 169 | 3-$NO_2$-phenyl | |
| 170 | 4-$NO_2$-phenyl | |
| 171 | 2-CN-phenyl | |
| 172 | 3-CN-phenyl | |
| 173 | 4-CN-phenyl | |
| 174 | 2-$CH_3$, 3-Cl-phenyl | |
| 175 | 2-$CH_3$, 4-Cl-phenyl | |
| 176 | 2-$CH_3$, 5-Cl-phenyl | |
| 177 | 2-$CH_3$, 6-Cl-phenyl | |
| 178 | 2-$CH_3$, 3-F-phenyl | |
| 179 | 2-$CH_3$, 4-F-phenyl | |
| 180 | 2-$CH_3$, 5-F-phenyl | |
| 181 | 2-$CH_3$, 6-F-phenyl | |
| 182 | 2-$CH_3$, 3-Br-phenyl | |
| 183 | 2-$CH_3$, 4-Br-phenyl | |
| 184 | 2-$CH_3$, 5-Br-phenyl | |
| 185 | 2-$CH_3$, 6-Br-phenyl | |
| 186 | 2-Cl, 3-$CH_3$-phenyl | |
| 187 | 2-Cl, 4-$CH_3$-phenyl | |
| 188 | 2-Cl, 5-$CH_3$-phenyl | |
| 189 | 2-Cl, 3-i-$C_3H_7$ | |
| 190 | 2-F, 3-$CH_3$-phenyl | |
| 191 | 2-F, 4-$CH_3$-phenyl | |
| 192 | 2-F, 5-$CH_3$-phenyl | |
| 193 | 2-Br, 3-$CH_3$-phenyl | |
| 194 | 2-Br, 4-$CH_3$-phenyl | |
| 195 | 3-$CH_3$, 4-Cl-phenyl | |
| 196 | 3-$CH_3$, 5-Cl-phenyl | |
| 197 | 2-Br, 5-$CH_3$-phenyl | |
| 198 | 3-$CH_3$, 4-F-phenyl | |
| 199 | 3-$CH_3$, 5-F-phenyl | |
| 200 | 3-$CH_3$, 4-Br-phenyl | |
| 201 | 3-$CH_3$, 5-Br-phenyl | |
| 202 | 3-F, 4-$CH_3$-phenyl | |

| Nr. | R | Fp($^{o}C$)/IR($cm^{-1}$) |
|---|---|---|
| 203 | 3-Cl, 4-$CH_3$-phenyl | |
| 204 | 3-Br, 4-$CH_3$-phenyl | |
| 205 | 2-Cl, 4,5-$(CH_3)_2$-phenyl | |
| 206 | 2-Br, 4,5-$(CH_3)_2$-phenyl | |
| 207 | 2-Cl, 3,5-$(CH_3)_2$-phenyl | |
| 208 | 2-Br, 3,5-$(CH_3)_2$-phenyl | |
| 209 | 2,6-$Cl_2$, 4-$CH_3$-phenyl | |
| 210 | 2,6-$F_2$, 4-$CH_3$-phenyl | |
| 211 | 2,6-$Br_2$, 4-$CH_3$-phenyl | |
| 212 | 2,4-$Cl_2$, 6-$CH_3$-phenyl | |
| 213 | 2,4-$F_2$, 6-$CH_3$-phenyl | |
| 214 | 2,4-$Br_2$, 6-$CH_3$-phenyl | |
| 215 | 2,6-$(CH_3)_2$, 4-F-phenyl | |
| 216 | 2,6-$(CH_3)_2$, 4-Cl-phenyl | |
| 217 | 2,6-$(CH_3)_2$, 4-Br-phenyl | |
| 218 | 3,5-$(CH_3)_2$, 4-F-phenyl | |
| 219 | 3,5-$(CH_3)_2$, 4-Cl-phenyl | |
| 220 | 3,5-$(CH_3)_2$, 4-Br-phenyl | |
| 221 | 2,3,6-$(CH_3)_3$, 4-F-phenyl | |
| 222 | 2,3,6-$(CH_3)_3$, 4-Cl-phenyl | |
| 223 | 2,3,6-$(CH_3)_3$, 4-Br-phenyl | |
| 224 | 2,4-$(CH_3)_2$, 6-F-phenyl | |
| 225 | 2,4-$(CH_3)_2$, 6-Cl-phenyl | |
| 226 | 2,4-$(CH_3)_2$, 6-Br-phenyl | |
| 227 | 2-i-$C_3H_7$, 4-Cl, 5-$CH_3$-phenyl | |
| 228 | 2-Cl, 4-$NO_2$-phenyl | |
| 229 | 2-$NO_2$, 4-Cl-phenyl | |
| 230 | 2-$OCH_3$, 5-$NO_2$-phenyl | |
| 231 | 2,4-$Cl_2$, 5-$NO_2$-phenyl | |
| 232 | 2,4-$Cl_2$, 6-$NO_2$-phenyl | |
| 233 | 2,6-$Cl_2$, 4-$NO_2$-phenyl | |
| 234 | 2,6-$Br_2$, 4-$NO_2$-phenyl | |
| 235 | 2,6-$J_2$, 4-$NO_2$-phenyl | |
| 236 | 2-$C_6H_5O$-phenyl | |
| 237 | 3-$C_6H_5O$-phenyl | |
| 238 | 4-$C_6H_5O$-phenyl | |
| 239 | 3-t-$C_4H_9O$-phenyl | |
| 240 | 4-t-$C_4H_9O$-phenyl | |
| 241 | 1-Naphthyl | |
| 242 | 2-Naphthyl | |
| 243 | 2-Pyridyl | |
| 244 | 6-Methyl-2-pyridyl | |

| Nr. | R | Fp(°C)/IR($cm^{-1}$) |
|---|---|---|
| 245 | 6-Ethyl-2-pyridyl | |
| 246 | 6-n-Propyl-2-pyridyl | |
| 247 | 6-iso-Propyl-2-pyridyl | |
| 248 | 6-n-Butyl-2-pyridyl | |
| 249 | 6-tert.-Butyl-2-pyridyl | |
| 250 | 6-n-Pentyl-2-pyridyl | |
| 251 | 6-n-Hexyl-2-pyridyl | |
| 252 | 6-Phenyl-2-pyridyl | |
| 253 | 6-Benzyl-2-pyridyl | |
| 254 | 6-Trifluormethyl-2-pyridyl | |
| 255 | 6-Methoxy-2-pyridyl | |
| 256 | 6-Chloro-2-pyridyl | |
| 257 | 3,6-Dimethyl-2-pyridyl | |
| 258 | 3,6-Diethyl-2-pyridyl | |
| 259 | 4,6-Dimethyl-2-pyridyl | |
| 260 | 5,6-Dimethyl-2-pyridyl | |
| 261 | 4-Phenyl-6-methyl-2-pyridyl | |
| 262 | 4,6-Diphenyl-2-pyridyl | |
| 263 | 3,4-Dichloro-6-methyl-2-pyridyl | |
| 264 | 3,4,5-Trichloro-6-phenyl-2-pyridyl | |
| 265 | 4-Trifluoromethyl-6-methyl-2-pyridyl | |
| 266 | 3-Cyano-6-methyl-2-pyridyl | |
| 267 | 3-Cyano-6-ethyl-2-pyridyl | |
| 268 | 3-Cyano-6-n-propyl-2-pyridyl | |
| 269 | 3-Cyano-6-iso-propyl-2-pyridyl | |
| 270 | 3-Cyano-6-cyclo-propyl-2-pyridyl | |
| 271 | 3-Cyano-6-n-butyl-2-pyridyl | |
| 272 | 3-Cyano-6-tert.-butyl-2-pyridyl | |
| 273 | 3-Cyano-6-cyclo-hexyl-2-pyridyl | |
| 274 | 3-Cyano-6-phenyl-2-pyridyl | |
| 275 | 3-Cyano-4,6-dimethyl-2-pyridyl | |
| 276 | 3,5,6-Trichloro-2-pyridyl | |
| 277 | 5-Trifluoromethyl-2-pyridyl | |
| 278 | 3-Chloro-5-trifluoromethyl-2-pyridyl | |
| 279 | 2-Chinolyl | |
| 280 | 3-Methyl-2-chinolyl | |
| 281 | 4-Methyl-2-chinolyl | |
| 282 | 4-Ethyl-2-chinolyl | |
| 283 | 4-Phenyl-2-chinolyl | |
| 284 | 6-Methyl-2-chinolyl | |
| 285 | 6-Chloro-2-chinolyl | |
| 286 | 8-Methyl-2-chinolyl | |

| Nr. | R | Fp(°C)/IR($cm^{-1}$) |
|---|---|---|
| 287 | 8-Chloro-2-chinolyl | |
| 288 | 3,4-Dimethyl-2-chinolyl | |
| 289 | 4-Methyl-8-methoxy-2-chinolyl | |
| 290 | 4-Phenyl-8-ethoxy-2-chinolyl | |
| 291 | 4-Methyl-8-chloro-2-chinolyl | |
| 292 | 4-Methyl-8-fluoro-2-chinolyl | |
| 293 | 4-Chinolyl | |
| 294 | 2-Methyl-4-chinolyl | |
| 295 | 2-Trifluoromethyl-4-chinolyl | |
| 296 | 2-iso-Propyl-4-chinolyl | |
| 297 | 2-n-Pentyl-4-chinolyl | |
| 298 | 2-Phenyl-4-chinolyl | |
| 299 | 2,6-Dimethyl-4-chinolyl | |
| 300 | 2-Methyl-6-chloro-4-chinolyl | |
| 301 | 2-Methyl-6-fluoro-4-chinolyl | |
| 302 | 8-Chinolyl | |
| 303 | 2-Methyl-8-chinolyl | |
| 304 | 5,7-Dichloro-8-chinolyl | |
| 305 | 4,6-Dimethyl-2-pyrimidinyl | |
| 306 | 4-Trifluormethyl-2-pyrimidinyl | |
| 307 | 4,5,6-Trimethyl-2-pyrimidinyl | |
| 308 | 4-Benzyl-6-methyl-2-pyrimidinyl | |
| 309 | 4-Methyl-6-phenyl-2-pyrimidinyl | |
| 310 | 4,6-Dimethyl-5-chloro-2-pyrimidinyl | |
| 311 | 2,6-Dimethyl-4-pyrimidinyl | |
| 312 | 2,6-Bis-(trifluormethyl)-4-pyrimidinyl | |
| 313 | 2-iso-Propyl-6-methyl-4-pyrimidinyl | |
| 314 | 2-cyclo-Propyl-6-methyl-4-pyrimidinyl | |
| 315 | 2-Methyl-6-methoxymethyl-4-pyrimidinyl | |
| 316 | 2-iso-Propyl-6-methoxymethyl-4-pyrimidinyl | |
| 317 | 2-Phenyl-4-pyrimidinyl | |
| 318 | 3,5-Dimethyl-4-pyrimidinyl | |
| 319 | 2-Methyl-6-trifluoromethyl-4-pyrimidinyl | |
| 320 | 2-n-Propyl-6-trifluoromethyl-4-pyrimidinyl | |
| 321 | 2-iso-Propyl-6-trifluoromethyl-4-pyrimidinyl | |
| 322 | 2-Methyl-5-chloro-6-trifluoromethyl-4-pyrimidinyl | |
| 323 | 2-n-Propyl-5-chloro-6-trifluoromethyl-4-pyrimidinyl | |
| 324 | 2-iso-Propyl-5-chloro-6-trifluoromethyl-4-pyrimidinyl | |
| 325 | 2-tert.-Butyl-5-chloro-6-trifluoromethyl-4-pyrimidinyl | |

Tabelle 13

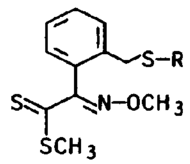

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | 1661, 1451, 1443, 1139, 1115, 1043, 1035, 1024, 846, 749 |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |

| Nr. | R | Fp(°C)/IR($cm^{-1}$) |
|---|---|---|
| 35 | 2,4-$Br_2$-phenyl | |
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |

| Nr. | R | Fp(°C)/IR($cm^{-1}$) |
|---|---|---|
| 77 | $Br_5$-phenyl | |
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-$(i\text{-}C_3H_7)_2$-phenyl | |
| 116 | 2,6-$(i\text{-}C_3H_7)_2$-phenyl | |
| 117 | 3,5-$(i\text{-}C_3H_7)_2$-phenyl | |
| 118 | 2,4,6-$(i\text{-}C_3H_7)_3$-phenyl | |

| Nr. | R | Fp(°C)/IR($cm^{-1}$) |
|---|---|---|
| 119 | 2,3-(t-$C_4H_9$)$_2$-phenyl | |
| 120 | 2,4-(t-$C_4H_9$)$_2$-phenyl | |
| 121 | 2,5-(t-$C_4H_9$)$_2$-phenyl | |
| 122 | 2,6-(t-$C_4H_9$)$_2$-phenyl | |
| 123 | 3,5-(t-$C_4H_9$)$_2$-phenyl | |
| 124 | 2,4,6-(t-$C_4H_9$)$_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-(t-$C_4H_9$)$_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-(t-$C_4H_9$)$_2$-6-i-$C_3H_7$-phenyl | |
| 135 | 2-cyclo-$C_6H_{11}$-phenyl | |
| 136 | 3-cyclo-$C_6H_{11}$-phenyl | |
| 137 | 4-cyclo-$C_6H_{11}$-phenyl | |
| 138 | 2,4-(cyclo-$C_6H_{11}$)$_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-($CH_3$)$_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |

| Nr. | R | Fp(°C)/IR($cm^{-1}$) |
|---|---|---|
| 161 | 2-Br, 4-cyclo-$C_6H_{11}$-phenyl | |
| 162 | 3-$OCH_3$-phenyl | |
| 163 | 2,4-$(OCH_3)_2$-phenyl | |
| 164 | 2-$CF_3$-phenyl | |
| 165 | 3-$CF_3$-phenyl | |
| 166 | 4-$CF_3$-phenyl | |
| 167 | 2-$NO_2$-phenyl | |
| 168 | 3-$NO_2$-phenyl | |
| 169 | 4-$NO_2$-phenyl | |
| 170 | 2-CN-phenyl | |
| 171 | 3-CN-phenyl | |
| 172 | 4-CN-phenyl | |
| 173 | 2-$CH_3$, 3-Cl-phenyl | |
| 174 | 2-$CH_3$, 4-Cl-phenyl | |
| 175 | 2-$CH_3$, 5-Cl-phenyl | |
| 176 | 2-$CH_3$, 6-Cl-phenyl | |
| 177 | 2-$CH_3$, 3-F-phenyl | |
| 178 | 2-$CH_3$, 4-F-phenyl | |
| 179 | 2-$CH_3$, 5-F-phenyl | |
| 180 | 2-$CH_3$, 6-F-phenyl | |
| 181 | 2-$CH_3$, 3-Br-phenyl | |
| 182 | 2-$CH_3$, 4-Br-phenyl | |
| 183 | 2-$CH_3$, 5-Br-phenyl | |
| 184 | 2-$CH_3$, 6-Br-phenyl | |
| 185 | 2-Cl, 3-$CH_3$-phenyl | |
| 186 | 2-Cl, 4-$CH_3$-phenyl | |
| 187 | 2-Cl, 5-$CH_3$-phenyl | |
| 188 | 2-Cl, 3-i-$C_3H_7$ | |
| 189 | 2-F, 3-$CH_3$-phenyl | |
| 190 | 2-F, 4-$CH_3$-phenyl | |
| 191 | 2-F, 5-$CH_3$-phenyl | |
| 192 | 2-Br, 3-$CH_3$-phenyl | |
| 193 | 2-Br, 4-$CH_3$-phenyl | |
| 194 | 3-$CH_3$, 4-Cl-phenyl | |
| 195 | 3-$CH_3$, 5-Cl-phenyl | |
| 196 | 2-Br, 5-$CH_3$-phenyl | |
| 197 | 3-$CH_3$, 4-F-phenyl | |
| 198 | 3-$CH_3$, 5-F-phenyl | |
| 199 | 3-$CH_3$, 4-Br-phenyl | |
| 200 | 3-$CH_3$, 5-Br-phenyl | |
| 201 | 3-F, 4-$CH_3$-phenyl | |
| 202 | 3-Cl, 4-$CH_3$-phenyl | |

| Nr. | R | Fp(°C)/IR($cm^{-1}$) |
|---|---|---|
| 203 | 3-Br, 4-$CH_3$-phenyl | |
| 204 | 2-Cl, 4,5-$(CH_3)_2$-phenyl | |
| 205 | 2-Br, 4,5-$(CH_3)_2$-phenyl | |
| 206 | 2-Cl, 3,5-$(CH_3)_2$-phenyl | |
| 207 | 2-Br, 3,5-$(CH_3)_2$-phenyl | |
| 208 | 2,6-$Cl_2$, 4-$CH_3$-phenyl | |
| 209 | 2,6-$F_2$, 4-$CH_3$-phenyl | |
| 210 | 2,6-$Br_2$, 4-$CH_3$-phenyl | |
| 211 | 2,4-$Cl_2$, 6-$CH_3$-phenyl | |
| 212 | 2,4-$F_2$, 6-$CH_3$-phenyl | |
| 213 | 2,4-$Br_2$, 6-$CH_3$-phenyl | |
| 214 | 2,6-$(CH_3)_2$, 4-F-phenyl | |
| 215 | 2,6-$(CH_3)_2$, 4-Cl-phenyl | |
| 216 | 2,6-$(CH_3)_2$, 4-Br-phenyl | |
| 217 | 3,5-$(CH_3)_2$, 4-F-phenyl | |
| 218 | 3,5-$(CH_3)_2$, 4-Cl-phenyl | |
| 219 | 3,5-$(CH_3)_2$, 4-Br-phenyl | |
| 220 | 2,3,6-$(CH_3)_3$, 4-F-phenyl | |
| 221 | 2,3,6-$(CH_3)_3$, 4-Cl-phenyl | |
| 222 | 2,3,6-$(CH_3)_3$, 4-Br-phenyl | |
| 223 | 2,4-$(CH_3)_2$, 6-F-phenyl | |
| 224 | 2,4-$(CH_3)_2$, 6-Cl-phenyl | |
| 225 | 2,4-$(CH_3)_2$, 6-Br-phenyl | |
| 226 | 2-i-$C_3H_7$, 4-Cl, 5-$CH_3$-phenyl | |
| 227 | 2-Cl, 4-$NO_2$-phenyl | |
| 228 | 2-$NO_2$, 4-Cl-phenyl | |
| 229 | 2-$OCH_3$, 5-$NO_2$-phenyl | |
| 230 | 2,4-$Cl_2$, 5-$NO_2$-phenyl | |
| 231 | 2,4-$Cl_2$, 6-$NO_2$-phenyl | |
| 232 | 2,6-$Cl_2$, 4-$NO_2$-phenyl | |
| 233 | 2,6-$Br_2$, 4-$NO_2$-phenyl | |
| 234 | 2,6-$J_2$, 4-$NO_2$-phenyl | |
| 235 | 2-$C_6H_5O$-phenyl | |
| 236 | 3-$C_6H_5O$-phenyl | |
| 237 | 4-$C_6H_5O$-phenyl | |
| 238 | 3-t-$C_4H_9O$-phenyl | |
| 239 | 4-t-$C_4H_9O$-phenyl | |
| 240 | 1-Naphthyl | |
| 241 | 2-Naphthyl | |
| 242 | 2-Pyridyl | |
| 243 | 6-Methyl-2-pyridyl | 89-94°C |
| 244 | 6-Ethyl-2-pyridyl | |

| Nr. | R | Fp(°C)/IR($cm^{-1}$) |
|---|---|---|
| 245 | 6-n-Propyl-2-pyridyl | |
| 246 | 6-iso-Propyl-2-pyridyl | |
| 247 | 6-n-Butyl-2-pyridyl | |
| 248 | 6-tert.-Butyl-2-pyridyl | |
| 249 | 6-n-Pentyl-2-pyridyl | |
| 250 | 6-n-Hexyl-2-pyridyl | |
| 251 | 6-Phenyl-2-pyridyl | |
| 252 | 6-Benzyl-2-pyridyl | |
| 253 | 6-Trifluormethyl-2-pyridyl | |
| 254 | 6-Methoxy-2-pyridyl | |
| 255 | 6-Chloro-2-pyridyl | |
| 256 | 3,6-Dimethyl-2-pyridyl | |
| 257 | 3,6-Diethyl-2-pyridyl | |
| 258 | 4,6-Dimethyl-2-pyridyl | |
| 259 | 5,6-Dimethyl-2-pyridyl | |
| 260 | 4-Phenyl-6-methyl-2-pyridyl | |
| 261 | 4,6-Diphenyl-2-pyridyl | |
| 262 | 3,4-Dichloro-6-methyl-2-pyridyl | |
| 263 | 3,4,5-Trichloro-6-phenyl-2-pyridyl | |
| 264 | 4-Trifluoromethyl-6-methyl-2-pyridyl | |
| 265 | 3-Cyano-6-methyl-2-pyridyl | |
| 266 | 3-Cyano-6-ethyl-2-pyridyl | |
| 267 | 3-Cyano-6-n-propyl-2-pyridyl | |
| 268 | 3-Cyano-6-iso-propyl-2-pyridyl | |
| 269 | 3-Cyano-6-cyclo-propyl-2-pyridyl | |
| 270 | 3-Cyano-6-n-butyl-2-pyridyl | |
| 271 | 3-Cyano-6-tert.-butyl-2-pyridyl | |
| 272 | 3-Cyano-6-cyclo-hexyl-2-pyridyl | |
| 273 | 3-Cyano-6-phenyl-2-pyridyl | |
| 274 | 3-Cyano-4,6-dimethyl-2-pyridyl | |
| 275 | 3,5,6-Trichloro-2-pyridyl | |
| 276 | 5-Trifluoromethyl-2-pyridyl | |
| 277 | 3-Chloro-5-trifluoromethyl-2-pyridyl | |
| 278 | 2-Chinolyl | |
| 279 | 3-Methyl-2-chinolyl | |
| 280 | 4-Methyl-2-chinolyl | |
| 281 | 4-Ethyl-2-chinolyl | |
| 282 | 4-Phenyl-2-chinolyl | |
| 283 | 6-Methyl-2-chinolyl | |
| 284 | 6-Chloro-2-chinolyl | |
| 285 | 8-Methyl-2-chinolyl | |
| 286 | 8-Chloro-2-chinolyl | |

| Nr. | R | Fp(°C)/IR($cm^{-1}$) |
|---|---|---|
| 287 | 3,4-Dimethyl-2-chinolyl | |
| 288 | 4-Methyl-8-methoxy-2-chinolyl | |
| 289 | 4-Phenyl-8-ethoxy-2-chinolyl | |
| 290 | 4-Methyl-8-chloro-2-chinolyl | |
| 291 | 4-Methyl-8-fluoro-2-chinolyl | |
| 292 | 4-Chinolyl | |
| 293 | 2-Methyl-4-chinolyl | |
| 294 | 2-Trifluoromethyl-4-chinolyl | |
| 295 | 2-iso-Propyl-4-chinolyl | |
| 296 | 2-n-Pentyl-4-chinolyl | |
| 297 | 2-Phenyl-4-chinolyl | |
| 298 | 2,6-Dimethyl-4-chinolyl | |
| 299 | 2-Methyl-6-chloro-4-chinolyl | |
| 300 | 2-Methyl-6-fluoro-4-chinolyl | |
| 301 | 8-Chinolyl | |
| 302 | 2-Methyl-8-chinolyl | |
| 303 | 5,7-Dichloro-8-chinolyl | |
| 304 | 4,6-Dimethyl-2-pyrimidinyl | |
| 305 | 4-Trifluormethyl-2-pyrimidinyl | |
| 306 | 4,5,6-Trimethyl-2-pyrimidinyl | |
| 307 | 4-Benzyl-6-methyl-2-pyrimidinyl | |
| 308 | 4-Methyl-6-phenyl-2-pyrimidinyl | |
| 309 | 4,6-Dimethyl-5-chloro-2-pyrimidinyl | |
| 310 | 2,6-Dimethyl-4-pyrimidinyl | |
| 311 | 2,6-Bis-(trifluormethyl)-4-pyrimidinyl | |
| 312 | 2-iso-Propyl-6-methyl-4-pyrimidinyl | |
| 313 | 2-cyclo-Propyl-6-methyl-4-pyrimidinyl | |
| 314 | 2-Methyl-6-methoxymethyl-4-pyrimidinyl | |
| 315 | 2-iso-Propyl-6-methoxymethyl-4-pyrimidinyl | |
| 316 | 2-Phenyl-4-pyrimidinyl | |
| 317 | 3,5-Dimethyl-4-pyrimidinyl | |
| 318 | 2-Methyl-6-trifluoromethyl-4-pyrimidinyl | |
| 319 | 2-n-Propyl-6-trifluoromethyl-4-pyrimidinyl | |
| 320 | 2-iso-Propyl-6-trifluoromethyl-4-pyrimidinyl | |
| 321 | 2-Methyl-5-chloro-6-trifluoromethyl-4-pyrimidinyl | |
| 322 | 2-n-Propyl-5-chloro-6-trifluoromethyl-4-pyrimidinyl | |
| 323 | 2-iso-Propyl-5-chloro-6-trifluoromethyl-4-pyrimidinyl | |
| 324 | 2-tert.-Butyl-5-chloro-6-trifluoromethyl-4-pyrimidinyl | |
| 325 | 6-EtO-benzothiazol-2-yl | |
| 326 | Benzoxazol-2-yl | |
| 327 | 1-Me-imidazol-2-yl | |
| 328 | 4-But-1H-imidazol-2-yl | |

| Nr. | R | Fp(°C)/IR($cm^{-1}$) |
|---|---|---|
| 329 | 2-Thiazolin-2-yl | |
| 330 | 5-$CF_3$-benzimidazol-2-yl | |
| 331 | 1-Ph-tetrazol-5-yl | |
| 332 | 5-$CF_3$-benzothiazol-2-yl | |
| 333 | 4,4-$Me_2$-5-methylene-2-thiazolin-2-yl | |
| 334 | 6-Cl-4-Me-benzothiazol-2-yl | |
| 335 | 5-Me-benzothiazol-2-yl | |
| 336 | 4-Cl-benzothiazol-2-yl | |
| 337 | 1-(3-$NO_2$-Ph)tetrazol-5-yl | |
| 338 | 2-Thienyl | |
| 339 | 5-Me-benzoxazol-2-yl | |
| 340 | 7-Cl-benzothiazol-2-yl | |
| 341 | 5,6-$Cl_3$-1H-benzimidazol-2-yl | |
| 342 | 5-Cl-benzoxazol-2-yl | |
| 343 | 6-Cl-benzothiazol-2-yl | |
| 344 | 2-Ph-thiazol-2-yl | |
| 345 | 3-CN-4,6-$Me_2$-2-pyridyl | |
| 346 | 1-Ph-1,2,4-triazol-3-yl | |
| 347 | 1-Pri-benzimidazol-2-yl | |
| 348 | 5-Br-benzothiazol-2-yl | |
| 349 | 5-Br-1H-benzimidazol-2-yl | |
| 350 | 7-Cl-4-MeO-benzothiazol-2-yl | |
| 351 | 1H-benzimidazol-2-yl | |
| 352 | 5-Cl-benzothiazol-2-yl | |
| 353 | 5-$NO_2$-benzoxazol-2-yl | |
| 354 | 5-t-Bu-benzoxazol-2-yl | |
| 355 | 4,6,7-$Cl_3$-benzothiozol-2-yl | |
| 356 | 5-Ph-thiazol-2-yl | |
| 357 | 5,7-$Me_2$-benzoxazol-2-yl | |
| 358 | 6-Me-benzoxazol-2-yl | |
| 359 | 1,2,4-triazin-3-yl | |
| 360 | 6-Pr-benzothiazol-2-yl | |
| 361 | 6-PhO-benzothiazol-2-yl | |
| 362 | 4-(4-Cl-Ph)-thiazol-2-yl | |
| 363 | 4-(4-Me-Ph)-thiazol-2-yl | |
| 364 | 5-Me-4-Ph-thiazol-2-yl | |
| 365 | 5-Cl-1-H-benzimidazol-2-yl | |
| 366 | 5-Ph-1,2,4-triazol-3-yl | |
| 367 | 3-Ph-1,2,4-thiadiazol-5-yl | |

Tabelle 14

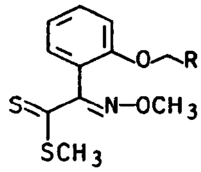

| Nr. | R | Fp($^{o}C$)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |
| 34 | 2-OMe-Phenyl | |

| Nr. | R | Fp(°C)/IR($cm^{-1}$) |
|---|---|---|
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 15

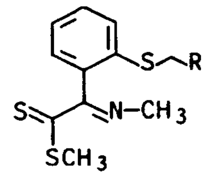

| Nr. | R | Fp($^{o}C$)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp(°C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 16

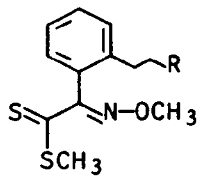

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp($^{o}$C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 17

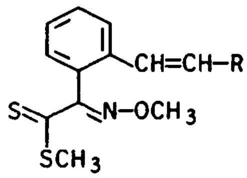

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp(°C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 18

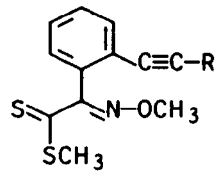

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_2$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp($^oC$)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 19

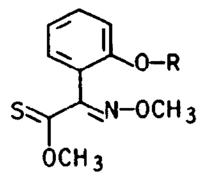

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |
| 35 | 2,4-$Br_2$-phenyl | |

| Nr. | R | Fp($^{o}C$)/IR($cm^{-1}$) |
|---|---|---|
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |
| 77 | $Br_5$-phenyl | |

| Nr. | R | Fp($^oC$)/IR($cm^{-1}$) |
|---|---|---|
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-(i-$C_3H_7)_2$-phenyl | |
| 116 | 2,6-(i-$C_3H_7)_2$-phenyl | |
| 117 | 3,5-(i-$C_3H_7)_2$-phenyl | |
| 118 | 2,4,6-(i-$C_3H_7)_3$-phenyl | |
| 119 | 2,3-(t-$C_4H_9)_2$-phenyl | |

| Nr. | R | Fp(°C)/IR($cm^{-1}$) |
|---|---|---|
| 120 | 2,4-(t-$C_4H_9$)$_2$-phenyl | |
| 121 | 2,5-(t-$C_4H_9$)$_2$-phenyl | |
| 122 | 2,6-(t-$C_4H_9$)$_2$-phenyl | |
| 123 | 3,5-(t-$C_4H_9$)$_2$-phenyl | |
| 124 | 2,4,6-(t-$C_4H_9$)$_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-(t-$C_4H_9$)$_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-(t-$C_4H_9$)$_2$-6-i-$C_3H_7$-phenyl | |
| 135 | 2-cyclo-$C_6H_{11}$-phenyl | |
| 136 | 3-cyclo-$C_6H_{11}$-phenyl | |
| 137 | 4-cyclo-$C_6H_{11}$-phenyl | |
| 138 | 2,4-(cyclo-$C_6H_{11}$)$_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-($CH_3$)$_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |
| 161 | 2-Br, 4-cyclo-$C_6H_{11}$-phenyl | |

| Nr. | R | Fp(°C)/IR($cm^{-1}$) |
|---|---|---|
| 162 | 2-$OCH_3$-phenyl | |
| 163 | 3-$OCH_3$-phenyl | |
| 164 | 4-$OCH_3$-phenyl | |
| 165 | 2,4-$(OCH_3)_2$-phenyl | |
| 166 | 2-$CF_3$-phenyl | |
| 167 | 3-$CF_3$-phenyl | |
| 168 | 4-$CF_3$-phenyl | |
| 169 | 2-$NO_2$-phenyl | |
| 170 | 3-$NO_2$-phenyl | |
| 171 | 4-$NO_2$-phenyl | |
| 172 | 2-CN-phenyl | |
| 173 | 3-CN-phenyl | |
| 174 | 4-CN-phenyl | |
| 175 | 2-$CH_3$, 3-Cl-phenyl | |
| 176 | 2-$CH_3$, 4-Cl-phenyl | |
| 177 | 2-$CH_3$, 5-Cl-phenyl | |
| 178 | 2-$CH_3$, 6-Cl-phenyl | |
| 179 | 2-$CH_3$, 3-F-phenyl | |
| 180 | 2-$CH_3$, 4-F-phenyl | |
| 181 | 2-$CH_3$, 5-F-phenyl | |
| 182 | 2-$CH_3$, 6-F-phenyl | |
| 183 | 2-$CH_3$, 3-Br-phenyl | |
| 184 | 2-$CH_3$, 4-Br-phenyl | |
| 185 | 2-$CH_3$, 5-Br-phenyl | |
| 186 | 2-$CH_3$, 6-Br-phenyl | |
| 187 | 2-Cl, 3-$CH_3$-phenyl | |
| 188 | 2-Cl, 4-$CH_3$-phenyl | |
| 189 | 2-Cl, 5-$CH_3$-phenyl | |
| 190 | 2-Cl, 3-i-$C_3H_7$ | |
| 191 | 2-F, 3-$CH_3$-phenyl | |
| 192 | 2-F, 4-$CH_3$-phenyl | |
| 193 | 2-F, 5-$CH_3$-phenyl | |
| 194 | 2-Br, 3-$CH_3$-phenyl | |
| 195 | 2-Br, 4-$CH_3$-phenyl | |
| 196 | 3-$CH_3$, 4-Cl-phenyl | |
| 197 | 3-$CH_3$, 5-Cl-phenyl | |
| 198 | 2-Br, 5-$CH_3$-phenyl | |
| 199 | 3-$CH_3$, 4-F-phenyl | |
| 200 | 3-$CH_3$, 5-F-phenyl | |
| 201 | 3-$CH_3$, 4-Br-phenyl | |
| 202 | 3-$CH_3$, 5-Br-phenyl | |
| 203 | 3-F, 4-$CH_3$-phenyl | |

| Nr. | R | Fp(°C)/IR($cm^{-1}$) |
|---|---|---|
| 204 | 3-Cl, 4-$CH_3$-phenyl | |
| 205 | 3-Br, 4-$CH_3$-phenyl | |
| 206 | 2-Cl, 4,5-$(CH_3)_2$-phenyl | |
| 207 | 2-Br, 4,5-$(CH_3)_2$-phenyl | |
| 208 | 2-Cl, 3,5-$(CH_3)_2$-phenyl | |
| 209 | 2-Br, 3,5-$(CH_3)_2$-phenyl | |
| 210 | 2,6-$Cl_2$, 4-$CH_3$-phenyl | |
| 211 | 2,6-$F_2$, 4-$CH_3$-phenyl | |
| 212 | 2,6-$Br_2$, 4-$CH_3$-phenyl | |
| 213 | 2,4-$Cl_2$, 6-$CH_3$-phenyl | |
| 214 | 2,4-$F_2$, 6-$CH_3$-phenyl | |
| 215 | 2,4-$Br_2$, 6-$CH_3$-phenyl | |
| 216 | 2,6-$(CH_3)_2$, 4-F-phenyl | |
| 217 | 2,6-$(CH_3)_2$, 4-Cl-phenyl | |
| 218 | 2,6-$(CH_3)_2$, 4-Br-phenyl | |
| 219 | 3,5-$(CH_3)_2$, 4-F-phenyl | |
| 220 | 3,5-$(CH_3)_2$, 4-Cl-phenyl | |
| 221 | 3,5-$(CH_3)_2$, 4-Br-phenyl | |
| 222 | 2,3,6-$(CH_3)_3$, 4-F-phenyl | |
| 223 | 2,3,6-$(CH_3)_3$, 4-Cl-phenyl | |
| 224 | 2,3,6-$(CH_3)_3$, 4-Br-phenyl | |
| 225 | 2,4-$(CH_3)_2$, 6-F-phenyl | |
| 226 | 2,4-$(CH_3)_2$, 6-Cl-phenyl | |
| 227 | 2,4-$(CH_3)_2$, 6-Br-phenyl | |
| 228 | 2-i-$C_3H_7$, 4-Cl, 5-$CH_3$-phenyl | |
| 229 | 2-Cl, 4-$NO_2$-phenyl | |
| 230 | 2-$NO_2$, 4-Cl-phenyl | |
| 231 | 2-$OCH_3$, 5-$NO_2$-phenyl | |
| 232 | 2,4-$Cl_2$, 5-$NO_2$-phenyl | |
| 233 | 2,4-$Cl_2$, 6-$NO_2$-phenyl | |
| 234 | 2,6-$Cl_2$, 4-$NO_2$-phenyl | |
| 235 | 2,6-$Br_2$, 4-$NO_2$-phenyl | |
| 236 | 2,6-$J_2$, 4-$NO_2$-phenyl | |
| 237 | 2-$C_6H_5O$-phenyl | |
| 238 | 3-$C_6H_5O$-phenyl | |
| 239 | 4-$C_6H_5O$-phenyl | |
| 240 | 3-t-$C_4H_9O$-phenyl | |
| 241 | 4-t-$C_4H_9O$-phenyl | |
| 242 | 1-Naphthyl | |
| 243 | 2-Naphthyl | |
| 244 | 2-Pyridyl | |
| 245 | 6-Methyl-2-pyridyl | |

| Nr. | R | Fp(°C)/IR($cm^{-1}$) |
|---|---|---|
| 246 | 6-Ethyl-2-pyridyl | |
| 247 | 6-n-Propyl-2-pyridyl | |
| 248 | 6-iso-Propyl-2-pyridyl | |
| 249 | 6-n-Butyl-2-pyridyl | |
| 250 | 6-tert.-Butyl-2-pyridyl | |
| 251 | 6-n-Pentyl-2-pyridyl | |
| 252 | 6-n-Hexyl-2-pyridyl | |
| 253 | 6-Phenyl-2-pyridyl | |
| 254 | 6-Benzyl-2-pyridyl | |
| 255 | 6-Trifluormethyl-2-pyridyl | |
| 256 | 6-Methoxy-2-pyridyl | |
| 257 | 6-Chloro-2-pyridyl | |
| 258 | 3,6-Dimethyl-2-pyridyl | |
| 259 | 3,6-Diethyl-2-pyridyl | |
| 260 | 4,6-Dimethyl-2-pyridyl | |
| 261 | 5,6-Dimethyl-2-pyridyl | |
| 262 | 4-Phenyl-6-methyl-2-pyridyl | |
| 263 | 4,6-Diphenyl-2-pyridyl | |
| 264 | 3,4-Dichloro-6-methyl-2-pyridyl | |
| 265 | 3,4,5-Trichloro-6-phenyl-2-pyridyl | |
| 266 | 4-Trifluoromethyl-6-methyl-2-pyridyl | |
| 267 | 3-Cyano-6-methyl-2-pyridyl | |
| 268 | 3-Cyano-6-ethyl-2-pyridyl | |
| 269 | 3-Cyano-6-n-propyl-2-pyridyl | |
| 270 | 3-Cyano-6-iso-propyl-2-pyridyl | |
| 271 | 3-Cyano-6-cyclo-propyl-2-pyridyl | |
| 272 | 3-Cyano-6-n-butyl-2-pyridyl | |
| 273 | 3-Cyano-6-tert.-butyl-2-pyridyl | |
| 274 | 3-Cyano-6-cyclo-hexyl-2-pyridyl | |
| 275 | 3-Cyano-6-phenyl-2-pyridyl | |
| 276 | 3-Cyano-4,6-dimethyl-2-pyridyl | |
| 277 | 3,5,6-Trichloro-2-pyridyl | |
| 278 | 5-Trifluoromethyl-2-pyridyl | |
| 279 | 3-Chloro-5-trifluoromethyl-2-pyridyl | |
| 280 | 2-Chinolyl | |
| 281 | 3-Methyl-2-chinolyl | |
| 282 | 4-Methyl-2-chinolyl | |
| 283 | 4-Ethyl-2-chinolyl | |
| 284 | 4-Phenyl-2-chinolyl | |
| 285 | 6-Methyl-2-chinolyl | |
| 286 | 6-Chloro-2-chinolyl | |
| 287 | 8-Methyl-2-chinolyl | |

| Nr. | R | Fp(°C)/IR($cm^{-1}$) |
|---|---|---|
| 288 | 8-Chloro-2-chinolyl | |
| 289 | 3,4-Dimethyl-2-chinolyl | |
| 290 | 4-Methyl-8-methoxy-2-chinolyl | |
| 291 | 4-Phenyl-8-ethoxy-2-chinolyl | |
| 292 | 4-Methyl-8-chloro-2-chinolyl | |
| 293 | 4-Methyl-8-fluoro-2-chinolyl | |
| 294 | 4-Chinolyl | |
| 295 | 2-Methyl-4-chinolyl | |
| 296 | 2-Trifluoromethyl-4-chinolyl | |
| 297 | 2-iso-Propyl-4-chinolyl | |
| 298 | 2-n-Pentyl-4-chinolyl | |
| 299 | 2-Phenyl-4-chinolyl | |
| 300 | 2,6-Dimethyl-4-chinolyl | |
| 301 | 2-Methyl-6-chloro-4-chinolyl | |
| 302 | 2-Methyl-6-fluoro-4-chinolyl | |
| 303 | 8-Chinolyl | |
| 304 | 2-Methyl-8-chinolyl | |
| 305 | 5,7-Dichloro-8-chinolyl | |
| 306 | 4,6-Dimethyl-2-pyrimidinyl | |
| 307 | 4-Trifluormethyl-2-pyrimidinyl | |
| 308 | 4,5,6-Trimethyl-2-pyrimidinyl | |
| 309 | 4-Benzyl-6-methyl-2-pyrimidinyl | |
| 310 | 4-Methyl-6-phenyl-2-pyrimidinyl | |
| 311 | 4,6-Dimethyl-5-chloro-2-pyrimidinyl | |
| 312 | 2,6-Dimethyl-4-pyrimidinyl | |
| 313 | 2,6-Bis-(trifluormethyl)-4-pyrimidinyl | |
| 314 | 2-iso-Propyl-6-methyl-4-pyrimidinyl | |
| 315 | 2-cyclo-Propyl-6-methyl-4-pyrimidinyl | |
| 316 | 2-Methyl-6-methoxymethyl-4-pyrimidinyl | |
| 317 | 2-iso-Propyl-6-methoxymethyl-4-pyrimidinyl | |
| 318 | 2-Phenyl-4-pyrimidinyl | |
| 319 | 3,5-Dimethyl-4-pyrimidinyl | |
| 320 | 2-Methyl-6-trifluoromethyl-4-pyrimidinyl | |
| 321 | 2-n-Propyl-6-trifluoromethyl-4-pyrimidinyl | |
| 322 | 2-iso-Propyl-6-trifluoromethyl-4-pyrimidinyl | |
| 323 | 2-Methyl-5-chloro-6-trifluoromethyl-4-pyrimidinyl | |
| 324 | 2-n-Propyl-5-chloro-6-trifluoromethyl-4-pyrimidinyl | |
| 325 | 2-iso-Propyl-5-chloro-6-trifluoromethyl-4-pyrimidinyl | |
| 326 | 2-tert.-Butyl-5-chloro-6-trifluoromethyl-4-pyrimidinyl | |

Tabelle 20

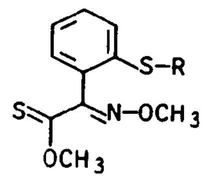

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |
| 35 | 2,4-$Br_2$-phenyl | |

| Nr. | R | Fp(°C)/IR($cm^{-1}$) |
|---|---|---|
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |
| 77 | $Br_5$-phenyl | |

| Nr. | R | Fp(°C)/IR($cm^{-1}$) |
|---|---|---|
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-(i-$C_3H_7)_2$-phenyl | |
| 116 | 2,6-(i-$C_3H_7)_2$-phenyl | |
| 117 | 3,5-(i-$C_3H_7)_2$-phenyl | |
| 118 | 2,4,6-(i-$C_3H_7)_3$-phenyl | |
| 119 | 2,3-(t-$C_4H_9)_2$-phenyl | |

| Nr. | R | Fp($^{o}$C)/IR($cm^{-1}$) |
|---|---|---|
| 120 | 2,4-(t-$C_4H_9$)$_2$-phenyl | |
| 121 | 2,5-(t-$C_4H_9$)$_2$-phenyl | |
| 122 | 2,6-(t-$C_4H_9$)$_2$-phenyl | |
| 123 | 3,5-(t-$C_4H_9$)$_2$-phenyl | |
| 124 | 2,4,6-(t-$C_4H_9$)$_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-(t-$C_4H_9$)$_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-(t-$C_4H_9$)$_2$-6-i-$C_3H_7$-phenyl | |
| 135 | 2-cyclo-$C_6H_{11}$-phenyl | |
| 136 | 3-cyclo-$C_6H_{11}$-phenyl | |
| 137 | 4-cyclo-$C_6H_{11}$-phenyl | |
| 138 | 2,4-(cyclo-$C_6H_{11}$)$_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-($CH_3$)$_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |
| 161 | 2-Br, 4-cyclo-$C_6H_{11}$-phenyl | |

| Nr. | R | Fp(°C)/IR($cm^{-1}$) |
|---|---|---|
| 162 | 3-$OCH_3$-phenyl | |
| 163 | 2,4-$(OCH_3)_2$-phenyl | |
| 164 | 2-$CF_3$-phenyl | |
| 165 | 3-$CF_3$-phenyl | |
| 166 | 4-$CF_3$-phenyl | |
| 167 | 2-$NO_2$-phenyl | |
| 168 | 3-$NO_2$-phenyl | |
| 169 | 4-$NO_2$-phenyl | |
| 170 | 2-CN-phenyl | |
| 171 | 3-CN-phenyl | |
| 172 | 4-CN-phenyl | |
| 173 | 2-$CH_3$, 3-Cl-phenyl | |
| 174 | 2-$CH_3$, 4-Cl-phenyl | |
| 175 | 2-$CH_3$, 5-Cl-phenyl | |
| 176 | 2-$CH_3$, 6-Cl-phenyl | |
| 177 | 2-$CH_3$, 3-F-phenyl | |
| 178 | 2-$CH_3$, 4-F-phenyl | |
| 179 | 2-$CH_3$, 5-F-phenyl | |
| 180 | 2-$CH_3$, 6-F-phenyl | |
| 181 | 2-$CH_3$, 3-Br-phenyl | |
| 182 | 2-$CH_3$, 4-Br-phenyl | |
| 183 | 2-$CH_3$, 5-Br-phenyl | |
| 184 | 2-$CH_3$, 6-Br-phenyl | |
| 185 | 2-Cl, 3-$CH_3$-phenyl | |
| 186 | 2-Cl, 4-$CH_3$-phenyl | |
| 187 | 2-Cl, 5-$CH_3$-phenyl | |
| 188 | 2-Cl, 3-i-$C_3H_7$ | |
| 189 | 2-F, 3-$CH_3$-phenyl | |
| 190 | 2-F, 4-$CH_3$-phenyl | |
| 191 | 2-F, 5-$CH_3$-phenyl | |
| 192 | 2-Br, 3-$CH_3$-phenyl | |
| 193 | 2-Br, 4-$CH_3$-phenyl | |
| 194 | 3-$CH_3$, 4-Cl-phenyl | |
| 195 | 3-$CH_3$, 5-Cl-phenyl | |
| 196 | 2-Br, 5-$CH_3$-phenyl | |
| 197 | 3-$CH_3$, 4-F-phenyl | |
| 198 | 3-$CH_3$, 5-F-phenyl | |
| 199 | 3-$CH_3$, 4-Br-phenyl | |
| 200 | 3-$CH_3$, 5-Br-phenyl | |
| 201 | 3-F, 4-$CH_3$-phenyl | |
| 202 | 3-Cl, 4-$CH_3$-phenyl | |
| 203 | 3-Br, 4-$CH_3$-phenyl | |

| Nr. | R | Fp(°C)/IR($cm^{-1}$) |
|---|---|---|
| 204 | 2-Cl, 4,5-$(CH_3)_2$-phenyl | |
| 205 | 2-Br, 4,5-$(CH_3)_2$-phenyl | |
| 206 | 2-Cl, 3,5-$(CH_3)_2$-phenyl | |
| 207 | 2-Br, 3,5-$(CH_3)_2$-phenyl | |
| 208 | 2,6-$Cl_2$, 4-$CH_3$-phenyl | |
| 209 | 2,6-$F_2$, 4-$CH_3$-phenyl | |
| 210 | 2,6-$Br_2$, 4-$CH_3$-phenyl | |
| 211 | 2,4-$Cl_2$, 6-$CH_3$-phenyl | |
| 212 | 2,4-$F_2$, 6-$CH_3$-phenyl | |
| 213 | 2,4-$Br_2$, 6-$CH_3$-phenyl | |
| 214 | 2,6-$(CH_3)_2$, 4-F-phenyl | |
| 215 | 2,6-$(CH_3)_2$, 4-Cl-phenyl | |
| 216 | 2,6-$(CH_3)_2$, 4-Br-phenyl | |
| 217 | 3,5-$(CH_3)_2$, 4-F-phenyl | |
| 218 | 3,5-$(CH_3)_2$, 4-Cl-phenyl | |
| 219 | 3,5-$(CH_3)_2$, 4-Br-phenyl | |
| 220 | 2,3,6-$(CH_3)_3$, 4-F-phenyl | |
| 221 | 2,3,6-$(CH_3)_3$, 4-Cl-phenyl | |
| 222 | 2,3,6-$(CH_3)_3$, 4-Br-phenyl | |
| 223 | 2,4-$(CH_3)_2$, 6-F-phenyl | |
| 224 | 2,4-$(CH_3)_2$, 6-Cl-phenyl | |
| 225 | 2,4-$(CH_3)_2$, 6-Br-phenyl | |
| 226 | 2-i-$C_3H_7$, 4-Cl, 5-$CH_3$-phenyl | |
| 227 | 2-Cl, 4-$NO_2$-phenyl | |
| 228 | 2-$NO_2$, 4-Cl-phenyl | |
| 229 | 2-$OCH_3$, 5-$NO_2$-phenyl | |
| 230 | 2,4-$Cl_2$, 5-$NO_2$-phenyl | |
| 231 | 2,4-$Cl_2$, 6-$NO_2$-phenyl | |
| 232 | 2,6-$Cl_2$, 4-$NO_2$-phenyl | |
| 233 | 2,6-$Br_2$, 4-$NO_2$-phenyl | |
| 234 | 2,6-$J_2$, 4-$NO_2$-phenyl | |
| 235 | 2-$C_6H_5O$-phenyl | |
| 236 | 3-$C_6H_5O$-phenyl | |
| 237 | 4-$C_6H_5O$-phenyl | |
| 238 | 3-t-$C_4H_9O$-phenyl | |
| 239 | 4-t-$C_4H_9O$-phenyl | |
| 240 | 1-Naphthyl | |
| 241 | 2-Naphthyl | |
| 242 | 2-Pyridyl | |
| 243 | 6-Methyl-2-pyridyl | |
| 244 | 6-Ethyl-2-pyridyl | |
| 245 | 6-n-Propyl-2-pyridyl | |

| Nr. | R | Fp(°C)/IR($cm^{-1}$) |
|---|---|---|
| 246 | 6-iso-Propyl-2-pyridyl | |
| 247 | 6-n-Butyl-2-pyridyl | |
| 248 | 6-tert.-Butyl-2-pyridyl | |
| 249 | 6-n-Pentyl-2-pyridyl | |
| 250 | 6-n-Hexyl-2-pyridyl | |
| 251 | 6-Phenyl-2-pyridyl | |
| 252 | 6-Benzyl-2-pyridyl | |
| 253 | 6-Trifluormethyl-2-pyridyl | |
| 254 | 6-Methoxy-2-pyridyl | |
| 255 | 6-Chloro-2-pyridyl | |
| 256 | 3,6-Dimethyl-2-pyridyl | |
| 257 | 3,6-Diethyl-2-pyridyl | |
| 258 | 4,6-Dimethyl-2-pyridyl | |
| 259 | 5,6-Dimethyl-2-pyridyl | |
| 260 | 4-Phenyl-6-methyl-2-pyridyl | |
| 261 | 4,6-Diphenyl-2-pyridyl | |
| 262 | 3,4-Dichloro-6-methyl-2-pyridyl | |
| 263 | 3,4,5-Trichloro-6-phenyl-2-pyridyl | |
| 264 | 4-Trifluoromethyl-6-methyl-2-pyridyl | |
| 265 | 3-Cyano-6-methyl-2-pyridyl | |
| 266 | 3-Cyano-6-ethyl-2-pyridyl | |
| 267 | 3-Cyano-6-n-propyl-2-pyridyl | |
| 268 | 3-Cyano-6-iso-propyl-2-pyridyl | |
| 269 | 3-Cyano-6-cyclo-propyl-2-pyridyl | |
| 270 | 3-Cyano-6-n-butyl-2-pyridyl | |
| 271 | 3-Cyano-6-tert.-butyl-2-pyridyl | |
| 272 | 3-Cyano-6-cyclo-hexyl-2-pyridyl | |
| 273 | 3-Cyano-6-phenyl-2-pyridyl | |
| 274 | 3-Cyano-4,6-dimethyl-2-pyridyl | |
| 275 | 3,5,6-Trichloro-2-pyridyl | |
| 276 | 5-Trifluoromethyl-2-pyridyl | |
| 277 | 3-Chloro-5-trifluoromethyl-2-pyridyl | |
| 278 | 2-Chinolyl | |
| 279 | 3-Methyl-2-chinolyl | |
| 280 | 4-Methyl-2-chinolyl | |
| 281 | 4-Ethyl-2-chinolyl | |
| 282 | 4-Phenyl-2-chinolyl | |
| 283 | 6-Methyl-2-chinolyl | |
| 284 | 6-Chloro-2-chinolyl | |
| 285 | 8-Methyl-2-chinolyl | |
| 286 | 8-Chloro-2-chinolyl | |
| 287 | 3,4-Dimethyl-2-chinolyl | |

| Nr. | R | Fp(°C)/IR($cm^{-1}$) |
|---|---|---|
| 288 | 4-Methyl-8-methoxy-2-chinolyl | |
| 289 | 4-Phenyl-8-ethoxy-2-chinolyl | |
| 290 | 4-Methyl-8-chloro-2-chinolyl | |
| 291 | 4-Methyl-8-fluoro-2-chinolyl | |
| 292 | 4-Chinolyl | |
| 293 | 2-Methyl-4-chinolyl | |
| 294 | 2-Trifluoromethyl-4-chinolyl | |
| 295 | 2-iso-Propyl-4-chinolyl | |
| 296 | 2-n-Pentyl-4-chinolyl | |
| 297 | 2-Phenyl-4-chinolyl | |
| 298 | 2,6-Dimethyl-4-chinolyl | |
| 299 | 2-Methyl-6-chloro-4-chinolyl | |
| 300 | 2-Methyl-6-fluoro-4-chinolyl | |
| 301 | 8-Chinolyl | |
| 302 | 2-Methyl-8-chinolyl | |
| 303 | 5,7-Dichloro-8-chinolyl | |
| 304 | 4,6-Dimethyl-2-pyrimidinyl | |
| 305 | 4-Trifluormethyl-2-pyrimidinyl | |
| 306 | 4,5,6-Trimethyl-2-pyrimidinyl | |
| 307 | 4-Benzyl-6-methyl-2-pyrimidinyl | |
| 308 | 4-Methyl-6-phenyl-2-pyrimidinyl | |
| 309 | 4,6-Dimethyl-5-chloro-2-pyrimidinyl | |
| 310 | 2,6-Dimethyl-4-pyrimidinyl | |
| 311 | 2,6-Bis-(trifluormethyl)-4-pyrimidinyl | |
| 312 | 2-iso-Propyl-6-methyl-4-pyrimidinyl | |
| 313 | 2-cyclo-Propyl-6-methyl-4-pyrimidinyl | |
| 314 | 2-Methyl-6-methoxymethyl-4-pyrimidinyl | |
| 315 | 2-iso-Propyl-6-methoxymethyl-4-pyrimidinyl | |
| 316 | 2-Phenyl-4-pyrimidinyl | |
| 317 | 3,5-Dimethyl-4-pyrimidinyl | |
| 318 | 2-Methyl-6-trifluoromethyl-4-pyrimidinyl | |
| 319 | 2-n-Propyl-6-trifluoromethyl-4-pyrimidinyl | |
| 320 | 2-iso-Propyl-6-trifluoromethyl-4-pyrimidinyl | |
| 321 | 2-Methyl-5-chloro-6-trifluoromethyl-4-pyrimidinyl | |
| 322 | 2-n-Propyl-5-chloro-6-trifluoromethyl-4-pyrimidinyl | |
| 323 | 2-iso-Propyl-5-chloro-6-trifluoromethyl-4-pyrimidinyl | |
| 324 | 2-tert.-Butyl-5-chloro-6-trifluoromethyl-4-pyrimidinyl | |
| 325 | 6-EtO-benzothiazol-2-yl | |
| 326 | Benzoxazol-2-yl | |
| 327 | 1-Me-imidazol-2-yl | |
| 328 | 4-But-1H-imidazol-2-yl | |
| 329 | 2-Thiazolin-2-yl | |

| Nr. | R | Fp(°C)/IR($cm^{-1}$) |
|---|---|---|
| 330 | 5-$CF_3$-benzimidazol-2-yl | |
| 331 | 1-Ph-tetrazol-5-yl | |
| 332 | 5-$CF_3$-benzothiazol-2-yl | |
| 333 | 4,4-$Me_2$-5-methylene-2-thiazolin-2-yl | |
| 334 | 6-Cl-4-Me-benzothiazol-2-yl | |
| 335 | 5-Me-benzothiazol-2-yl | |
| 336 | 4-Cl-benzothiazol-2-yl | |
| 337 | 1-(3-$NO_2$-Ph)tetrazol-5-yl | |
| 338 | 2-thienyl | |
| 339 | 5-Me-benzoxazol-2-yl | |
| 340 | 7-Cl-benzothiazol-2-yl | |
| 341 | 5,6-$Cl_3$-1H-benzimidazol-2-yl | |
| 342 | 5-Cl-benzoxazol-2-yl | |
| 343 | 6-Cl-benzothiazol-2-yl | |
| 344 | 2-Ph-thiazol-2-yl | |
| 345 | 3-CN-4,6-$Me_2$-2-pyridyl | |
| 346 | 1-Ph-1,2,4-triazol-3-yl | |
| 347 | 1-Pri-benzimidazol-2-yl | |
| 348 | 5-Br-benzothiazol-2-yl | |
| 349 | 5-Br-1H-benzimidazol-2-yl | |
| 350 | 7-Cl-4-MeO-benzothiazol-2-yl | |
| 351 | 1H-benzimidazol-2-yl | |
| 352 | 5-Cl-benzothiazol-2-yl | |
| 353 | 5-$NO_2$-benzoxazol-2-yl | |
| 354 | 5-t-Bu-benzoxazol-2-yl | |
| 355 | 4,6,7-$Cl_3$-benzothiozol-2-yl | |
| 356 | 5-Ph-thiazol-2-yl | |
| 357 | 5,7-$Me_2$-benzoxazol-2-yl | |
| 358 | 6-Me-benzoxazol-2-yl | |
| 359 | 1,2,4-triazin-3-yl | |
| 360 | 6-Pr-benzothiazol-2-yl | |
| 361 | 6-PhO-benzothiazol-2-yl | |
| 362 | 4-(4-Cl-Ph)-thiazol-2-yl | |
| 363 | 4-(4-Me-Ph)-thiazol-2-yl | |
| 364 | 5-Me-4-Ph-thiazol-2-yl | |
| 365 | 5-Cl-1-H-benzimidazol-2-yl | |
| 366 | 5-Ph-1,2,4-triazol-3-yl | |
| 367 | 3-Ph-1,2,4-thiadiazol-5-yl | |

Tabelle 21

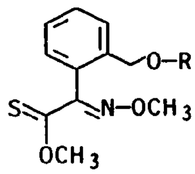

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | 58- 60°C |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-$CH_3$-Phenyl | 1662, 1494, 1461, 1240, 1190, 1122, 1051, 1025, 845, 753 ($cm^{-1}$) |
| 25 | 2-CN-Phenyl | |
| 26 | 3-CN-Phenyl | |
| 27 | 4-CN-Phenyl | |
| 28 | 2,3-$F_2$-phenyl | |
| 29 | 2,4-$F_2$-phenyl | |
| 30 | 2,3-$Cl_2$-phenyl | |
| 31 | 2,4-$Cl_2$-phenyl | |
| 32 | 2,5-$Cl_2$-phenyl | |
| 33 | 2,6-$Cl_2$-phenyl | |
| 34 | 3,4-$Cl_2$-phenyl | |

| Nr. | R | Fp($^{o}C$)/IR($cm^{-1}$) |
|---|---|---|
| 35 | 3,5-$Cl_2$-phenyl | |
| 36 | 2,4-$Br_2$-phenyl | |
| 37 | 2,5-$Br_2$-phenyl | |
| 38 | 2,6-$Br_2$-phenyl | |
| 39 | 2,4-$J_2$-phenyl | |
| 40 | 2-Cl, 3-F-phenyl | |
| 41 | 2-Cl, 4-F-phenyl | |
| 42 | 2-Cl, 5-F-phenyl | |
| 43 | 2-Cl, 6-F-phenyl | |
| 44 | 2-Cl, 3-Br-phenyl | |
| 45 | 2-Cl, 4-Br-phenyl | |
| 46 | 2-Cl, 5-Br-phenyl | |
| 47 | 2-Cl, 6-Br-phenyl | |
| 48 | 2-Br, 3-Cl-phenyl | |
| 49 | 2-Br, 4-Cl-phenyl | |
| 50 | 2-Br, 3-F-phenyl | |
| 51 | 2-Br, 4-F-phenyl | |
| 52 | 2-Br, 5-F-phenyl | |
| 53 | 2-Br, 6-F-phenyl | |
| 54 | 2-F, 3-Cl-phenyl | |
| 55 | 2-F, 4-Cl-phenyl | |
| 56 | 2-F, 5-Cl-phenyl | |
| 57 | 3-Cl, 4-F-phenyl | |
| 58 | 3-Cl, 5-F-phenyl | |
| 59 | 3-Cl, 4-Br-phenyl | |
| 60 | 3-Cl, 5-Br-phenyl | |
| 61 | 3-F, 4-Cl-phenyl | |
| 62 | 3-F, 4-Br-phenyl | |
| 63 | 3-Br, 4-Cl-phenyl | |
| 64 | 3-Br, 4-F-phenyl | |
| 65 | 2,4,6-$F_3$-phenyl | |
| 66 | 2,3,4,-$Cl_3$-phenyl | |
| 67 | 2,3,5-$Cl_3$-phenyl | |
| 68 | 2,3,6-$Cl_3$-phenyl | |
| 69 | 2,4,5-$Cl_3$-phenyl | |
| 70 | 2,4,6-$Cl_3$-phenyl | |
| 71 | 3,4,5-$Cl_3$-phenyl | |
| 72 | 2,4,6-$Br_3$-phenyl | |
| 73 | 2,6-$Cl_2$-4-Br-phenyl | |
| 74 | 2,3,4,6-$Cl_4$-phenyl | |
| 75 | 2,3,5,6-$Cl_4$-phenyl | |
| 76 | $F_5$-phenyl | |

| Nr. | R | Fp($^{o}$C)/IR($cm^{-1}$) |
|---|---|---|
| 77 | $Cl_5$-phenyl | |
| 78 | $Br_5$-phenyl | |
| 79 | 2-$CH_3$-phenyl | |
| 80 | 3-$CH_3$-phenyl | |
| 81 | 4-$CH_3$-phenyl | |
| 82 | 2-$C_2H_5$-phenyl | |
| 83 | 3-$C_2H_5$-phenyl | |
| 84 | 4-$C_2H_5$-phenyl | |
| 85 | 2-n-$C_3H_7$-phenyl | |
| 86 | 3-n-$C_3H_7$-phenyl | |
| 87 | 4-n-$C_3H_7$-phenyl | |
| 88 | 2-i-$C_3H_7$-phenyl | |
| 89 | 3-i-$C_3H_7$-phenyl | |
| 90 | 4-i-$C_3H_7$-phenyl | |
| 91 | 2-s-$C_4H_9$-phenyl | |
| 92 | 3-s-$C_4H_9$-phenyl | |
| 93 | 4-s-$C_4H_9$-phenyl | |
| 94 | 2-t-$C_4H_9$-phenyl | |
| 95 | 3-t-$C_4H_9$-phenyl | |
| 96 | 4-t-$C_4H_9$-phenyl | |
| 97 | 2,3-$(CH_3)_2$-phenyl | |
| 98 | 2,4-$(CH_3)_2$-phenyl | |
| 99 | 2,5-$(CH_3)_2$-phenyl | |
| 100 | 2,6-$(CH_3)_2$-phenyl | |
| 101 | 3,4-$(CH_3)_2$-phenyl | |
| 102 | 3,5-$(CH_3)_2$-phenyl | |
| 103 | 2,3,4-$(CH_3)_3$-phenyl | |
| 104 | 2,3,5-$(CH_3)_3$-phenyl | |
| 105 | 2,3,6-$(CH_3)_3$-phenyl | |
| 106 | 2,4,5-$(CH_3)_3$-phenyl | |
| 107 | 2,4,6-$(CH_3)_3$-phenyl | |
| 108 | 3,4,5-$(CH_3)_3$-phenyl | |
| 109 | 2,3,4,6-$(CH_3)_4$-phenyl | |
| 110 | 2,3,5,6-$(CH_3)_4$-phenyl | |
| 111 | $(CH_3)_5$-phenyl | |
| 112 | 2,4-$(C_2H_5)_2$-phenyl | |
| 113 | 2,6-$(C_2H_5)_2$-phenyl | |
| 114 | 3,5-$(C_2H_5)_2$-phenyl | |
| 115 | 2,4,6-$(C_2H_5)_3$-phenyl | |
| 116 | 2,4-$(i\text{-}C_3H_7)_2$-phenyl | |
| 117 | 2,6-$(i\text{-}C_3H_7)_2$-phenyl | |
| 118 | 3,5-$(i\text{-}C_3H_7)_2$-phenyl | |

| Nr. | R | Fp($^{o}C$)/IR($cm^{-1}$) |
|---|---|---|
| 119 | 2,4,6-(i-$C_3H_7$)$_3$-phenyl | |
| 120 | 2,3-(t-$C_4H_9$)$_2$-phenyl | |
| 121 | 2,4-(t-$C_4H_9$)$_2$-phenyl | |
| 122 | 2,5-(t-$C_4H_9$)$_2$-phenyl | |
| 123 | 2,6-(t-$C_4H_9$)$_2$-phenyl | |
| 124 | 3,5-(t-$C_4H_9$)$_2$-phenyl | |
| 125 | 2,4,6-(t-$C_4H_9$)$_3$-phenyl | |
| 126 | 2-t-$C_4H_9$, 4-$CH_3$-phenyl | |
| 127 | 2-t-$C_4H_9$, 5-$CH_3$-phenyl | |
| 128 | 2,6-(t-$C_4H_9$)$_2$, 4-$CH_3$-phenyl | |
| 129 | 2-$CH_3$, 4-t-$C_4H_9$-phenyl | |
| 130 | 2-$CH_3$, 6-t-$C_4H_9$-phenyl | |
| 131 | 1-$CH_3$, 4-i-$C_3H_7$-phenyl | |
| 132 | 2-$CH_3$, 5-i-$C_3H_7$-phenyl | |
| 133 | 3-$CH_3$, 4-i-$C_3H_7$-phenyl | |
| 134 | 2-i-$C_3H_7$, 5-$CH_3$-phenyl | |
| 135 | 2,4-(t-$C_4H_9$)$_2$-6-i-$C_3H_7$-phenyl | |
| 136 | 2-cyclo-$C_6H_{11}$-phenyl | |
| 137 | 3-cyclo-$C_6H_{11}$-phenyl | |
| 138 | 4-cyclo-$C_6H_{11}$-phenyl | |
| 139 | 2,4-(cyclo-$C_6H_{11}$)$_2$-6-$CH_3$-phenyl | |
| 140 | 2-$CH_3$, 4-cyclo-$C_6H_{11}$-phenyl | |
| 141 | 2-$CH_2C_6H_5$-phenyl | |
| 142 | 3-$CH_2C_6H_5$-phenyl | |
| 143 | 4-$CH_2C_6H_5$-phenyl | |
| 144 | 2-$CH_2C_6H_5$, 4-$CH_3$-phenyl | |
| 145 | 2-$CH_3$, 4-$CH_2C_6H_5$-phenyl | |
| 146 | 2-$C_6H_5$-phenyl | |
| 147 | 3-$C_6H_5$-phenyl | |
| 148 | 4-$C_6H_5$-phenyl | |
| 149 | 4-(2-i-$C_3H_7$-$C_6H_4$)-phenyl | |
| 150 | 4-$C_6H_5$, 2,6-($CH_3$)$_2$-phenyl | |
| 151 | 2-Cl, 4-$C_6H_5$-phenyl | |
| 152 | 2-Br, 4-$C_6H_5$-phenyl | |
| 153 | 2-$C_6H_5$, 4-Cl-phenyl | |
| 154 | 2-$C_6H_5$, 4-Br-phenyl | |
| 155 | 2-$CH_2C_6H_5$, 4-Cl-phenyl | |
| 156 | 2-$CH_2C_6H_5$, 4-Br-phenyl | |
| 157 | 2-Cl, 4-$CH_2C_6H_5$-phenyl | |
| 158 | 2-Br, 4-$CH_2C_6H_5$-phenyl | |
| 159 | 2-cyclo-$C_6H_{11}$, 4-Cl-phenyl | |
| 160 | 2-cyclo-$C_6H_{11}$, 4-Br-phenyl | |

| Nr. | R | Fp(°C)/IR($cm^{-1}$) |
|---|---|---|
| 161 | 2-Cl, 4-cyclo-$C_6H_{11}$-phenyl | |
| 162 | 2-Br, 4-cyclo-$C_6H_{11}$-phenyl | |
| 163 | 3-$OCH_3$-phenyl | |
| 164 | 2,4-$(OCH_3)_2$-phenyl | |
| 165 | 2-$CF_3$-phenyl | |
| 166 | 3-$CF_3$-phenyl | |
| 167 | 4-$CF_3$-phenyl | |
| 168 | 2-$NO_2$-phenyl | |
| 169 | 3-$NO_2$-phenyl | |
| 170 | 4-$NO_2$-phenyl | |
| 171 | 2-CN-phenyl | |
| 172 | 3-CN-phenyl | |
| 173 | 4-CN-phenyl | |
| 174 | 2-$CH_3$, 3-Cl-phenyl | |
| 175 | 2-$CH_3$, 4-Cl-phenyl | |
| 176 | 2-$CH_3$, 5-Cl-phenyl | |
| 177 | 2-$CH_3$, 6-Cl-phenyl | |
| 178 | 2-$CH_3$, 3-F-phenyl | |
| 179 | 2-$CH_3$, 4-F-phenyl | |
| 180 | 2-$CH_3$, 5-F-phenyl | |
| 181 | 2-$CH_3$, 6-F-phenyl | |
| 182 | 2-$CH_3$, 3-Br-phenyl | |
| 183 | 2-$CH_3$, 4-Br-phenyl | |
| 184 | 2-$CH_3$, 5-Br-phenyl | |
| 185 | 2-$CH_3$, 6-Br-phenyl | |
| 186 | 2-Cl, 3-$CH_3$-phenyl | |
| 187 | 2-Cl, 4-$CH_3$-phenyl | |
| 188 | 2-Cl, 5-$CH_3$-phenyl | |
| 189 | 2-Cl, 3-i-$C_3H_7$ | |
| 190 | 2-F, 3-$CH_3$-phenyl | |
| 191 | 2-F, 4-$CH_3$-phenyl | |
| 192 | 2-F, 5-$CH_3$-phenyl | |
| 193 | 2-Br, 3-$CH_3$-phenyl | |
| 194 | 2-Br, 4-$CH_3$-phenyl | |
| 195 | 3-$CH_3$, 4-Cl-phenyl | |
| 196 | 3-$CH_3$, 5-Cl-phenyl | |
| 197 | 2-Br, 5-$CH_3$-phenyl | |
| 198 | 3-$CH_3$, 4-F-phenyl | |
| 199 | 3-$CH_3$, 5-F-phenyl | |
| 200 | 3-$CH_3$, 4-Br-phenyl | |
| 201 | 3-$CH_3$, 5-Br-phenyl | |
| 202 | 3-F, 4-$CH_3$-phenyl | |

| Nr. | R | Fp(°C)/IR($cm^{-1}$) |
|---|---|---|
| 203 | 3-Cl, 4-$CH_3$-phenyl | |
| 204 | 3-Br, 4-$CH_3$-phenyl | |
| 205 | 2-Cl, 4,5-$(CH_3)_2$-phenyl | |
| 206 | 2-Br, 4,5-$(CH_3)_2$-phenyl | |
| 207 | 2-Cl, 3,5-$(CH_3)_2$-phenyl | |
| 208 | 2-Br, 3,5-$(CH_3)_2$-phenyl | |
| 209 | 2,6-$Cl_2$, 4-$CH_3$-phenyl | |
| 210 | 2,6-$F_2$, 4-$CH_3$-phenyl | |
| 211 | 2,6-$Br_2$, 4-$CH_3$-phenyl | |
| 212 | 2,4-$Cl_2$, 6-$CH_3$-phenyl | |
| 213 | 2,4-$F_2$, 6-$CH_3$-phenyl | |
| 214 | 2,4-$Br_2$, 6-$CH_3$-phenyl | |
| 215 | 2,6-$(CH_3)_2$, 4-F-phenyl | |
| 216 | 2,6-$(CH_3)_2$, 4-Cl-phenyl | |
| 217 | 2,6-$(CH_3)_2$, 4-Br-phenyl | |
| 218 | 3,5-$(CH_3)_2$, 4-F-phenyl | |
| 219 | 3,5-$(CH_3)_2$, 4-Cl-phenyl | |
| 220 | 3,5-$(CH_3)_2$, 4-Br-phenyl | |
| 221 | 2,3,6-$(CH_3)_3$, 4-F-phenyl | |
| 222 | 2,3,6-$(CH_3)_3$, 4-Cl-phenyl | |
| 223 | 2,3,6-$(CH_3)_3$, 4-Br-phenyl | |
| 224 | 2,4-$(CH_3)_2$, 6-F-phenyl | |
| 225 | 2,4-$(CH_3)_2$, 6-Cl-phenyl | |
| 226 | 2,4-$(CH_3)_2$, 6-Br-phenyl | |
| 227 | 2-i-$C_3H_7$, 4-Cl, 5-$CH_3$-phenyl | |
| 228 | 2-Cl, 4-$NO_2$-phenyl | |
| 229 | 2-$NO_2$, 4-Cl-phenyl | |
| 230 | 2-$OCH_3$, 5-$NO_2$-phenyl | |
| 231 | 2,4-$Cl_2$, 5-$NO_2$-phenyl | |
| 232 | 2,4-$Cl_2$, 6-$NO_2$-phenyl | |
| 233 | 2,6-$Cl_2$, 4-$NO_2$-phenyl | |
| 234 | 2,6-$Br_2$, 4-$NO_2$-phenyl | |
| 235 | 2,6-$J_2$, 4-$NO_2$-phenyl | |
| 236 | 2-$C_6H_5O$-phenyl | |
| 237 | 3-$C_6H_5O$-phenyl | |
| 238 | 4-$C_6H_5O$-phenyl | |
| 239 | 3-t-$C_4H_9O$-phenyl | |
| 240 | 4-t-$C_4H_9O$-phenyl | |
| 241 | 1-Naphthyl | |
| 242 | 2-Naphthyl | |
| 243 | 2-Pyridyl | |
| 244 | 6-Methyl-2-pyridyl | |

| Nr. | R | Fp($^{o}$C)/IR(cm$^{-1}$) |
|---|---|---|
| 245 | 6-Ethyl-2-pyridyl | |
| 246 | 6-n-Propyl-2-pyridyl | |
| 247 | 6-iso-Propyl-2-pyridyl | |
| 248 | 6-n-Butyl-2-pyridyl | |
| 249 | 6-tert.-Butyl-2-pyridyl | |
| 250 | 6-n-Pentyl-2-pyridyl | |
| 251 | 6-n-Hexyl-2-pyridyl | |
| 252 | 6-Phenyl-2-pyridyl | |
| 253 | 6-Benzyl-2-pyridyl | |
| 254 | 6-Trifluormethyl-2-pyridyl | |
| 255 | 6-Methoxy-2-pyridyl | |
| 256 | 6-Chloro-2-pyridyl | |
| 257 | 3,6-Dimethyl-2-pyridyl | |
| 258 | 3,6-Diethyl-2-pyridyl | |
| 259 | 4,6-Dimethyl-2-pyridyl | |
| 260 | 5,6-Dimethyl-2-pyridyl | |
| 261 | 4-Phenyl-6-methyl-2-pyridyl | |
| 262 | 4,6-Diphenyl-2-pyridyl | |
| 263 | 3,4-Dichloro-6-methyl-2-pyridyl | |
| 264 | 3,4,5-Trichloro-6-phenyl-2-pyridyl | |
| 265 | 4-Trifluoromethyl-6-methyl-2-pyridyl | |
| 266 | 3-Cyano-6-methyl-2-pyridyl | |
| 267 | 3-Cyano-6-ethyl-2-pyridyl | |
| 268 | 3-Cyano-6-n-propyl-2-pyridyl | |
| 269 | 3-Cyano-6-iso-propyl-2-pyridyl | |
| 270 | 3-Cyano-6-cyclo-propyl-2-pyridyl | |
| 271 | 3-Cyano-6-n-butyl-2-pyridyl | |
| 272 | 3-Cyano-6-tert.-butyl-2-pyridyl | |
| 273 | 3-Cyano-6-cyclo-hexyl-2-pyridyl | |
| 274 | 3-Cyano-6-phenyl-2-pyridyl | |
| 275 | 3-Cyano-4,6-dimethyl-2-pyridyl | |
| 276 | 3,5,6-Trichloro-2-pyridyl | |
| 277 | 5-Trifluoromethyl-2-pyridyl | |
| 278 | 3-Chloro-5-trifluoromethyl-2-pyridyl | |
| 279 | 2-Chinolyl | |
| 280 | 3-Methyl-2-chinolyl | |
| 281 | 4-Methyl-2-chinolyl | |
| 282 | 4-Ethyl-2-chinolyl | |
| 283 | 4-Phenyl-2-chinolyl | |
| 284 | 6-Methyl-2-chinolyl | |
| 285 | 6-Chloro-2-chinolyl | |
| 286 | 8-Methyl-2-chinolyl | |

| Nr. | R | Fp(°C)/IR($cm^{-1}$) |
|---|---|---|
| 287 | 8-Chloro-2-chinolyl | |
| 288 | 3,4-Dimethyl-2-chinolyl | |
| 289 | 4-Methyl-8-methoxy-2-chinolyl | |
| 290 | 4-Phenyl-8-ethoxy-2-chinolyl | |
| 291 | 4-Methyl-8-chloro-2-chinolyl | |
| 292 | 4-Methyl-8-fluoro-2-chinolyl | |
| 293 | 4-Chinolyl | |
| 294 | 2-Methyl-4-chinolyl | |
| 295 | 2-Trifluoromethyl-4-chinolyl | |
| 296 | 2-iso-Propyl-4-chinolyl | |
| 297 | 2-n-Pentyl-4-chinolyl | |
| 298 | 2-Phenyl-4-chinolyl | |
| 299 | 2,6-Dimethyl-4-chinolyl | |
| 300 | 2-Methyl-6-chloro-4-chinolyl | |
| 301 | 2-Methyl-6-fluoro-4-chinolyl | |
| 302 | 8-Chinolyl | |
| 303 | 2-Methyl-8-chinolyl | |
| 304 | 5,7-Dichloro-8-chinolyl | |
| 305 | 4,6-Dimethyl-2-pyrimidinyl | |
| 306 | 4-Trifluormethyl-2-pyrimidinyl | |
| 307 | 4,5,6-Trimethyl-2-pyrimidinyl | |
| 308 | 4-Benzyl-6-methyl-2-pyrimidinyl | |
| 309 | 4-Methyl-6-phenyl-2-pyrimidinyl | |
| 310 | 4,6-Dimethyl-5-chloro-2-pyrimidinyl | |
| 311 | 2,6-Dimethyl-4-pyrimidinyl | |
| 312 | 2,6-Bis-(trifluormethyl)-4-pyrimidinyl | |
| 313 | 2-iso-Propyl-6-methyl-4-pyrimidinyl | |
| 314 | 2-cyclo-Propyl-6-methyl-4-pyrimidinyl | |
| 315 | 2-Methyl-6-methoxymethyl-4-pyrimidinyl | |
| 316 | 2-iso-Propyl-6-methoxymethyl-4-pyrimidinyl | |
| 317 | 2-Phenyl-4-pyrimidinyl | |
| 318 | 3,5-Dimethyl-4-pyrimidinyl | |
| 319 | 2-Methyl-6-trifluoromethyl-4-pyrimidinyl | |
| 320 | 2-n-Propyl-6-trifluoromethyl-4-pyrimidinyl | |
| 321 | 2-iso-Propyl-6-trifluoromethyl-4-pyrimidinyl | |
| 322 | 2-Methyl-5-chloro-6-trifluoromethyl-4-pyrimidinyl | |
| 323 | 2-n-Propyl-5-chloro-6-trifluoromethyl-4-pyrimidinyl | |
| 324 | 2-iso-Propyl-5-chloro-6-trifluoromethyl-4-pyrimidinyl | |
| 325 | 2-tert.-Butyl-5-chloro-6-trifluoromethyl-4-pyrimidinyl | |

Tabelle 22

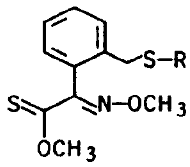

| Nr. | R | Fp(°C)/IR($cm^{-1}$) |
|---|---|---|
| 1 | Methyl | |
| 2 | Ethyl | |
| 3 | n-Propyl | |
| 4 | iso-Propyl | |
| 5 | n-Butyl | |
| 6 | s-Butyl | |
| 7 | iso-Butyl | |
| 8 | tert.-Butyl | |
| 9 | Pentyl | |
| 10 | Hexyl | |
| 11 | Phenyl | |
| 12 | 2-F-Phenyl | |
| 13 | 3-F-Phenyl | |
| 14 | 4-F-Phenyl | |
| 15 | 2-Cl-Phenyl | 1661, 1451, 1443, 1139, 1115, 1043, 1035, 1024, 846, 749 |
| 16 | 3-Cl-Phenyl | |
| 17 | 4-Cl-Phenyl | |
| 18 | 2-Br-Phenyl | |
| 19 | 3-Br-Phenyl | |
| 20 | 4-Br-Phenyl | |
| 21 | 2-J-Phenyl | |
| 22 | 3-J-Phenyl | |
| 23 | 4-J-Phenyl | |
| 24 | 2-CN-Phenyl | |
| 25 | 3-CN-Phenyl | |
| 26 | 4-CN-Phenyl | |
| 27 | 2,3-$F_2$-phenyl | |
| 28 | 2,4-$F_2$-phenyl | |
| 29 | 2,3-$Cl_2$-phenyl | |
| 30 | 2,4-$Cl_2$-phenyl | |
| 31 | 2,5-$Cl_2$-phenyl | |
| 32 | 2,6-$Cl_2$-phenyl | |
| 33 | 3,4-$Cl_2$-phenyl | |
| 34 | 3,5-$Cl_2$-phenyl | |

| Nr. | R | Fp(°C)/IR($cm^{-1}$) |
|---|---|---|
| 35 | 2,4-$Br_2$-phenyl | |
| 36 | 2,5-$Br_2$-phenyl | |
| 37 | 2,6-$Br_2$-phenyl | |
| 38 | 2,4-$J_2$-phenyl | |
| 39 | 2-Cl, 3-F-phenyl | |
| 40 | 2-Cl, 4-F-phenyl | |
| 41 | 2-Cl, 5-F-phenyl | |
| 42 | 2-Cl, 6-F-phenyl | |
| 43 | 2-Cl, 3-Br-phenyl | |
| 44 | 2-Cl, 4-Br-phenyl | |
| 45 | 2-Cl, 5-Br-phenyl | |
| 46 | 2-Cl, 6-Br-phenyl | |
| 47 | 2-Br, 3-Cl-phenyl | |
| 48 | 2-Br, 4-Cl-phenyl | |
| 49 | 2-Br, 3-F-phenyl | |
| 50 | 2-Br, 4-F-phenyl | |
| 51 | 2-Br, 5-F-phenyl | |
| 52 | 2-Br, 6-F-phenyl | |
| 53 | 2-F, 3-Cl-phenyl | |
| 54 | 2-F, 4-Cl-phenyl | |
| 55 | 2-F, 5-Cl-phenyl | |
| 56 | 3-Cl, 4-F-phenyl | |
| 57 | 3-Cl, 5-F-phenyl | |
| 58 | 3-Cl, 4-Br-phenyl | |
| 59 | 3-Cl, 5-Br-phenyl | |
| 60 | 3-F, 4-Cl-phenyl | |
| 61 | 3-F, 4-Br-phenyl | |
| 62 | 3-Br, 4-Cl-phenyl | |
| 63 | 3-Br, 4-F-phenyl | |
| 64 | 2,4,6-$F_3$-phenyl | |
| 65 | 2,3,4,-$Cl_3$-phenyl | |
| 66 | 2,3,5-$Cl_3$-phenyl | |
| 67 | 2,3,6-$Cl_3$-phenyl | |
| 68 | 2,4,5-$Cl_3$-phenyl | |
| 69 | 2,4,6-$Cl_3$-phenyl | |
| 70 | 3,4,5-$Cl_3$-phenyl | |
| 71 | 2,4,6-$Br_3$-phenyl | |
| 72 | 2,6-$Cl_2$-4-Br-phenyl | |
| 73 | 2,3,4,6-$Cl_4$-phenyl | |
| 74 | 2,3,5,6-$Cl_4$-phenyl | |
| 75 | $F_5$-phenyl | |
| 76 | $Cl_5$-phenyl | |

| Nr. | R | Fp(°C)/IR($cm^{-1}$) |
|---|---|---|
| 77 | $Br_5$-phenyl | |
| 78 | 2-$CH_3$-phenyl | |
| 79 | 3-$CH_3$-phenyl | |
| 80 | 4-$CH_3$-phenyl | |
| 81 | 2-$C_2H_5$-phenyl | |
| 82 | 3-$C_2H_5$-phenyl | |
| 83 | 4-$C_2H_5$-phenyl | |
| 84 | 2-n-$C_3H_7$-phenyl | |
| 85 | 3-n-$C_3H_7$-phenyl | |
| 86 | 4-n-$C_3H_7$-phenyl | |
| 87 | 2-i-$C_3H_7$-phenyl | |
| 88 | 3-i-$C_3H_7$-phenyl | |
| 89 | 4-i-$C_3H_7$-phenyl | |
| 90 | 2-s-$C_4H_9$-phenyl | |
| 91 | 3-s-$C_4H_9$-phenyl | |
| 92 | 4-s-$C_4H_9$-phenyl | |
| 93 | 2-t-$C_4H_9$-phenyl | |
| 94 | 3-t-$C_4H_9$-phenyl | |
| 95 | 4-t-$C_4H_9$-phenyl | |
| 96 | 2,3-$(CH_3)_2$-phenyl | |
| 97 | 2,4-$(CH_3)_2$-phenyl | |
| 98 | 2,5-$(CH_3)_2$-phenyl | |
| 99 | 2,6-$(CH_3)_2$-phenyl | |
| 100 | 3,4-$(CH_3)_2$-phenyl | |
| 101 | 3,5-$(CH_3)_2$-phenyl | |
| 102 | 2,3,4-$(CH_3)_3$-phenyl | |
| 103 | 2,3,5-$(CH_3)_3$-phenyl | |
| 104 | 2,3,6-$(CH_3)_3$-phenyl | |
| 105 | 2,4,5-$(CH_3)_3$-phenyl | |
| 106 | 2,4,6-$(CH_3)_3$-phenyl | |
| 107 | 3,4,5-$(CH_3)_3$-phenyl | |
| 108 | 2,3,4,6-$(CH_3)_4$-phenyl | |
| 109 | 2,3,5,6-$(CH_3)_4$-phenyl | |
| 110 | $(CH_3)_5$-phenyl | |
| 111 | 2,4-$(C_2H_5)_2$-phenyl | |
| 112 | 2,6-$(C_2H_5)_2$-phenyl | |
| 113 | 3,5-$(C_2H_5)_2$-phenyl | |
| 114 | 2,4,6-$(C_2H_5)_3$-phenyl | |
| 115 | 2,4-$(i\text{-}C_3H_7)_2$-phenyl | |
| 116 | 2,6-$(i\text{-}C_3H_7)_2$-phenyl | |
| 117 | 3,5-$(i\text{-}C_3H_7)_2$-phenyl | |
| 118 | 2,4,6-$(i\text{-}C_3H_7)_3$-phenyl | |

| Nr. | R | Fp(°C)/IR($cm^{-1}$) |
|---|---|---|
| 119 | 2,3-$(t-C_4H_9)_2$-phenyl | |
| 120 | 2,4-$(t-C_4H_9)_2$-phenyl | |
| 121 | 2,5-$(t-C_4H_9)_2$-phenyl | |
| 122 | 2,6-$(t-C_4H_9)_2$-phenyl | |
| 123 | 3,5-$(t-C_4H_9)_2$-phenyl | |
| 124 | 2,4,6-$(t-C_4H_9)_3$-phenyl | |
| 125 | 2-t-$C_4H_9$, 4-$CH_3$-phenyl | |
| 126 | 2-t-$C_4H_9$, 5-$CH_3$-phenyl | |
| 127 | 2,6-$(t-C_4H_9)_2$, 4-$CH_3$-phenyl | |
| 128 | 2-$CH_3$, 4-t-$C_4H_9$-phenyl | |
| 129 | 2-$CH_3$, 6-t-$C_4H_9$-phenyl | |
| 130 | 2-$CH_3$, 4-i-$C_3H_7$-phenyl | |
| 131 | 2-$CH_3$, 5-i-$C_3H_7$-phenyl | |
| 132 | 3-$CH_3$, 4-i-$C_3H_7$-phenyl | |
| 133 | 2-i-$C_3H_7$, 5-$CH_3$-phenyl | |
| 134 | 2,4-$(t-C_4H_9)_2$-6-i-$C_3H_7$-phenyl | |
| 135 | 2-cyclo-$C_6H_{11}$-phenyl | |
| 136 | 3-cyclo-$C_6H_{11}$-phenyl | |
| 137 | 4-cyclo-$C_6H_{11}$-phenyl | |
| 138 | 2,4-$(cyclo-C_6H_{11})_2$-6-$CH_3$-phenyl | |
| 139 | 2-$CH_3$, 4-cyclo-$C_6H_{11}$-phenyl | |
| 140 | 2-$CH_2C_6H_5$-phenyl | |
| 141 | 3-$CH_2C_6H_5$-phenyl | |
| 142 | 4-$CH_2C_6H_5$-phenyl | |
| 143 | 2-$CH_2C_6H_5$, 4-$CH_3$-phenyl | |
| 144 | 2-$CH_3$, 4-$CH_2C_6H_5$-phenyl | |
| 145 | 2-$C_6H_5$-phenyl | |
| 146 | 3-$C_6H_5$-phenyl | |
| 147 | 4-$C_6H_5$-phenyl | |
| 148 | 4-(2-i-$C_3H_7$-$C_6H_4$)-phenyl | |
| 149 | 4-$C_6H_5$, 2,6-$(CH_3)_2$-phenyl | |
| 150 | 2-Cl, 4-$C_6H_5$-phenyl | |
| 151 | 2-Br, 4-$C_6H_5$-phenyl | |
| 152 | 2-$C_6H_5$, 4-Cl-phenyl | |
| 153 | 2-$C_6H_5$, 4-Br-phenyl | |
| 154 | 2-$CH_2C_6H_5$, 4-Cl-phenyl | |
| 155 | 2-$CH_2C_6H_5$, 4-Br-phenyl | |
| 156 | 2-Cl, 4-$CH_2C_6H_5$-phenyl | |
| 157 | 2-Br, 4-$CH_2C_6H_5$-phenyl | |
| 158 | 2-cyclo-$C_6H_{11}$, 4-Cl-phenyl | |
| 159 | 2-cyclo-$C_6H_{11}$, 4-Br-phenyl | |
| 160 | 2-Cl, 4-cyclo-$C_6H_{11}$-phenyl | |

| Nr. | R | Fp(°C)/IR($cm^{-1}$) |
|---|---|---|
| 161 | 2-Br, 4-cyclo-$C_6H_{11}$-phenyl | |
| 162 | 3-$OCH_3$-phenyl | |
| 163 | 2,4-$(OCH_3)_2$-phenyl | |
| 164 | 2-$CF_3$-phenyl | |
| 165 | 3-$CF_3$-phenyl | |
| 166 | 4-$CF_3$-phenyl | |
| 167 | 2-$NO_2$-phenyl | |
| 168 | 3-$NO_2$-phenyl | |
| 169 | 4-$NO_2$-phenyl | |
| 170 | 2-CN-phenyl | |
| 171 | 3-CN-phenyl | |
| 172 | 4-CN-phenyl | |
| 173 | 2-$CH_3$, 3-Cl-phenyl | |
| 174 | 2-$CH_3$, 4-Cl-phenyl | |
| 175 | 2-$CH_3$, 5-Cl-phenyl | |
| 176 | 2-$CH_3$, 6-Cl-phenyl | |
| 177 | 2-$CH_3$, 3-F-phenyl | |
| 178 | 2-$CH_3$, 4-F-phenyl | |
| 179 | 2-$CH_3$, 5-F-phenyl | |
| 180 | 2-$CH_3$, 6-F-phenyl | |
| 181 | 2-$CH_3$, 3-Br-phenyl | |
| 182 | 2-$CH_3$, 4-Br-phenyl | |
| 183 | 2-$CH_3$, 5-Br-phenyl | |
| 184 | 2-$CH_3$, 6-Br-phenyl | |
| 185 | 2-Cl, 3-$CH_3$-phenyl | |
| 186 | 2-Cl, 4-$CH_3$-phenyl | |
| 187 | 2-Cl, 5-$CH_3$-phenyl | |
| 188 | 2-Cl, 3-i-$C_3H_7$ | |
| 189 | 2-F, 3-$CH_3$-phenyl | |
| 190 | 2-F, 4-$CH_3$-phenyl | |
| 191 | 2-F, 5-$CH_3$-phenyl | |
| 192 | 2-Br, 3-$CH_3$-phenyl | |
| 193 | 2-Br, 4-$CH_3$-phenyl | |
| 194 | 3-$CH_3$, 4-Cl-phenyl | |
| 195 | 3-$CH_3$, 5-Cl-phenyl | |
| 196 | 2-Br, 5-$CH_3$-phenyl | |
| 197 | 3-$CH_3$, 4-F-phenyl | |
| 198 | 3-$CH_3$, 5-F-phenyl | |
| 199 | 3-$CH_3$, 4-Br-phenyl | |
| 200 | 3-$CH_3$, 5-Br-phenyl | |
| 201 | 3-F, 4-$CH_3$-phenyl | |
| 202 | 3-Cl, 4-$CH_3$-phenyl | |

| Nr. | R | Fp(°C)/IR($cm^{-1}$) |
|---|---|---|
| 203 | 3-Br, 4-$CH_3$-phenyl | |
| 204 | 2-Cl, 4,5-$(CH_3)_2$-phenyl | |
| 205 | 2-Br, 4,5-$(CH_3)_2$-phenyl | |
| 206 | 2-Cl, 3,5-$(CH_3)_2$-phenyl | |
| 207 | 2-Br, 3,5-$(CH_3)_2$-phenyl | |
| 208 | 2,6-$Cl_2$, 4-$CH_3$-phenyl | |
| 209 | 2,6-$F_2$, 4-$CH_3$-phenyl | |
| 210 | 2,6-$Br_2$, 4-$CH_3$-phenyl | |
| 211 | 2,4-$Cl_2$, 6-$CH_3$-phenyl | |
| 212 | 2,4-$F_2$, 6-$CH_3$-phenyl | |
| 213 | 2,4-$Br_2$, 6-$CH_3$-phenyl | |
| 214 | 2,6-$(CH_3)_2$, 4-F-phenyl | |
| 215 | 2,6-$(CH_3)_2$, 4-Cl-phenyl | |
| 216 | 2,6-$(CH_3)_2$, 4-Br-phenyl | |
| 217 | 3,5-$(CH_3)_2$, 4-F-phenyl | |
| 218 | 3,5-$(CH_3)_2$, 4-Cl-phenyl | |
| 219 | 3,5-$(CH_3)_2$, 4-Br-phenyl | |
| 220 | 2,3,6-$(CH_3)_3$, 4-F-phenyl | |
| 221 | 2,3,6-$(CH_3)_3$, 4-Cl-phenyl | |
| 222 | 2,3,6-$(CH_3)_3$, 4-Br-phenyl | |
| 223 | 2,4-$(CH_3)_2$, 6-F-phenyl | |
| 224 | 2,4-$(CH_3)_2$, 6-Cl-phenyl | |
| 225 | 2,4-$(CH_3)_2$, 6-Br-phenyl | |
| 226 | 2-i-$C_3H_7$, 4-Cl, 5-$CH_3$-phenyl | |
| 227 | 2-Cl, 4-$NO_2$-phenyl | |
| 228 | 2-$NO_2$, 4-Cl-phenyl | |
| 229 | 2-$OCH_3$, 5-$NO_2$-phenyl | |
| 230 | 2,4-$Cl_2$, 5-$NO_2$-phenyl | |
| 231 | 2,4-$Cl_2$, 6-$NO_2$-phenyl | |
| 232 | 2,6-$Cl_2$, 4-$NO_2$-phenyl | |
| 233 | 2,6-$Br_2$, 4-$NO_2$-phenyl | |
| 234 | 2,6-$J_2$, 4-$NO_2$-phenyl | |
| 235 | 2-$C_6H_5O$-phenyl | |
| 236 | 3-$C_6H_5O$-phenyl | |
| 237 | 4-$C_6H_5O$-phenyl | |
| 238 | 3-t-$C_4H_9O$-phenyl | |
| 239 | 4-t-$C_4H_9O$-phenyl | |
| 240 | 1-Naphthyl | |
| 241 | 2-Naphthyl | |
| 242 | 2-Pyridyl | |
| 243 | 6-Methyl-2-pyridyl | 89-94°C |
| 244 | 6-Ethyl-2-pyridyl | |

| Nr. | R | Fp($^{o}C$)/IR($cm^{-1}$) |
|---|---|---|
| 245 | 6-n-Propyl-2-pyridyl | |
| 246 | 6-iso-Propyl-2-pyridyl | |
| 247 | 6-n-Butyl-2-pyridyl | |
| 248 | 6-tert.-Butyl-2-pyridyl | |
| 249 | 6-n-Pentyl-2-pyridyl | |
| 250 | 6-n-Hexyl-2-pyridyl | |
| 251 | 6-Phenyl-2-pyridyl | |
| 252 | 6-Benzyl-2-pyridyl | |
| 253 | 6-Trifluormethyl-2-pyridyl | |
| 254 | 6-Methoxy-2-pyridyl | |
| 255 | 6-Chloro-2-pyridyl | |
| 256 | 3,6-Dimethyl-2-pyridyl | |
| 257 | 3,6-Diethyl-2-pyridyl | |
| 258 | 4,6-Dimethyl-2-pyridyl | |
| 259 | 5,6-Dimethyl-2-pyridyl | |
| 260 | 4-Phenyl-6-methyl-2-pyridyl | |
| 261 | 4,6-Diphenyl-2-pyridyl | |
| 262 | 3,4-Dichloro-6-methyl-2-pyridyl | |
| 263 | 3,4,5-Trichloro-6-phenyl-2-pyridyl | |
| 264 | 4-Trifluoromethyl-6-methyl-2-pyridyl | |
| 265 | 3-Cyano-6-methyl-2-pyridyl | |
| 266 | 3-Cyano-6-ethyl-2-pyridyl | |
| 267 | 3-Cyano-6-n-propyl-2-pyridyl | |
| 268 | 3-Cyano-6-iso-propyl-2-pyridyl | |
| 269 | 3-Cyano-6-cyclo-propyl-2-pyridyl | |
| 270 | 3-Cyano-6-n-butyl-2-pyridyl | |
| 271 | 3-Cyano-6-tert.-butyl-2-pyridyl | |
| 272 | 3-Cyano-6-cyclo-hexyl-2-pyridyl | |
| 273 | 3-Cyano-6-phenyl-2-pyridyl | |
| 274 | 3-Cyano-4,6-dimethyl-2-pyridyl | |
| 275 | 3,5,6-Trichloro-2-pyridyl | |
| 276 | 5-Trifluoromethyl-2-pyridyl | |
| 277 | 3-Chloro-5-trifluoromethyl-2-pyridyl | |
| 278 | 2-Chinolyl | |
| 279 | 3-Methyl-2-chinolyl | |
| 280 | 4-Methyl-2-chinolyl | |
| 281 | 4-Ethyl-2-chinolyl | |
| 282 | 4-Phenyl-2-chinolyl | |
| 283 | 6-Methyl-2-chinolyl | |
| 284 | 6-Chloro-2-chinolyl | |
| 285 | 8-Methyl-2-chinolyl | |
| 286 | 8-Chloro-2-chinolyl | |

| Nr. | R | Fp($^{o}$C)/IR(cm$^{-1}$) |
|---|---|---|
| 287 | 3,4-Dimethyl-2-chinolyl | |
| 288 | 4-Methyl-8-methoxy-2-chinolyl | |
| 289 | 4-Phenyl-8-ethoxy-2-chinolyl | |
| 290 | 4-Methyl-8-chloro-2-chinolyl | |
| 291 | 4-Methyl-8-fluoro-2-chinolyl | |
| 292 | 4-Chinolyl | |
| 293 | 2-Methyl-4-chinolyl | |
| 294 | 2-Trifluoromethyl-4-chinolyl | |
| 295 | 2-iso-Propyl-4-chinolyl | |
| 296 | 2-n-Pentyl-4-chinolyl | |
| 297 | 2-Phenyl-4-chinolyl | |
| 298 | 2,6-Dimethyl-4-chinolyl | |
| 299 | 2-Methyl-6-chloro-4-chinolyl | |
| 300 | 2-Methyl-6-fluoro-4-chinolyl | |
| 301 | 8-Chinolyl | |
| 302 | 2-Methyl-8-chinolyl | |
| 303 | 5,7-Dichloro-8-chinolyl | |
| 304 | 4,6-Dimethyl-2-pyrimidinyl | |
| 305 | 4-Trifluormethyl-2-pyrimidinyl | |
| 306 | 4,5,6-Trimethyl-2-pyrimidinyl | |
| 307 | 4-Benzyl-6-methyl-2-pyrimidinyl | |
| 308 | 4-Methyl-6-phenyl-2-pyrimidinyl | |
| 309 | 4,6-Dimethyl-5-chloro-2-pyrimidinyl | |
| 310 | 2,6-Dimethyl-4-pyrimidinyl | |
| 311 | 2,6-Bis-(trifluormethyl)-4-pyrimidinyl | |
| 312 | 2-iso-Propyl-6-methyl-4-pyrimidinyl | |
| 313 | 2-cyclo-Propyl-6-methyl-4-pyrimidinyl | |
| 314 | 2-Methyl-6-methoxymethyl-4-pyrimidinyl | |
| 315 | 2-iso-Propyl-6-methoxymethyl-4-pyrimidinyl | |
| 316 | 2-Phenyl-4-pyrimidinyl | |
| 317 | 3,5-Dimethyl-4-pyrimidinyl | |
| 318 | 2-Methyl-6-trifluoromethyl-4-pyrimidinyl | |
| 319 | 2-n-Propyl-6-trifluoromethyl-4-pyrimidinyl | |
| 320 | 2-iso-Propyl-6-trifluoromethyl-4-pyrimidinyl | |
| 321 | 2-Methyl-5-chloro-6-trifluoromethyl-4-pyrimidinyl | |
| 322 | 2-n-Propyl-5-chloro-6-trifluoromethyl-4-pyrimidinyl | |
| 323 | 2-iso-Propyl-5-chloro-6-trifluoromethyl-4-pyrimidinyl | |
| 324 | 2-tert.-Butyl-5-chloro-6-trifluoromethyl-4-pyrimidinyl | |
| 325 | 6-EtO-benzothiazol-2-yl | |
| 326 | Benzoxazol-2-yl | |
| 327 | 1-Me-imidazol-2-yl | |
| 328 | 4-But-1H-imidazol-2-yl | |

| Nr. | R | Fp(°C)/IR($cm^{-1}$) |
|---|---|---|
| 329 | 2-Thiazolin-2-yl | |
| 330 | 5-$CF_3$-benzimidazol-2-yl | |
| 331 | 1-Ph-tetrazol-5-yl | |
| 332 | 5-$CF_3$-benzothiazol-2-yl | |
| 333 | 4,4-$Me_2$-5-methylene-2-thiazolin-2-yl | |
| 334 | 6-Cl-4-Me-benzothiazol-2-yl | |
| 335 | 5-Me-benzothiazol-2-yl | |
| 336 | 4-Cl-benzothiazol-2-yl | |
| 337 | 1-(3-$NO_2$-Ph)tetrazol-5-yl | |
| 338 | 2-thienyl | |
| 339 | 5-Me-benzoxazol-2-yl | |
| 340 | 7-Cl-benzothiazol-2-yl | |
| 341 | 5,6-$Cl_3$-1H-benzimidazol-2-yl | |
| 342 | 5-Cl-benzoxazol-2-yl | |
| 343 | 6-Cl-benzothiazol-2-yl | |
| 344 | 2-Ph-thiazol-2-yl | |
| 345 | 3-CN-4,6-$Me_2$-2-pyridyl | |
| 346 | 1-Ph-1,2,4-triazol-3-yl | |
| 347 | 1-Pri-benzimidazol-2-yl | |
| 348 | 5-Br-benzothiazol-2-yl | |
| 349 | 5-Br-1H-benzimidazol-2-yl | |
| 350 | 7-Cl-4-MeO-benzothiazol-2-yl | |
| 351 | 1H-benzimidazol-2-yl | |
| 352 | 5-Cl-benzothiazol-2-yl | |
| 353 | 5-$NO_2$-benzoxazol-2-yl | |
| 354 | 5-t-Bu-benzoxazol-2-yl | |
| 355 | 4,6,7-$Cl_3$-benzothiozol-2-yl | |
| 356 | 5-Ph-thiazol-2-yl | |
| 357 | 5,7-$Me_2$-benzoxazol-2-yl | |
| 358 | 6-Me-benzoxazol-2-yl | |
| 359 | 1,2,4-triazin-3-yl | |
| 360 | 6-Pr-benzothiazol-2-yl | |
| 361 | 6-PhO-benzothiazol-2-yl | |
| 362 | 4-(4-Cl-Ph)-thiazol-2-yl | |
| 363 | 4-(4-Me-Ph)-thiazol-2-yl | |
| 364 | 5-Me-4-Ph-thiazol-2-yl | |
| 365 | 5-Cl-1-H-benzimidazol-2-yl | |
| 366 | 5-Ph-1,2,4-triazol-3-yl | |
| 367 | 3-Ph-1,2,4-thiadiazol-5-yl | |

Tabelle 23

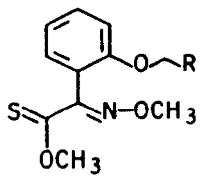

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |
| 34 | 2-OMe-Phenyl | |

| Nr. | R | Fp($^{o}C$)/IR($cm^{-1}$) |
|---|---|---|
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 24

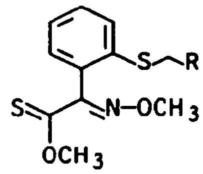

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp(°C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 25

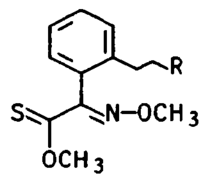

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp(°C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 26

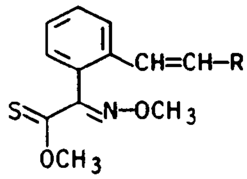

| Nr. | R | Fp(°C)/IR($cm^{-1}$) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_3$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp(°C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Tabelle 27

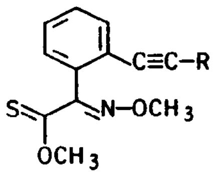

| Nr. | R | Fp(°C)/IR(cm⁻¹) |
|---|---|---|
| 1 | H | |
| 2 | Methyl | |
| 3 | Ethyl | |
| 4 | n-Propyl | |
| 5 | i-Propyl | |
| 6 | n-Butyl | |
| 7 | i-Butyl | |
| 8 | s-Butyl | |
| 9 | t-Butyl | |
| 10 | Pentyl | |
| 11 | Hexyl | |
| 12 | Phenyl | |
| 13 | 1-Naphthyl | |
| 14 | 2-Naphthyl | |
| 15 | 3-Phenanthrenyl | |
| 16 | 2-Fluor-phenyl | |
| 17 | 3-Fluor-phenyl | |
| 18 | 4-Fluor-phenyl | |
| 19 | 2-Chlor-phenyl | |
| 20 | 3-Chlor-phenyl | |
| 21 | 4-Chlor-phenyl | |
| 22 | 2-Brom-phenyl | |
| 23 | 3-Brom-phenyl | |
| 24 | 4-Brom-phenyl | |
| 25 | 2-Jod-phenyl | |
| 26 | 3-Jod-phenyl | |
| 27 | 4-Jod-phenyl | |
| 28 | 2-$NO_2$-phenyl | |
| 29 | 3-$NO_2$-phenyl | |
| 30 | 4-$NO_2$-phenyl | |
| 31 | 2-$CH_3$-phenyl | |
| 32 | 3-$CH_3$-phenyl | |
| 33 | 4-$CH_3$-phenyl | |

| Nr. | R | Fp(°C)/IR($cm^{-1}$) |
|---|---|---|
| 34 | 2-OMe-Phenyl | |
| 35 | 3-OMe-Phenyl | |
| 36 | 4-OMe-Phenyl | |
| 37 | 2-$CF_3$-Phenyl | |
| 38 | 3-$CF_3$-Phenyl | |
| 39 | 4-$CF_3$-Phenyl | |
| 40 | 2-Ph-Phenyl | |
| 41 | 3-Ph-Phenyl | |
| 42 | 4-Ph-Phenyl | |
| 43 | 2-PhO-Phenyl | |
| 44 | 3-PhO-Phenyl | |
| 45 | 4-PhO-Phenyl | |
| 46 | 2-Pyridyl | |
| 47 | 2-Pyrimidyl | |
| 48 | 2-Chinalinyl | |
| 49 | 2-Pyrryl | |
| 50 | 2-Thienyl | |
| 51 | 2-Furyl | |
| 52 | 2-Imidazolyl | |

Die neuen Thiolcarbonsäureester I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Besonders interessant sind sie für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse, wie Gurken, Bohnen und Kürbisgewächse.

Die neuen Thiolcarbonsäureester sind insbesondere geeignet zur Bekämpfung folgender Schadpilze:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.
Paecilomyces variotii an Holz.

Die Thiolcarbonsäureester werden angewendet, indem man die Pflanzen, Materialien oder Flächen mit den Wirkstoffen besprüht oder bestäubt oder die Samen der Pflanzen mit den Wirkstoffen behandelt. Die Anwendung erfolgt vor oder nach der Infektion durch die Pilze. Die Verbindungen werden in fungizid wirksamen Mengen angewendet.

Die Thiolcarbonsäureester können in die üblichen fungiziden Mittel (Formulierungen) übergeführt werden, d.h. in Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise und mit den üblichen Hilfsstoffen hergestellt, z.B. durch Verstrecken des Wirkstoffs mit Lösungsmitteln und/oder Trägerstoffen, gegebenenfalls unter Verwendung von Emulgiermitteln und Dispergiermitteln. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser (letzteres gegebenenfalls zusammen mit organischen Lösungsmitteln); Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-, Sulfitablaugen und Methylcellulose.

Die Mittel können auch im Holzschutz angewendet werden.

Die fungiziden Mittel enthalten im allgemeinen zwischen 0,1 und 95 Gew.-%, vorzugsweise zwischen 0,5 und 90 Gew.-% Wirkstoff. Die Aufwandmengen liegen je nach Art des gewünschten Effektes zwischen 0,02 und 3 kg Wirkstoff je ha. Bei der Saatgutbehandlung werden Aufwandmengen von 0,01 bis 50 g, vorzugsweise 0,01 bis 10 g Wirkstoff je Kilogramm Saatgut benötigt.

Die Mittel werden, gegebenenfalls nach weiterer Verdünnung, in bekannter Weise angewendet, beispielsweise durch Versprühen, Vernebeln, Verstäuben, Verstreuen, Beizen oder Gießen.

Beispiele für Zubereitungen sind:

I. Man vermischt 90 Gew.-Teile der Verbindung Nr. 15 (Tab. 3) mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 14 (Tab. 4) werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 243 (Tab. 4) werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 15 Tab. 3) werden in einer Mischung gelöst, die aus 25 Gew.-Teilen Cyclohexanol, 65 Gew.-Teilen einer Mineralölfraktion vom Siedepunkt 210 bis 280°C und 80 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 14 (Tab. 4) werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 243 (Tab. 4) werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 15 (Tab. 3) werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 14 (Tab. 4) werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 243 (Tab. 4) werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit

Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Die fungizide Wirkung der Thiolcarbonsäureester I wurde mit der von 2-(Phenyloxymethyl)-phenylglyoxylsäuremethylester-O-methyloxim (A) - bekannt aus EP 253 213 - im Gewächshausversuch verglichen.

Beispiel 1

Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach 24 Stunden wurden die behandelten Pflanzen mit einer Sporensuspension von Botrytis cinerea gespritzt. Nach 8-tägiger Kultur der behandelten Pflanzen im Gewächshaus wurden sie mit einer Sporensuspension von Botrytis cinerea, die $1.7x10^6$ Sporen/ml in einer 2%igen Biomalzlösung enthielt, inoculiert. Anschließend wurden sie in eine Klimakammer mit 22-24°C und hoher Luftfeuchtigkeit gestellt. Nach 5 Tagen konnte das Ausmaß der Pilzentwicklung ausgewertet werden.

Bonitur: Angabe der befallenen Blattflächen in %

| Wirkstoff-Nr. | Befall der Blätter nach Applikation von 0,05%iger wäßriger Wirkstoffaufbereitung Note | ≙ % Befall |
|---|---|---|
| Tabelle 3, Nr. 24 | 1 | 5 |
| Tabelle 4, Nr. 14 | 2 | 15 |
| Tabelle 4, Nr. 243 | 2 | 15 |
| Vergleichsprodukt A | 4-5 | 50 |
| Unbehandelt | 5 | 65 |

Beispiel 2

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% Wirkstoff und 20% Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70% relativer Luftfeuchte aufgestellt. Nach 8 Tagen wurde das Ausmaß der Rostpilzentwicklung auf den Blättern ermittelt.

Bonitur:

0 = kein Pilzbefall, abgestuft bis

5 = Totalbefall

| Wirkstoff-Nr. | Befall der Blätter nach Applikation von 0,025%iger wäßriger Wirkstoffaufbereitung Note | ≙ % Befall |
|---|---|---|
| Tabelle 4, Nr. 14 | 2 | 15 |
| Vergleichsprodukt A | 4-5 | 50 |
| Unbehandelt | 5 | 70 |

## Patentansprüche

1. Thiolcarbonsäureester der allgemeinen Formel I,

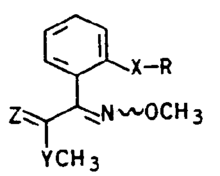

I

in der die Substituenten folgende Bedeutung haben:

X Sauerstoff, Schwefel, Oxymethylen, Methylenoxy, Thiomethylen, Methylenthio, Ethylen, Ethenylen oder Ethinylen,

Y, Z Schwefel oder Sauerstoff, wobei nicht Y mit Z zusammen gleichzeitig Sauerstoff bedeuten,

R $C_1$-$C_6$-Alkyl, ein-, zwei- oder dreikerniges Aryl oder Heteroaryl, wobei Aryl und Heteroaryl folgende Reste $R^1$ tragen können:

$R^1$ Halogen, Cyano, Nitro, $C_1$-$C_6$-Alkyl, $C_3$-$C_6$-Cycloalkyl, $C_1$-$C_6$-Alkoxy, Trifluormethyl, ein- oder zweikerniges Aryloxy oder ein-, zwei- oder dreikerniges Aryl, wobei Aryloxy und Aryl ihrerseits durch die genannten Reste $R^1$ substituiert sein können.

2. Thiolcarbonsäureester der Formel I gemäß Anspruch 1, dadurch gekennzeichnet, daß X Methylenoxy, R Phenyl und $R^1$ 2-Chlor bedeutet.

3. Thiolcarbonsäureester der Formel I gemäß Anspruch 1, dadurch gekennzeichnet, daß X Methylenoxy, R Phenyl und $R^1$ 2-Methyl bedeutet.

4. Thiolcarbonsäureester der Formel I gemäß Anspruch 1, dadurch gekennzeichnet, daß X Methylenthio, R Phenyl und $R^1$ 2-Chlor bedeutet.

5. Thiolcarbonsäureester der Formel I gemäß Anspruch 1, dadurch gekennzeichnet, daß X Methylenthio, R 2-Pyridyl und $R^1$ 6-Methyl bedeutet.

6. Verfahren zur Herstellung der Thiolcarbonsäureester der allgemeinen Formel I,

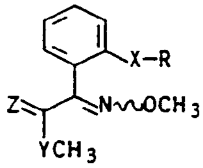

I

in der die Substituenten folgende Bedeutung haben:

X Sauerstoff, Schwefel, Oxymethylen, Methylenoxy, Thiomethylen, Methylenthio, Ethylen, Ethenylen oder Ethinylen,

Y, Z Schwefel oder Sauerstoff, wobei nicht Y mit Z zusammen gleichzeitig Sauerstoff bedeuten,

R $C_1$-$C_6$-Alkyl, ein-, zwei- oder dreikerniges Aryl oder Heteroaryl, wobei Aryl und Heteroaryl folgende Reste $R^1$ tragen können:

$R^1$ Halogen, Cyano, Nitro, $C_1$-$C_6$-Alkyl, $C_3$-$C_6$-Cycloalkyl, $C_1$-$C_6$-Alkoxy, Trifluormethyl, ein- oder zweikerniges Aryloxy oder ein-, zwei- oder dreikerniges Aryl, wobei Aryloxy und Aryl ihrerseits durch die genannten Reste $R^1$ substituiert sein können,

dadurch gekennzeichnet, daß man einen Carbonsäureester der allgemeinen Formel II,

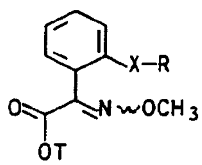

II

in der T den Rest eines Alkohols bedeutet, in an sich bekannter Weise zur Carbonsäure der allgemeinen Formel III,

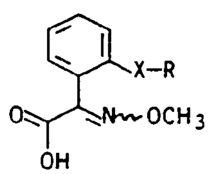

III

verseift und diese dann in ebenfalls bekannter Weise mit Methanthiol oder einem Alkalimethanthiolat zu dem Thiolcarbonsäureester umsetzt oder einen Carbonsäureester der Formel

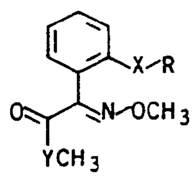

IV

mit $P_4S_{10}$ oder 2,4-Bis(4-methoxyphenyl)-1,3-dithia-2,4-diphosphetan-2,4-disulfid umsetzt.

7. Fungizide Mittel, enthalted eine fungizid wirksame Menge eines Thiolcarbonsäureesters der allgemeinen Formel I,

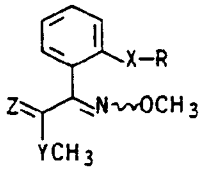

I

in der die Substituenten folgende Bedeutung haben:

X Sauerstoff, Schwefel, Oxymethylen, Methylenoxy, Thiomethylen, Methylenthio, Ethylen, Ethenylen oder Ethinylen,

Y, Z Schwefel oder Sauerstoff, wobei nicht Y mit Z zusammen gleichzeitig Sauerstoff bedeuten,

R $C_1$-$C_6$-Alkyl, ein-, zwei- oder dreikerniges Aryl oder Heteroaryl, wobei Aryl und Heteroaryl folgende Reste $R^1$ tragen können:

$R^1$ Halogen, Cyano, Nitro, $C_1$-$C_6$-Alkyl, $C_3$-$C_6$-Cycloalkyl, $C_1$-$C_6$-Alkoxy, Trifluormethyl, ein- oder zweikerniges Aryloxy oder ein-, zwei- oder dreikerniges Aryl, wobei Aryloxy und Aryl ihrerseits durch die genannten Reste $R^1$ substituiert sein können

und einen üblichen Trägerstoff.

**8.** Verfahren zur Bekämpfung von Schadpilzen, dadurch gekennzeichnet, daß man die Pilze oder die vom Pilzbefall bedrohten Pflanzen, Samen, Materialien oder Flächen mit einer fungizid wirksamen Menge eines Thiolcarbonsäureesters der allgemeinen Formel I,

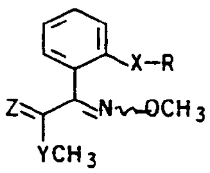

I

in der die Substituenten folgende Bedeutung haben:

X Sauerstoff, Schwefel, Oxymethylen, Methylenoxy, Thiomethylen, Methylenthio, Ethylen, Ethenylen oder Ethinylen,

Y, Z Schwefel oder Sauerstoff, wobei nicht Y mit Z zusammen gleichzeitig Sauerstoff bedeuten,

R $C_1$-$C_6$-Alkyl, ein-, zwei- oder dreikerniges Aryl oder Heteroaryl, wobei Aryl und Heteroaryl folgende Reste $R^1$ tragen können:

$R^1$ Halogen, Cyano, Nitro, $C_1$-$C_6$-Alkyl, $C_3$-$C_6$-Cycloalkyl, $C_1$-$C_6$-Alkoxy, Trifluormethyl, ein- oder zweikerniges Aryloxy oder ein-, zwei- oder dreikerniges Aryl, wobei Aryloxy und Aryl ihrerseits durch die genannten Reste $R^1$ substituiert sein können,

behandelt.

## Claims

**1.** A thiocarboxylic ester of the formula I

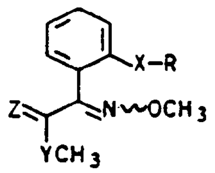

I

where X is oxygen, sulfur, oxymethylene, methyleneoxy, thiomethylene, methylenethio, ethylene, ethenylene or ethynylene,

Y and Z are each sulfur or oxygen, Y and Z not simultaneously being oxygen,

R is $C_1$-$C_6$-alkyl, mononuclear, dinuclear or trinuclear aryl or hetaryl, where aryl and hetaryl may carry the following radicals $R^1$:

halogen, cyano, nitro, $C_1$-$C_6$-alkyl, $C_3$-$C_6$-cycloalkyl, $C_1$-$C_6$-alkoxy, trifluoromethyl, mononuclear or dinuclear aryloxy or mononuclear, dinuclear or trinuclear aryl, where aryloxy and aryl may in turn be substituted by the stated radicals $R^1$.

**2.** A thiocarboxylic ester of the formula I as claimed in claim 1, wherein X is methyleneoxy, R is phenyl and $R^1$ is 2-chloro.

**3.** A thiocarboxylic ester of the formula I as claimed in claim 1, wherein X is methyleneoxy, R is phenyl and $R^1$ is 2-methyl.

4. A thiocarboxylic ester of the formula I as claimed in claim 1, wherein X is methylenethio, R is phenyl and $R^1$ is 2-chloro.

5. A thiocarboxylic ester of the formula I as claimed in claim 1, wherein X is methylenethio, R is 2-pyridyl and $R^1$ is 6-methyl.

6. A process for the preparation of a thiocarboxylic ester of the formula I

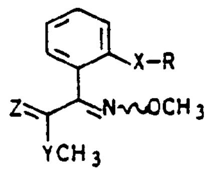

I

where
X is oxygen, sulfur, oxymethylene, methyleneoxy, thiomethylene, methylenethio, ethylene, ethenylene or ethynylene,
Y and Z are each sulfur or oxygen, Y and Z not simultaneously being oxygen,
R is $C_1$-$C_6$-alkyl, mononuclear, dinuclear or trinuclear aryl or hetaryl, where aryl and hetaryl may carry the following radicals $R^1$:
halogen, cyano, nitro, $C_1$-$C_6$-alkyl, $C_3$-$C_6$-cycloalkyl, $C_1$-$C_6$-alkoxy, trifluoromethyl, mononuclear or dinuclear aryloxy or mononuclear, dinuclear or trinuclear aryl, where aryloxy and aryl may in turn be substituted by the stated radicals $R^1$,
wherein a carboxylic ester of the formula II

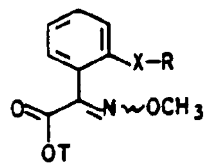

II

where T is a radical of an alcohol, is hydrolyzed in a conventional manner to a carboxylic acid of the formula III

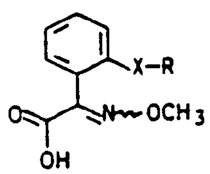

III

and the latter is then reacted, likewise in a known manner, with methanethiol or an alkali metal methanethiolate to give a thiocarboxylic ester
or
a carboxylic ester of the formula

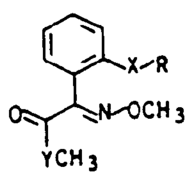

IV

is reacted with $P_4S_{10}$ or 2,4-bis(4-methoxyphenyl)-1,3-dithia-2,4-diphosphetane 2,4-disulfide.

7. A fungicide containing a fungicidally effective amount of a thiocarboxylic ester of the formula I

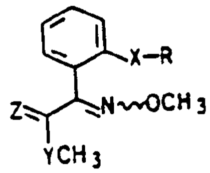

I

where
X is oxygen, sulfur, oxymethylene, methyleneoxy, thiomethylene, methylenethio, ethylene, ethenylene or ethynylene,
Y and Z are each sulfur or oxygen, Y and Z not simultaneously being oxygen,
R is $C_1$-$C_6$-alkyl, mononuclear, dinuclear or trinuclear aryl or hetaryl, where aryl and hetaryl may carry the following radicals $R^1$:
halogen, cyano, nitro, $C_1$-$C_6$-alkyl, $C_3$-$C_6$-cycloalkyl, $C_1$-$C_6$-alkoxy, trifluoromethyl, mononuclear or dinuclear aryloxy or mononuclear, dinuclear or trinuclear aryl, where aryloxy and aryl may in turn be substituted by the stated radicals $R^1$,
and a conventional carrier.

8. A method for controlling harmful fungi, wherein the fungi or the plants, seed, materials or areas threatened by fungal attack are treated with a fungicidally effective amount of a thiocarboxylic ester of the formula I

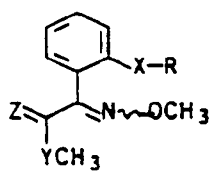

I

where
X is oxygen, sulfur, oxymethylene, methyleneoxy, thiomethylene, methylenethio, ethylene, ethenylene or ethynylene,
Y and Z are each sulfur or oxygen, Y and Z not simultaneously being oxygen,
R is $C_1$-$C_6$-alkyl, mononuclear, dinuclear or trinuclear aryl or hetaryl, where aryl and hetaryl may carry the following radicals $R^1$:
halogen, cyano, nitro, $C_1$-$C_6$-alkyl, $C_3$-$C_6$-cycloalkyl, $C_1$-$C_6$-alkoxy, trifluoromethyl, mononuclear or dinuclear aryloxy or mononuclear, dinuclear or trinuclear aryl, where aryloxy and aryl may in turn be substituted by the stated radicals $R^1$.

## Revendications

1. Esters d'acide thiolcarboxylique de formules générale I,

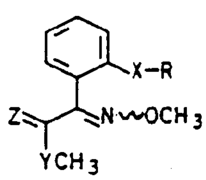

I

dans laquelle les substituants ont les significations suivantes :

| | |
|---|---|
| X | oxygène, soufre, oxyméthylène, méthylénoxy, thiométhylène, méthylènethio, éthylène, éthénylène ou éthylnylène, |
| Y, Z | soufre ou oxygène, Y et Z ne pouvant pas représenter simultanément oxygène. |

R alkyle en C1-C6, aryle ou hétéro-aryle à un, deux ou trois noyaux, aryle et hétéro-aryle pouvant porter les restes $R^1$ suivants :

$R^1$ halogène, cyano, nitro, alkyle en C1-C6, cycloalkyle en C3-C6, alcoxy en C1-C6, trifluorométhyle, aryloxy à un ou deux noyaux ou aryle à un, deux ou trois noyaux, aryloxy et aryle pouvant à leur tour être substitués par les restes $R^1$ précités.

2. Esters d'acide thiolcarboxylique de formule I, selon la revendication 1, caractérisés par le fait que X représente méthylénoxy, R phényle et $R^1$ 2-chlore.

3. Esters d'acide thiolcarboxylique de formule I, selon la revendication 1, caractérisés par le fait que X représente méthylénoxy, R phényle et $R^1$ 2-méthyle.

4. Esters d'acide thiocarboxylique de formule I, selon la revendication 1, caractérisés par le fait que X représente méthylènethio, R phényle et $R^1$ 2-chlore.

5. Esters d'acide thiolcarboxylique de formule I, selon la revendication 1, caractérisés par le fait que X représente méthylènethio, R 2-pyridyle et $R^1$ 6-méthyle.

6. Procédé de préparation des esters d'acide thiolcarboxylique de formule générale I,

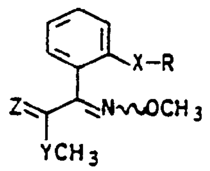

I

dans laquelle les substituants ont les significations suivantes :

X oxygène, soufre, oxyméthylène, méthylénoxy, thiométhylène, méthylènethio, éthylène, éthénylène ou éthylnylène,

Y, Z soufre ou oxygène, Y et Z ne pouvant pas représenter simultanément oxygène.

R alkyle en C1-C6, aryle ou hétéro-aryle à un, deux ou trois noyaux, aryle et hétéro-aryle pouvant porter les restes $R^1$ suivants :

$R^1$ halogène, cyano, nitro, alkyle en C1-C6, cycloalkyle en C3-C6, alcoxy en C1-C6, trifluorométhyle, aryloxy à un ou deux noyaux ou aryle à un, deux ou trois noyaux, aryloxy et aryle pouvant à leur tour être substitués par les restes $R^1$ précités, caractérisé par le fait que l'on saponifie, de manière, en soi, connue, un ester d'acide carboxylique de formule générale II,

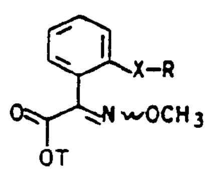

II

dans laquelle T représente le reste d'un alcool, en acide carboxylique de formule générale III,

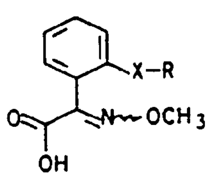

III

et on transforme celui-ci, de manière également connue, en ester d'acide thiolcarboxylique avec du méthanethiol ou un thiolate de méthanate alcalin, ou
on met à réagir un ester d'acide carboxylique de formule

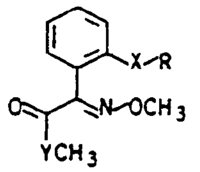

IV

avec du $P_4S_{10}$ ou du 2,4-bis(4-méthoxyphényl)-1,3-dithia-2,4-diphosphétane-2,4-disulfure.

**7.** Agent fongicide, contenant une quantité efficace au point de vue fongicide d'un ester d'acide thiolcarboxylique de formule générale I,

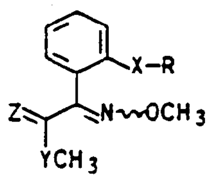

I

dans laquelle les substituants ont les significations suivantes :

- X — oxygène, soufre, oxyméthylène, méthylénoxy, thiométhylène, méthylènethio, éthylène, éthénylène ou éthylnylène,
- Y, Z — soufre ou oxygène, Y et Z ne pouvant pas représenter simultanément oxygène.
- R — alkyle en C1-C6, aryle ou hétéro-aryle à un, deux ou trois noyaux, aryle et hétéro-aryle pouvant porter les restes $R^1$ suivants :
- $R^1$ — halogène, cyano, nitro, alkyle en C1-C6, cycloalkyle en C3-C6, alcoxy en C1-C6, trifluorométhyle, aryloxy à un ou deux noyaux ou aryle à un, deux ou trois noyaux, aryloxy et aryle pouvant à leur tour être substitués par les restes $R^1$ précités, et un support usuel.

**8.** Procédé de lutte contre les champignons nuisibles, caractérisé par le fait que l'on traite les champignons ou les plantes, semences, matériaux ou surfaces menacés par l'attaque par les champignons avec une quantité efficace au point de vue fongicide d'un ester d'acide thiolcarboxylique de formule générale I,

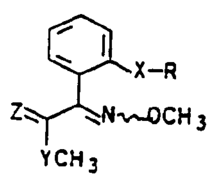

I

dans laquelle les substituants ont les significations suivantes :

- X — oxygène, soufre, oxyméthylène, méthylénoxy, thiométhylène, méthylènethio, éthylène, éthénylène ou éthylnylène,
- Y, Z — soufre ou oxygène, Y et Z ne pouvant pas représenter simultanément oxygène.
- R — alkyle en C1-C6, aryle ou hétéro-aryle à un, deux ou trois noyaux, aryle et hétéro-aryle pouvant porter les restes $R^1$ suivants :
- $R^1$ — halogène, cyano, nitro, alkyle en C1-C6, cycloalkyle en C3-C6, alcoxy en C1-C6, trifluorométhyle, aryloxy à un ou deux noyaux ou aryle à un, deux ou trois noyaux, aryloxy et aryle pouvant à leur tour être substitués par les restes $R^1$ précités.